# FASCICULE DE BREVET EUROPEEN

(11) **EP 1 590 321 B1**
(45) Date de publication et mention de la délivrance du brevet: **28.04.2010**
(21) Numéro de dépôt: 04704251.0
(22) Date de dépôt: 22.01.2004
(51) Int. Cl.: C07C 271/10, C07C 269/06, C07C 269/00, C07D 263/44, A61K 31/27, A61K 31/33

(54) **DERIVES D' ARYLALKYLCARBAMATES, LEUR PREPARATION ET LEUR APPLICATION EN THERAPEUTIQUE**
ARYLALKYLCARBAMATDERIVATE, VERFAHREN ZUR IHRER HERSTELLUNG UND DEREN THERAPEUTISCHEN ANWENDUNGEN
ARYL ALKYL CARBAMATE DERIVATIVES PRODUCTION AND USE THEREOF IN THERAPY

(30) Priorité: 23.01.2003 FR 0300704
(43) Date de publication de la demande: 02.11.2005
(73) Titulaire: Sanofi-Aventis, 75013 Paris (FR)
(72) Inventeur: ABOUABDELLAH, Ahmed, F-94320 Thiais (FR); ALMARIO GARCIA, Antonio, F-92290 Chatenay Malabry (FR); HOORNAERT, Christian, F-92160 Antony (FR); RAVET, Antoine, F-92340 Bourg la Reine (FR)
(74) Mandataire: Monain, Patrice
(86) Numéro de dépôt international: PCT/FR2004/000139
(87) Numéro de publication internationale: WO 2004/067498

(56) Documents cités:
- EP-A- 0 545 478
- WO-A-02/087569
- WO-A-03/065989
- DE-A1- 3 003 653
- US-A- 2 909 467
- US-A- 3 311 655
- US-A- 3 742 022
- US-B1- 6 462 054
- CHEMICAL ABSTRACTS, vol. 73, no. 3, 20 juillet 1970 (1970-07-20), Columbus, Ohio, US; abstract no.: 14748u, DOVLATYAN, V. V. ET AL.: "Synthesis of pesticides." page 358 colonne 1 XP002257138 & ARM. KHIM. ZH., vol. 23, no. 2, 1970, pages 173-179,
- DATABASE CA [Online] CHEMICAL ABSTRACTS SERVICE, COLUMBUS, OHIO, US; KANO, SHINZO ET AL: "A new generation of .alpha.-oxa acyliminium ions and an application to a synthesis of oxazolo[4,3-a]isoquinoline and related compounds" XP002504322 extrait de STN Database accession no. 1984:51491 & CHEMISTRY LETTERS , (9), 1475-6 CODEN: CMLTAG; ISSN: 0366-7022, 1983,
- KANO S ET AL: "DIASTEREOCONVERSION OF THREO 2-AMINO ALCOHOLS TO BRYTHRO ISOMERS THROUGH A NEW CYCLOCARBAMATION" HETEROCYCLES, ELSEVIER SCIENCE PUBLISHERS B.V. AMSTERDAM, NL, vol. 27, no. 5, 1 janvier 1988 (1988-01-01), pages 1241-1248, XP001098667 ISSN: 0385-5414
- DATABASE CA [Online] CHEMICAL ABSTRACTS SERVICE, COLUMBUS, OHIO, US; KANO, SHINZO ET AL: "An enantioselective synthesis of 1-(.alpha.-hydroxyalkyl)-1,2,3,4- tetrahydroisoquinolines through optically active N-oxaacyliminium ion intermediates" XP002504323 extrait de STN Database accession no. 1985:437352 & HETEROCYCLES , 23(2), 395-8 CODEN: HTCYAM; ISSN: 0385-5414, 1985,
- DATABASE CA [Online] CHEMICAL ABSTRACTS SERVICE, COLUMBUS, OHIO, US; DUGGAN, M. E. ET AL: "Copper(I) chloride-catalyzed addition of alcohols to alkyl isocyanates. A mild and expedient method for alkyl carbamate formation" XP002504324 extrait de STN Database accession no. 1989:573711 & SYNTHESIS , (2), 131-2 CODEN: SYNTBF; ISSN: 0039-7881, 1989,
- CESA S ET AL: "Tetraethylammonium Hydrogen Carbonate in Organic Synthesis: Synthesis of Oxazolidine-2,4-diones" TETRAHEDRON, ELSEVIER SCIENCE PUBLISHERS, AMSTERDAM, NL, vol. 55, no. 1, 1 janvier 1999 (1999-01-01), pages 193-200, XP004150862 ISSN: 0040-4020
- SHAPIRO S L ET AL: "N-Substituted Oxazolidinediones" JOURNAL OF THE AMERICAN CHEMICAL SOCIETY, AMERICAN CHEMICAL SOCIETY, WASHINGTON, DC.; US, US, vol. 81, no. 24, 1 janvier 1959 (1959-01-01), pages 6498-6504, XP002395909 ISSN: 0002-7863
- CHEMICAL ABSTRACTS, vol. 73, no. 3, 20 juillet 1970 (1970-07-20), Columbus, Ohio, US; abstract no.: 14748u, DOVLATYAN, V. V. ET AL.: "Synthesis of pesticides." page 358 colonne 1 XP002257138 & ARM. KHIM. ZH., vol. 23, no. 2, 1970, pages 173-179,
- GIORGIO TARZIA ET AL.: "Design, synthesis, and structure-activity relationships of alkylcarbamic acid aryl esters, a new class of Fatty Acid Amide Hydrolase inhibitors" JOURNAL OF MEDICINAL CHEMISTRY., vol. 46, no. 12, 2003, pages 2352-2360, XP002257137 AMERICAN CHEMICAL SOCIETY. WASHINGTON., US ISSN: 0022-2623

## Description

L'invention a pour objet des dérivés d'arylalkylcarbamates, leur préparation et leur application en thérapeutique.

WO02/087569 se rapporte à des dérivés bisarylimidazolyl et des compositions pharmaceutiques comprenant ces dérivés inhibant l'enzyme fatty acid amide hydrolase.

US 6,462,541 se rapporte à des inhibiteurs de l'enzyme fatty acid hydrolase utilisant un pharmacophore hétérocyclique.

Les composés de l'invention répondent à la formule générale (I) : 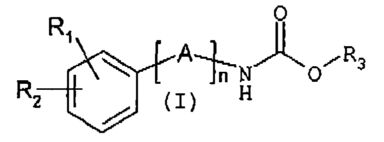 dans laquelle
n représente un nombre entier allant de 1 à 7 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente soit un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; soit un groupe C₂-alcynylène ;
Z représente un groupe C₃₋₇-cycloalkyle, de formule : 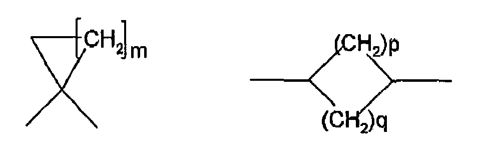 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxy, cyano, nitro, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle ; C₁₋₄-thioalkyle, C₁₋₄-fluoroalkyle, C₁₋₄-fluoroalcoxy, C₁₋₄-fluorothioalkyle,
ou un groupe choisi parmi notamment un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle , pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -0- (C₁₋₃-alkylène) -O-, 4-pipérazinyle éventuellement substitué par un C₁₋₃-alkyle ou par un benzyle ;
R₆ et R₇ représentent indépendamment l'un de l'autre un groupe C₁₋₃-alkyle, un phényle ; et
R₃ représente un groupe de formule générale CHR₄CONHR₅ dans laquelle
R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène.

Dans tout le texte de la demande de brevet, le composé suivant ne fait pas partie de l'invention :
benzylcarbamate de 2-amino-2-oxoéthyle.

Dans le cadre de l'invention, les composés de formule générale (I) peuvent donc comporter plusieurs groupes A identiques ou différents entre eux.

Parmi les composés de formule générale (I), une première famille de composés préférés est constituée des composés pour lesquels :
n représente un nombre entier allant de 1 à 7 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente soit un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; soit un groupe C₂-alcynylène ;
Z représente un groupe C₃₋₇-cycloalkyle, de formule : 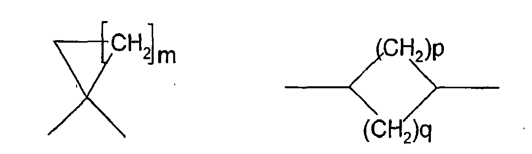
m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxy, cyano, nitro, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle ;
R₂ représente
un atome d'hydrogène, d'halogène
ou un groupe cyano, nitro, hydroxy, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle,
ou un groupe choisi parmi notamment un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle , pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₆ et R₇ représentent indépendamment l'un de l'autre un groupe C₁₋₃-alkyle, un phényle ; et
R₃ représente un groupe de formule générale CHR₄CONHR₅ dans laquelle
R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène ;
à la condition que si R₁ et R₂ représentent un atome d'hydrogène et A est un groupe X, X étant un méthylène, alors n est différent de 1.

Parmi les composés de formule générale (I), une deuxième famille de composés préférés est constituée des composés pour lesquels :
- quand n est égal à 1 :
   A est choisi parmi un ou plusieurs groupes X, Y et/ou Z ;
   X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
   Y représente soit un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; soit un groupe C₂-alcynylène ;
   Z représente un groupe C₃₋₇-cycloalkyle, de formule : 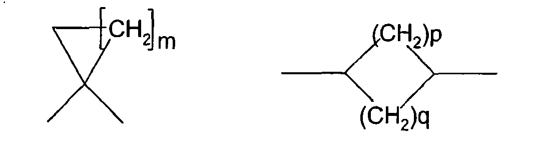
   m représente un nombre entier allant de 1 à 5 ;
   p et q représentent des nombres entiers et sont définis tels que p+q soit un nombre allant de 1 à 5 ;
   R₁ représente un atome d'hydrogène, d'halogène ou un groupe hydroxy, cyano, nitro, C₁₋₉-alkyle, C₁₋₄-alcoxy, C₁₋₄-thioalkyle, C₁₋₄-fluoroalkyle, C₁₋₄-fluoroalcoxy, C₁₋₄-fluorothioalkyle ;
   R₂ représente
   un atome d'halogène
   ou un groupe cyano, nitro, hydroxy, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle,
   ou un groupe choisi parmi notamment un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
   R₆ et R₇ représentent indépendamment l'un de l'autre un groupe C₁₋₃-alkyle, un phényle ; et
   R₃ représente un groupe de formule générale CHR₄CONHR₅ dans laquelle
   R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène ;
- quand n représente un nombre entier allant de 2 à 7 :
   A est choisi parmi un ou plusieurs groupes X, Y et/ou Z ;
   X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
   Y représente soit un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; soit un groupe C₂-alcynylène ;
   Z représente un groupe C₃₋₇-cycloalkyle, de formule : 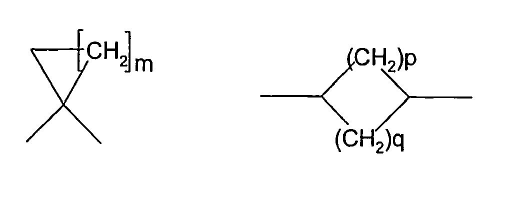
   m représente un nombre entier allant de 1 à 5 ;
   p et q représentent des nombres entiers et sont définis tels que p+q soit un nombre allant de 1 à 5 ;
   R₁ représente un atome d'hydrogène, d'halogène ou un groupe hydroxy, cyano, nitro, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle ;
   R₂ représente
   un atome d'hydrogène, d'halogène
   ou un groupe cyano, nitro, hydroxy, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle,
   ou un groupe choisi parmi notamment un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle , pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
   R₆ et R₇ représentent indépendamment l'un de l'autre un groupe C₁₋₃-alkyle, un phényle ; et
   R₃ représente un groupe de formule générale CHR₄CONHR₅ dans laquelle
   R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène.

Parmi les composés de formule générale (I), une troisième famille de composés particulièrement préférés est constituée des composés pour lesquels :
n représente un nombre entier de 1 à 5 ; et/ou
A est choisi parmi un ou plusieurs groupes X et/ou Z ;
X représente un groupe C₁₋₂-alkylène, plus particulièrement méthylène, éventuellement substitué par un ou plusieurs groupes C₁₋₃-alkyle, plus particulièrement méthyle;
Z représente un groupe C₃₋₇-cycloalkyle, de formule : 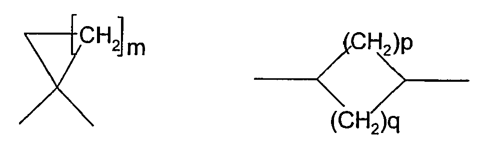
m représente un nombre entier allant de 1 à 5, plus particulièrement égal à 1 ;
p et q représentent des nombres entiers et sont définis tels que p+q soit un nombre allant de 1 à 5, plus particulièrement égal à 4 ; et/ou
R₁ représente un hydrogène ou un halogène, plus particulièrement un chlore ou un fluor, ou un groupe C₁₋₄-alcoxy, plus particulièrement un méthoxy ; et/ou
R₂ représente un atome d'hydrogène, d'halogène, plus particulièrement un chlore, un brome ou un fluor, ou un groupe hydroxy, C₁₋₄-alkyle, plus particulièrement méthyle, C₁₋₄-alcoxy, plus particulièrement méthoxy, C₁₋₄-fluoroalkyle, plus particulièrement trifluorométhyle, C₁₋₄-fluoroalcoxy, plus particulièrement trifluorométhoxy, ou un groupe choisi parmi un phényle, naphtalènyle, biphényle, phényléthylènyle, pyridinyle, pyrimidinyle, pyrazinyle, quinolinyle, isoquinolinyle, thiényle, furanyle, isoxazolyle, thiadiazolyle, phénylimidazolyle, benzothiényle, dibenzofuranyle, benzimidazolyle, pyrrolopyridinyle, phényloxy, phénylsulfonyle, benzoyle, benzyloxy ou phénylpropoxy, et éventuellement substitué par un ou plusieurs substituants choisis parmi un atome d'halogène, plus particulièrement un chlore ou un fluor, un groupe cyano, nitro, C₁₋₄-alkyle, plus particulièrement méthyle, éthyle, isopropyle, butyle, tertiobutyle, C₁₋₄-alcoxy, plus particulièrement méthoxy, éthoxy, C₁₋₄-thioalkyle, plus particulièrement thiométhyle, C₁₋₃-fluoroalkyle, plus particulièrement trifluorométhyle, C₁₋₃-fluoroalcoxy, plus particulièrement trifluorométhoxy, phényloxy, benzyloxy, NR₆R₇, NHCOR₆, COR₆, CO₂R₆, SO₂R₆, -O-(C₁₋₃-alkylène)-O-, plus particulièrement -O-(CH₂)-O- ; et/ou
R₆ et R₇ représentent indépendamment l'un de l'autre un groupe C₁₋₃-alkyle, plus particulièrement un méthyle ; et/ou
R₃ représente un groupe de formule générale CHR₄CONHR₅ dans laquelle
R₄ représente un atome d'hydrogène ou un groupe C₁₋₃-alkyle et R₅ représente un atome d'hydrogène ou un groupe C₁₋₃-alkyle, plus particulièrement méthyle, éthyle, C₃₋₅-cycloalkyle, plus particulièrement cyclopropyle, C₃₋₇-cycloalkyle-C₁₋₆-alkylène, plus particulièrement cyclopropylméthyle.

Parmi les composés de cette troisième famille de composés particulièrement préférés, sont plus particulièrement préférés les composés pour lesquels :
n représente un nombre entier de 1 à 5 ; et/ou
A représente un groupe C₁₋₂-alkylène, plus particulièrement méthylène ; et/ou
R₁ représente un hydrogène ou un halogène, plus particulièrement un chlore ou un fluor ; et/ou
R₂ représente un groupe choisi parmi un phényle, naphtalènyle, phényloxy, benzyloxy, pyridinyle, quinolinyle, isoquinolinyle, phénylimidazole ou pyrrolopyridinyle, et éventuellement substitué par un ou plusieurs substituants choisis parmi un atome d'halogène, plus particulièrement un chlore ou un fluor, un groupe cyano, C₁₋₄-alkyle, plus particulièrement méthyle, C₁₋₄-alcoxy, plus particulièrement méthoxy, C₁₋₃-fluoroalkyle, plus particulièrement trifluorométhyle, C₁₋₃-fluoroalcoxy, plus particulièrement trifluorométhoxy ; et/ou
R₃ représente un groupe de formule générale CHR₄CONHR₅ dans laquelle
R₄ représente un hydrogène et R₅ représente un atome d'hydrogène ou un groupe C₁₋₃-alkyle, plus particulièrement méthyle, éthyle, C₃₋₅-cycloalkyle, plus particulièrement cyclopropyle, C₃₋₇-cycloalkyle-C₁₋₆-alkylène, plus particulièrement cyclopropylméthyle.

Parmi les composés de formule générale (I), une quatrième famille de composés particulièrement préférés est constituée des composés pour lesquels :
n présente un nombre entier de 5 à 7 ; et/ou
A représente un groupe C₁₋₂-alkylène, plus particulièrement méthylène ; et/ou
R₁ et R₂ représentent indépendamment l'un de l'autre un atome d'hydrogène ou d'halogène ou un groupe cyano, hydroxy, C₁₋₄-alkyle, C₁₋₄-alcoxy, C₁₋₄-fluoroalkyle, C₁₋₄-fluoroalcoxy ; et/ou
R₃ représente un groupe de formule générale CHR₄CONHR₅ dans laquelle
R₄ représente un hydrogène et R₅ représente un atome d'hydrogène ou un groupe C₁₋₃-alkyle, plus particulièrement méthyle, éthyle, C₃₋₅-cycloalkyle, plus particulièrement cyclopropyle, C₃₋₇-cycloalkyle-C₁₋₆-alkylène, plus particulièrement cyclopropylméthyle.

L'invention a également pour objet, parmi les composés de formule générale (I), des composés répondant à la formule générale (I') : 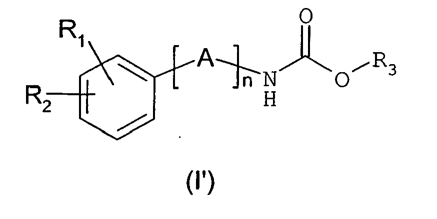 dans laquelle
n représente un nombre entier compris entre 1 et 6 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène;
Z représente un groupe C₃₋₇-cycloalkyle, de formule : 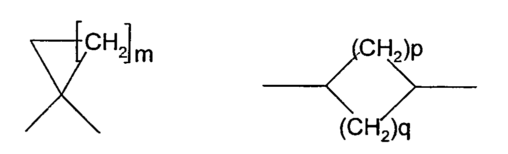 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxy, cyano, nitro, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle ;
R₂ représente
un atome d'hydrogène, d'halogène
ou un groupe cyano, nitro, hydroxy, C₁₋₃-alkyle, C₁₋₃-alcoxy, C₁₋₃-thioalkyle, C₁₋₃-fluoroalkyle, C₁₋₃-fluoroalcoxy, C₁₋₃-fluorothioalkyle,
ou un groupe choisi parmi un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₆ et R₇ représentent indépendamment l'un de l'autre un groupe C₁₋₃-alkyle, un phényle ; et
R₃ représente un groupe de formule générale CHR₄CONHR₅ dans laquelle
R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène.

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par :
- C_{t-z} où t et z peuvent prendre les valeurs de 1 à 12, une chaîne carbonée pouvant avoir de t à z atomes de carbone, par exemple C₁₋₃ une chaîne carbonée qui peut avoir de 1 à 3 atomes de carbone;
- alkyle, un groupe aliphatique saturé, linéaire ou ramifié; par exemple un groupe C₁₋₃-alkyle représente une chaîne carbonée de 1 à 3 atomes de carbone, linéaire ou ramifiée, plus particulièrement un méthyle, éthyle, propyle, 1-méthyléthyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₅-cycloalkyle représente un groupe carboné cyclique de 3 à 5 atomes de carbone, plus particulièrement un cyclopropyle, cyclobutyle, cyclopentyle ;
- alcènylène, un groupe aliphatique à 2 carbones, insaturé divalent, plus particulièrement un éthylène ;
- C₂-alcynylène, un groupe -C≡C- ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- fluoroalkyle, un groupe alkyle dont un ou plusieurs atomes d'hydrogène ont été substitués par un atome de fluor ;
- fluoroalcoxy, un groupe alcoxy dont un ou plusieurs atomes d'hydrogène ont été substitués par un atome de fluor ;
- fluorothioalkyle, un groupe thioalkyle dont un ou plusieurs atomes d'hydrogène ont été substitués par un atome de fluor
- atome d'halogène, un fluor, un chlore, un brome ou un iode.

Les composés de l'invention peuvent être préparés selon différentes méthodes, illustrées par les schémas qui suivent.

Ainsi une première méthode (schéma 1) consiste à faire réagir une amine de formule générale (II), dans laquelle R₁, R₂, n et A sont tels que définis ci-dessus, avec un carbonate de formule générale (III), dans laquelle U représente un atome d'hydrogène ou un groupe nitro et R₃ est tel que défini ci-dessus, dans un solvant tel que le toluène ou le dichloroéthane, à une température comprise entre 0 et 80°C.

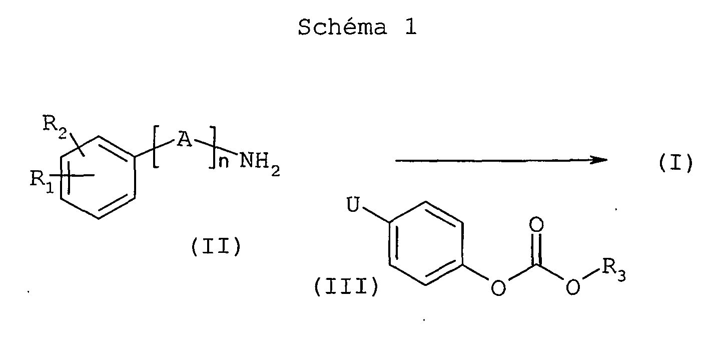

Les carbonates de formule générale (III) peuvent être préparés selon toute méthode décrite dans la littérature, par exemple par réaction d'un alcool de formule générale HOR₃ avec le chloroformiate de phényle ou de 4-nitrophényle, en présence d'une base telle que la triéthylamine ou la diisopropyléthylamine.

Une autre méthode (schéma 2) d'obtention des composés de formule générale (I), consiste à faire réagir une amine de formule générale (II), telle que définie ci-dessus, avec un carbonate de formule générale (IIIa) dans laquelle V représente un atome d'hydrogène ou un groupe nitro, R₄ est tel que défini ci-dessus et R représente un groupe méthyle ou éthyle. Le carbamate-ester de formule générale (Ia) ainsi obtenu est ensuite transformé en composé de formule générale (I), par aminolyse au moyen d'une amine de formule générale R₅NH₂ où R₅ est tel que défini ci-dessus. La réaction d'aminolyse peut être réalisée dans un solvant tel que le méthanol ou un mélange de solvants tels que le méthanol et le tétrahydrofurane.

Les carbonates de formule générale (IIIa) peuvent être préparés de manière analogue aux carbonates de formule (III).

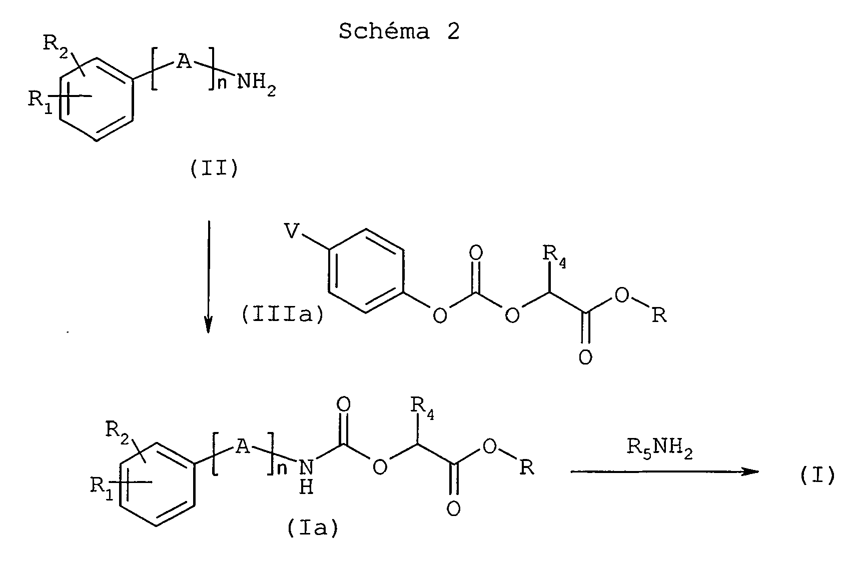

Une variante de préparation (schéma 3) des composés de formule générale (I), consiste à faire réagir un dérivé de formule générale (IIa) dans laquelle R₁, R₂, n et A sont tels que définis ci-dessus et W représente un groupement hydroxy, mésylate, tosylate, ou un atome de chlore, de brome ou d'iode, avec une oxazolidine-dione de structure générale (IV) dans laquelle R₄ est tel que défini ci-dessus pour fournir le dérivé oxazolidine-dione de structure générale (V) .

Dans le cas où W représente un groupement hydroxy, la réaction peut être conduite suivant les conditions de Mitsunobu (Synthesis 1981, 1-28), par exemple, par action de l'azodicarboxylate de diéthyle ou diisopropyle en présence de triphénylphosphine. Dans le cas où X représente un atome de chlore, de brome, ou d'iode, ou un groupement mésylate ou tosylate, la réaction peut être conduite en présence d'une base telle que la 1,1,3,3-tétraméthylguanidine, l'hydrure de sodium ou le tert-butoxyde de sodium dans un solvant tel que le tétrahydrofurane, l'acétonitrile ou le diméthylformamide à une température comprise entre 0°C et 80°C. Le dérivé oxazolidine-dione de formule générale (V) ainsi obtenu est ensuite transformé en composé de formule générale (I), par aminolyse au moyen d'une amine de formule générale R₅NH₂ où R₅ est tel que défini ci-dessus.

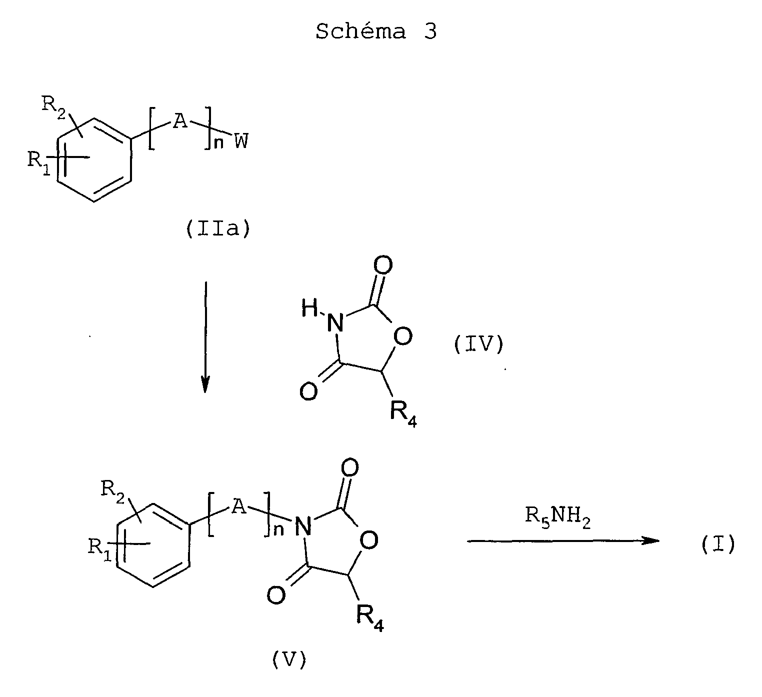

Quand R₂ représente un groupe de type aryle ou hétéroaryle dans un composé de formule (I), (Ia), (II), (IIa) ou (V), l'introduction de R₂ sur le cycle phényle peut être réalisée par réaction d'un dérivé d'un composé de formule générale (I), (Ia), (II), (IIa) ou (V), dont le cycle phényle porte un atome de chlore , de brome, d'iode ou un groupement triflate dans la position où l'on souhaite introduire R₂, avec un dérivé d'acide boronique d'aryle ou d'hétéroaryle suivant les conditions de réaction de Suzuki (Chem. Rev. (1995), 95, 2457-2483), ou avec un dérivé tri-alkylstanneux d'aryle ou d'hétéroaryle suivant les conditions de réaction de Stille (Angew. Chem. (1986), 25, 508-524).

Quand R₂ représente un groupe de type aryloxy ou de type imidazolyle, pyrrolopyridinyle ou indolyle dans un composé de formule (I), (Ia), (II), (IIa) ou (V), l'introduction de R₂ sur le cycle phényle peut être réalisé par une réaction de O-arylation ou de N-arylation suivant les conditions de réaction de Buchwald (Angew. Chem. (2003), 42, 5400-5449).

Les composés de formules générales (II), (IIa) et (IV), quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les composés de formule générale (Ia) dans laquelle n, A, R₁, R₂ et R₄ sont tels que définis dans la formule générale (I) et R représente un groupe méthyle ou éthyle, sont nouveaux et font également partie de l'invention. Ils sont utiles en tant qu'intermédiaires de synthèse pour la préparation des composés de formule générale (I).

Les composés de formule générale (V) dans laquelle n, A, R₁ et R₄ sont tels que définis dans la formule générale (I) et où R₂ représente
un atome d'hydrogène, de brome, d'iode ou de fluor,
ou un groupe cyano, nitro, hydroxy, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle,
ou un groupe choisi parmi un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolylyle, benzisothiazolyle, dihydroindolyle , pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ; et
R₆ et R₇ représentent indépendamment l'un de l'autre un groupe C₁₋₃-alkyle, un phényle ;
sont nouveaux et font également partie de l'invention. Ils sont utiles en tant qu'intermédiaires de synthèse pour la préparation des composés de formule générale (I).

Les exemples qui vont suivre illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus. PF(°C) représente le point de fusion en degrés Celsius.
Les numéros indiqués entre parenthèses dans les titres des exemples correspondent à ceux de la 1ère colonne du tableau ci-après.
La nomenclature UICPA (Union Internationale de Chimie Pure et Appliquée - IUPAC en anglais) a été utilisée pour la dénomination des composés dans les exemples suivants. Par exemple, pour le groupe biphényle, la numérotation suivante a été respectée :

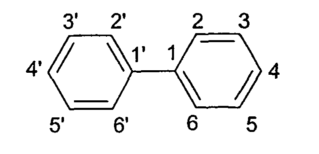

### Exemple 1 (Composé N°1)

### 1,1'-biphényl-4-ylméthylcarbamate de 2-(méthylamino)-2-oxoéthyle

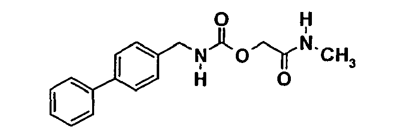

A 0,1 g (0,97 mmole) de N-méthyl-2-hydroxyacétamide, on ajoute goutte à goutte et à température ambiante une solution de 0,196 g (0,97 mmole) de chloroformiate de 4-nitrophényle dans 3 ml de chlorure de méthylène et 0,166 ml (0,97 mmole) de *N,N*-diisopropyléthylamine. On agite le mélange à température ambiante pendant 45 min, puis on ajoute goutte à goutte et à température ambiante une solution de 0,195 g (1,067 mmole) de 4-phénylbenzylamine dans 3 ml de chlorure de méthylène et 0,166 ml (0,97 mmole) de N,N-diisopropyléthylamine. On agite le mélange à température ambiante pendant 1 h. On lave avec une solution aqueuse saturée en chlorure d'ammonium, avec une solution aqueuse 10% de carbonate de sodium et avec une solution aqueuse saturée de chlorure de sodium. On sépare les phases et on sèche la phase organique sur sulfate de sodium. On filtre, on concentre le filtrat sous pression réduite et on purifie le résidu par chromatographie sur gel de silice avec de l'acétate d'éthyle.

On obtient 0,1 g de solide blanc.
LC-MS : M+H = 299
PF(°C) : 189 - 190°C
RMN ¹H (DMSO-d₆) δ (ppm) : 7, 90 - 7,35 (m, 11H) ; 4,40 (s, 2H); 4,30 (d, 2H); 2,65 (d, 3H).

### Exemple 2 (Composé N°125)

### 2-[4-(trifluorométhyl)phényl]éthylcarbamate de 2-(méthylamino)-2-oxoéthyle

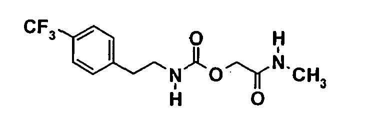

### 2.1. 3-{2-[4-(trifluorométhyl)phényl]éthyl}-1,3-oxazolidin-2,4-dione

A une solution de 1,4 g (7,36 mmoles) de 2-[4-(trifluorométhyl)phényl]éthanol, de 2,22 g (8,47 mmoles) de triphénylphosphine et de 0,82 g (8,1 mmoles) de 1,3-oxazolidin-2,4-dione (J. Med. Chem. 1991, 34, 1542-1543) dans 25 ml de tétrahydrofurane, refroidie à environ -10°C, on ajoute goutte à goutte, sous atmosphère inerte, une solution de 1,7 g (8,47 mmoles) de diisopropylazidocarboxylate (DIAD) dans 5 ml de tétrahydrofurane tout en maintenant la température du milieu réactionnel entre -10°C et 0°C. On poursuit l'agitation à 0°C pendant 1 heure, puis à 25°C pendant 20 heures.

On concentre le filtrat sous pression réduite, on reprend le résidu avec du dichlorométhane et une solution aqueuse d'hydroxyde de sodium à 5% (10 ml). On sépare la phase aqueuse puis on l'extrait 2 fois avec du dichlorométhane. On réunit les phases organiques et on les lave successivement avec une solution aqueuse d'acide chlorhydrique (1N), puis une solution aqueuse saturée d'hydrogénocarbonate de sodium et une solution aqueuse saturée de chlorure de sodium. On sèche la phase organique sur sulfate de sodium et on concentre le filtrat sous pression réduite. On purifie le résidu ainsi obtenu par chromatographie sur gel de silice en éluant avec un mélange 20/80 d'acétate d'éthyle et de cyclohexane.

On obtient 1,5 g d'oxazolidine-dione sous forme d'huile.

### 2.2. 2-[4-(trifluorométhyl)phényl]éthylcarbamate de 2-(méthylamino)-2-oxoéthyle

A une solution de 0,75 g (2,74 mmoles) de 3-{2-[4-(trifluorométhyl)phényl]éthyl}-1,3-oxazolidin-2,4-dione, obtenu à l'étape 2.1., dans 10 ml de méthanol, on ajoute 8 ml (16,47 mmoles) d'une solution de méthylamine (2M) dans le tétrahydrofurane. On poursuit l'agitation à température ambiante pendant 12 heures.

Après concentration sous pression réduite, on purifie le résidu obtenu par chromatographie sur gel de silice en éluant avec un mélange 95/5 de dichlorométhane et de méthanol. On obtient un solide blanc que l'on recristallise dans un mélange d'acétate d'éthyle et de diisopropyléther. On obtient 0,530 g de produit pur.
LC-MS : M+H = 305
PF(°C) : 140-142°C
RMN¹H (CDCI₃) δ (ppm) : 2,85 (d, 3H) ; 2, 95 (t, 2H) ; 3,50 (q, 2H) ; 4,60 (s, 2H) ; 4,90 (large s, 1H) ; 6,15 (large s, 1H) ; 7,35 (d, 2H) ; 7,60 (d, 2H).

### Exemple 3 (Composé N°150)

### 2-(4'-chloro-1,1'-biphényl-4-yl)éthylcarbamate de 2-(méthylamino)-2-oxoéthyle

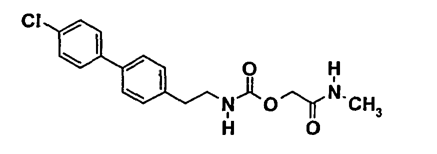

### 3.1. 3-[2-(4-bromophényl)éthyl]-1,3-oxazolidin-2,4-dione

On utilise le mode opératoire décrit dans l'exemple 2 (étape 2.1.) ; à partir de 4 g (19,89 mmoles) de 2-(4-bromophényl)éthanol, de 6,3 g (23,87 mmoles) de triphénylphosphine, de 2,4 g (23,87 mmoles) de 1,3-oxazolidin-2,4-dione et de 4,8 g (23,87 mmoles) de diisopropylazodicarboxylate, on obtient 4,6 g de produit pur sous forme de solide blanc, après chromatographie sur gel de silice en éluant avec de dichlorométhane.
PF(°C) : 122-124°C

### 3.2. 3-[2-(4'-chloro-1,1'-biphényl-4-yl)éthyl]-1,3-oxazolidin-2,4-dione

Dans un ballon tricol de 250 ml, placé sous atmosphère inerte, on introduit 2 g (7,04 mmoles) de 3-[2-(4-bromophényl)éthyl]-1,3-oxazolidin-2,4-dione, obtenu à l'étape 3.1., 2,2 g (14,08 mmoles) de l'acide 4-chlorophénylboronique, 6,5 g (28,16 mmoles) de phosphate de potassium hydraté en suspension dans 100 ml de 1,2-diméthoxyéthane. On ajoute ensuite 0,80 g (0,70 mmole) de palladium tétrakis(triphénylphosphine). On porte ensuite le mélange réactionnel au reflux pendant une nuit.

On sépare les sels par filtration sur célite, puis on concentre le filtrat sous pression réduite. On reprend le résidu avec de l'acétate d'éthyle et de l'eau. On sépare la phase organique, on la lave avec une solution aqueuse saturée de chlorure de sodium. On concentre le filtrat sous pression réduite, et on purifie le résidu par chromatographie sur gel de silice en éluant avec de dichlorométhane.

On obtient 1,22 g de produit pur sous forme de solide blanc.
PF(°C) : 182-184°C

### 3.3. 2-(4'-chloro-1,1'-biphényl-4-yl)éthylcarbamate de 2-(méthylamino)-2-oxoéthyle

On opère comme décrit dans l'exemple 2 (étape 2.2.). A partir de 0,40 g (1,27 mmoles) de 3-[2-(4'-chloro-1,1'-biphényl-4-yl)éthyl]-1,3-oxazolidin-2,4-dione, obtenu à l'étape 3.2., et de 2,5 ml (5,07 mmoles) de méthylamine (2M) dans le tétrahydrofurane, on obtient 0,250 g de produit pur sous forme de solide blanc, après chromatographie sur gel de silice en éluant avec un mélange 98/2 de dichlorométhane et de méthanol.
LC-MS : M+H = 348
PF(°C) : 186-188°C
RMN ¹H (CDCl₃) δ (ppm) : 2,80 (d, 3H) ; 2,95 (t, 2H) ; 3,55 (q, 2H) ; 4,60 (s, 2H) ; 4,90 (large s, 1H) ; 6,15 (large s, 1H) ; 7,33 (d, 2H) ; 7,40-7,70 (massif, 6H).

### Exemple 4 (Composé N°192)

### 2-(3'-chloro-4'-fluoro-1,1'-biphényl-4-yl)éthylcarbamate de 2-(méthylamino)-2-oxoéthyle

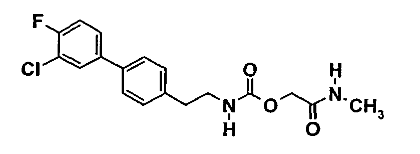

### 4.1. 2-(4-bromophényl)éthylcarbamate de 2-(méthylamino)-2-oxoéthyle

On opère comme décrit dans l'exemple 2 (étape 2.2.). A partir de 2,6 g (9,15 mmoles) de 3-[2-(4-bromophényl)éthyl]-1,3-oxazolidin-2,4-dione, préparé dans l'exemple 3 (étape 3.1.) et de 18,3 ml (36,6 mmoles) de méthylamine (2M) dans le tétrahydrofurane et après reprise dans le diisopropyléther, on obtient 2,6 g de produit pur sous forme d'un solide blanc.
PF(°C) : 122-124°C

### 4.2. 2-(3'-chloro-4'-fluoro-1,1'-biphényl-4-yl)éthylcarbamate de 2-(méthylamino)-2-oxoéthyle

On utilise la méthode décrite dans l'exemple 2 (étape 2.2.). A partir de 0,820 g (2,6 mmoles) de 2-(4-bromophényl)-éthylcarbamate de 2-(méthylamino)-2-oxoéthyle obtenu à l'étape 4.1, de 0,4 g (2,86 mmoles) de l'acide 3-chloro-4-fluorophénylboronique, de 2,86 ml (5,72 mmoles) d'une solution aqueuse de carbonate de sodium (2 M), de 3 ml d'éthanol et de 0,15 g (0,13 mmole) de palladium tétrakis(triphénylphosphine), on obtient 0,42 g de produit pur sous forme de solide blanc, après chromatographie sur gel de silice en éluant avec un mélange 95/5 de dichlorométhane et de méthanol, suivie d'une recristallisation dans l'acétate d'éthyle.
LC-MS : M+H = 365
PF(°C) : 178-180°C
RMN¹H (CDCl₃) δ (ppm) : 2,80 (d, 3H) ; 2,90 (t, 2H) ; 3,55 (q, 2H) ; 4,60 (s, 2H) ; 4,90 (large s, 1H) ; 6,15 (large s, 1H) ; 7,10-7,30 (massif, 3H) ; 7,40-7,55 (massif, 3H) ; 7,65 (dd, 1H).

### Exemple 5 (Composé N°9)

### (3-chloro-4'-fluoro-1,1'-biphényl-4-yl)méthylcarbamate de 2-(méthylamino)-2-oxoéthyle

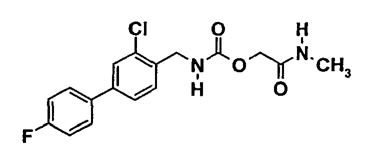

### 5.1. Acide 3-chloro-4'-fluoro-1,1'-biphényl-4-carboxylique

Sous atmosphère inerte, on introduit 5 g (21,2 mmoles) d'acide 4-bromo-2-chlorobenzoïque, 2,96 g (23,3 mmoles) d'acide 4-fluorophénylboronique, 31,8 ml (63,6 mmoles) d'une solution aqueuse de carbonate de sodium (2M) en suspension dans 40 ml de toluène. On ajoute ensuite 0,80 g (0,70 mmole) de palladium tétrakis(triphénylphosphine). On porte ensuite le mélange réactionnel au reflux pendant une nuit.

On sépare les sels par filtration sur célite, puis on concentre le filtrat sous pression réduite. On reprend le résidu avec de l'acétate d'éthyle et une solution aqueuse d'acide chlorhydrique (4N). On sépare la phase organique, on la lave avec une solution aqueuse saturée de chlorure de sodium, et on concentre le filtrat sous pression réduite.

On obtient 3,1 g d'acide sous forme de solide beige utilisé tel quel dans l'étape suivante.

### 5.2. (3-chloro-4'-fluoro-1,1'-biphényl-4-yl)méthanol

A une solution de 3,1 g (12,4 mmoles) d'acide 3-chloro-4'-fluoro-1,1'-biphényl-4-carboxylique, préparé à l'étape 5.1., dans 50 ml de tétrahydrofurane, on ajoute goutte à goutte à température ambiante 9,3 ml (18,56 mmoles) d'une solution (2M) de complexe borane-diméthylsulfure dans le tétrahydrofurane. On poursuit l'agitation à température ambiante pendant 18 heures.

On concentre sous pression réduite et on reprend le résidu par de l'acétate d'éthyle et 100 ml d'une solution aqueuse d'acide chlorhydrique 0,1 N. On sépare la phase aqueuse puis on l'extrait deux fois avec de l'acétate d'éthyle. On réunit les phases organiques et on les lave successivement avec une solution aqueuse saturée d'hydrogénocarbonate de sodium puis une solution aqueuse saturée de chlorure de sodium. On sèche la phase organique sur sulfate de sodium et on concentre le filtrat sous pression réduite. On purifie le résidu ainsi obtenu par chromatographie sur gel de silice en éluant avec un mélange 20/80 d'acétate d'éthyle et de cyclohexane.

On obtient 1,9 g de produit pur sous forme de solide blanc. PF(°C) : 86-88°C

### 5.3. 3-chloro-4-(chlorométhyl)-4'-fluoro-1,1'-biphényle

A une solution de 1,9 g (8 mmoles) de (3-chloro-4'-fluoro-1,1'-biphényl-4-yl)méthanol, préparé à l'étape 5.2., dans 20 ml de chloroforme, on ajoute goutte à goutte à température ambiante 2,3 ml (32 mmoles) de chlorure de thionyle. On agite 18 heures à température ambiante et on concentre le filtrat à sec sous pression réduite. On co-évapore le résidu obtenu avec 50 ml de toluène.

On obtient 2 g de chlorure sous forme d'une huile utilisée telle quelle dans l'étape suivante.

### 5.4. 3-[(3-chloro-4'-fluoro-1,1'-biphényl-4-yl)méthyl]-1,3-oxazolidin-2,4-dione

On porte au reflux pendant 18 heures une solution de 0,5 g (1,96 mmole) de 3-chloro-4-(chlorométhyl)-4'-fluoro-1,1'-biphényle, préparé à l'étape 5.3., de 0,240 g (2,35 mmoles) de 1,3-oxazolidin-2,4-dione et de 0,45 g (3,92 mmoles) de 1,1,3,3-tétraméthylguanidine, dans 10 ml de tétrahydrofurane.

On laisse revenir à température ambiante et on concentre sous pression réduite. On reprend le résidu par du dichlorométhane et de l'eau, on sépare la phase aqueuse et on l'extrait deux fois avec du dichlorométhane. On lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 20/80 d'acétate d'éthyle et de cyclohexane.

On obtient 0,33 g de produit pur sous forme de solide blanc. PF (°C) : 108-110°C

### 5.5. (3-chloro-4'-fluoro-1,1'-biphényl-4-yl)méthylcarbamate de 2-(méthylamino)-2-oxoéthyle

On opère comme décrit dans l'exemple 2 (étape 2.2.). A partir de 0,33 g (0,9 mmole) de 3-[(3-chloro-4'-fluoro-1,1'-biphényl-4-yl)méthyl]-1,3-oxazolidin-2,4-dione, obtenu à l'étape 5.4., et de 1,35 ml (2,7 mmoles) d'une solution de méthylamine (2M) dans le tétrahydrofurane, on obtient 0,21 g de produit pur sous forme de solide blanc, après chromatographie sur gel de silice en éluant avec mélange 95/5 de dichlorométhane et de méthanol suivie d'une recristallisation dans l'acétate d'éthyle.
LC-MS : M+H = 351
PF(°C) : 170-172°C
RMN ¹H (CDCl₃) δ (ppm) : 2,90 (d, 3H) ; 4,50 (d, 2H) ; 4,60 (s, 2H) ; 5,40 (large s, 1H) ; 6,10 (large s, 1H) ; 7,15 (t, 2H) ; 7,40-7,70 (massif, 5H).

### Exemple 6 (Composé N°141)

### 2-(3,4'-difluoro-1,1'-biphényl-4-yl)éthylcarbamate de 2-(méthylamino)-2-oxoéthyle

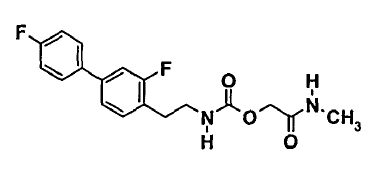

### 6.1. 3,4'-difluoro-1,1'-biphényl-4-carboxaldéhyde

On utilise la méthode décrite dans l'exemple 3 (étape 3.2.). A partir de 5,3 g (26 mmoles) de 4-bromo-2-fluorobenzaldéhyde, de 4 g (28,6 mmoles) de l'acide 4-fluorophénylboronique, de 26 ml (52 mmoles) d'une solution aqueuse de carbonate de sodium (2M) et de 0,9 g (0,78 mmole) de palladium tétrakis(triphénylphosphine), on obtient 3,4 g de produit pur sous forme de solide blanc, après chromatographie sur gel de silice en éluant avec un mélange 10/90 d'acétate d'éthyle et de cyclohexane.
PF(°C) : 98°C

### 6.2. 3,4'-difluoro-4-[(Z/E)-2-nitrovinyl]-1,1'-biphényl

On chauffe à 50°C pendant une nuit une suspension de 3,4 g (15,6 mmoles) de 3,4'-difluoro-1,1'-biphényl-4-carboxaldéhyde, préparé à l'étape 6.1., de 1,5 ml (27,3 mmoles) de nitrométhane et de 0,9 g (11,7 mmoles) d'acétate d'ammonium. On laisse revenir à température ambiante et on reprend par du dichlorométhane et de l'eau. On sépare la phase aqueuse et on l'extrait deux fois avec du dichlorométhane, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 10/90 d'acétate d'éthyle et de cyclohexane.
On obtient ainsi 2 g de produit pur sous forme d'une huile jaune.

### 6.3. 2-(3,4'-difluoro-1,1'-biphényl-4-yl)éthanamine

A une suspension de 0,90 g (23,7 mmoles) d'hydrure de lithium et d'aluminium dans 20 ml d'éther, refroidie à environ 0°C, on additionne goutte à goutte une solution de 2 g (7,7 mmoles) de 3,4'-difluoro-4-[(Z/E)-2-nitrovinyl]-1,1'-biphényl obtenu à l'étape 6.2., dans 40 ml d'un mélange de tétrahydrofurane et d'éther (1/1). On porte ensuite le mélange réactionnel à 60 °C pendant 2 heures.

On laisse revenir à température ambiante, on filtre sur papier puis on traite le filtrat par 0,9 ml d'eau et 0,9 ml d'une solution aqueuse d'hydroxyde de sodium à 15 % puis 2,7 ml d'eau. On laisse sous agitation à température ambiante pendant 1 heure. On reprend par de l'acétate d'éthyle, on sépare la phase aqueuse et on l'extrait trois fois avec de l'acétate d'éthyle, on lave les phases organiques réunies avec une solution aqueuse saturée en chlorure de sodium, on les sèche sur sulfate de sodium et on concentre le filtrat sous pression réduite. On purifie le résidu obtenu par chromatographie sur gel de silice en éluant avec un mélange 97/3/0,3 de dichlorométhane, de méthanol et d'ammoniaque à 28 %.

On obtient 0.31 g de produit sous forme d'une huile incolore.

### 6.4. [(Phényloxycarbonyl)oxy]acétate d'éthyle

A une solution de 25 g (240 mmoles) de glycolate d'éthyle et de 55 ml (315 mmoles) de diisopropyléthylamine dans 500 ml de toluène, on ajoute lentement à température ambiante 32 ml (256 mmoles) de chloroformiate de phényle. On poursuit l'agitation à température ambiante pendant 2 heures.

On sépare le sel formé et on concentre le filtrat sous pression réduite.

On obtient 53,7 g de produit huileux que l'on utilise tel quel dans l'étape suivante.

### 6.5. (((2-(3,4'-difluoro-1,1'-biphényl-4-yl)éthyl)-amino)carbonyl)oxyacétate d'éthyle

On chauffe à 60°C pendant 18 heures, une solution de 0,31 g (1,33 mmoles) de 2-(3,4'-difluoro-1,1'-biphényl-4-yl)éthanamine, préparé à l'étape 6.3., et de 0,33 g (1,46 mmole) de [(phényloxycarbonyl)oxy]acétate d'éthyle, obtenu à l'étape 6.4., dans 10 ml de toluène.

On laisse revenir à température ambiante, on sépare l'insoluble par filtration et on concentre le filtrat sous pression réduite. On reprend le résidu par du dichlorométhane, on lave les phases organiques réunies avec une solution aqueuse saturée en chlorure de sodium et on les sèche sur sulfate de sodium. Après évaporation du solvant, on purifie le résidu obtenu par chromatographie sur gel de silice en éluant avec un mélange 30/70 d'acétate d'éthyle et de cyclohexane.

On obtient ainsi 0,33 g de produit pur sous forme de solide blanc.
PF(°C) : 73-75°C

### 6.6. 2-(3,4'-difluoro-1,1'-biphényl-4-yl)éthylcarbamate de 2-(méthylamino)-2-oxoéthyle

On opère comme dans l'exemple 2 (étape 2.2.). A partir de 0,33 g (0,9 mmole) de (((2-(3,4'-difluoro-1,1'-biphényl-4-yl)éthyl)amino)carbonyl)oxyacétate d'éthyle, préparé à l'étape 6.5., et de 1,35 ml (2,7 mmoles) d'une solution de méthylamine (2M) dans le tétrahydrofurane, on obtient 0,210 g de produit pur sous forme de solide blanc, après recristallisation dans l'acétate d'éthyle.
LC-MS : M+H = 349
PF(°C) : 164-166°C
RMN ¹H (CDCl₃) δ (ppm) : 2,90 (d, 3H) ; 3,0 (t, 2H) ; 3,50 (q, 2H) ; 4,60 (s, 2H) ; 5,0 (large s, 1H) ; 6,10 (large s, 1H) ; 7,10-7,40 (massif, 5H) ; 7,55 (dd, 2H).

### Exemple 7 (Composé N°145)

### 1-(4'-fluoro-1,1'-biphényl-4-yl)cyclopropylméthyle carbamate de 2-amino-2-oxoéthyle

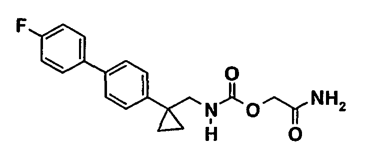

### 7.1. (4'-fluoro-1,1'-biphényl-4-yl)acétonitrile

On utilise la méthode décrite dans l'exemple 3 (étape 3.2.). A partir de 4,12 g (32,48 mmoles) de (4-bromophényl)-acétonitrile, de 5 g (35,73 mmoles) d'acide 4-fluorophénylboronique, de 32,48 ml (64,96 mmoles) d'une solution aqueuse de carbonate de sodium (2 M) et de 1,24 g (1,07 mmole) de palladium tétrakis(triphénylphosphine), on obtient 3,3 g de produit pur sous forme de solide blanc, après chromatographie sur gel de silice en éluant avec un mélange 15/85 d'acétate d'éthyle et de cyclohexane.
PF(°C) : 100-102°C

### 7.2. 1-(4'-fluoro-1,1'-biphényl-4-yl)cyclopropane-carbonitrile

A une suspension de 3,1 g (14,7 mmoles) de (4'-fluoro-1,1'-biphényl-4-yl)acétonitrile, préparé à l'étape 7.1., de 2,4 ml (29,4 mmoles) de 1-bromo-2-chloroéthane et de 0,067 g (0,294 mmole) de chlorure de N-triéthylbenzylammonium, portée à environ 50°C, on additionne goutte à goutte 6,7 g (102,8 mmoles) d'une solution aqueuse d'hydroxyde de potassium à 60%. L'agitation est poursuivie à 50°C pendant 18 heures.

On laisse revenir à température ambiante, on sépare l'insoluble par filtration et on reprend le filtrat par de l'acétate d'éthyle. On sépare la phase aqueuse et on l'extrait trois fois par de l'acétate d'éthyle. On lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 10/90 d'acétate d'éthyle et de cyclohexane.

On obtient ainsi 2,97 g de produit pur sous forme de solide blanc.
PF(°C) : 70-72°C

### 7.3. 1-[1-(4'-fluoro-1,1'-biphényl-4-yl)cyclopropyl]-méthanamine

A une solution de 2,5 g (10 mmoles) de 1-(4'-fluoro-1,1'-biphényl-4-yl)cyclopropylcarbonitrile, préparé à l'étape 7.2., dans 50 ml de tétrahydrofurane, refroidie à environ 0°C, on ajoute goutte à goutte 10 ml (10 mmoles) d'une solution d'hydrure de lithium et d'aluminium (1M) dans le tétrahydrofurane. On poursuit l'agitation à 0°C pendant 1 heure, puis à température ambiante pendant 18 heures.

On filtre sur papier puis on traite le filtrat par 0,4 ml d'eau et 0,4 ml d'une solution aqueuse d'hydroxyde de sodium à 15% puis 1,2 ml d'eau. On laisse sous agitation à température ambiante pendant 1 heure. On reprend par de l'acétate d'éthyle, on sépare la phase aqueuse et on l'extrait deux fois avec de l'acétate d'éthyle, on lave les phases organiques réunies avec une solution aqueuse saturée en chlorure de sodium, on les sèche sur sulfate de sodium et on concentre le filtrat sous pression réduite.

On obtient 2,1 g de produit sous forme de solide blanc utilisé tel quel dans l'étape suivante.
PF(°C) : 100-102°C

### 7.4. 1-((((((4'-fluoro-1,1'-biphényl-4-yl)cyclopropyl)-méthyl)amino)carbonyl)oxy)acétate d'éthyle

On utilise le mode opératoire décrit dans l'exemple 6 (étape 6.4.). A partir de 2,4 g (10 mmoles) de 1-[1-(4'-fluoro-1,1'-biphényl-4-yl)cyclopropyl]méthanamine, préparé à l'étape 7.3., et de 2,7 g (12 mmoles) de [(phényloxycarbonyl)oxy]acétate d'éthyle, préparé dans l'exemple 6 (étape 6.2.), on obtient 2,7 g de produit pur sous forme de solide blanc après chromatographie sur gel de silice en éluant avec un mélange 15/85 d'acétate d'éthyle et de cyclohexane.
PF(°C) : 96°C

### 7.5. 1-(4'-fluoro-1,1'-biphényl-4-yl)cyclopropylméthyle carbamate de 2-amino-2-oxoéthyle

A une solution de 1,4 g (3,77 mmoles) de 1-((((((4'-fluoro-1,1'-biphényl-4-yl)cyclopropyl)méthyl)amino)carbonyl)oxy)-acétate d'éthyle, obtenu à l'étape 7.4., dans 10 ml d'un mélange 1/1 de méthanol et de tétrahydrofurane, on ajoute 11 ml (75 mmoles) d'une solution d'ammoniac (7N) dans le méthanol. On poursuit l'agitation à température ambiante pendant 12 heures.

Après concentration sous pression réduite, on purifie le résidu obtenu par chromatographie sur gel de silice en éluant avec un mélange 97/3 de dichlorométhane et de méthanol suivie d'une recristallisation dans l'acétate d'éthyle.

On obtient 0,738 g de produit pur sous forme de solide blanc.
LC-MS : M+H = 343
PF(°C) : 139-141°C
RMN ¹H (CDCl₃) δ (ppm) : 1,0 (s, 4H) ; 3,50 (d, 2H) ; 4,55 (s, 2H) ; 4,90 (large s, 1H) ; 5,50 (large s, 1H) ; 5,90 (large s, 1H) ; 7,15 (t, 2H) ; 7,30-7,70 (massif, 6H).

### Exemple 8 (Composé N°197)

### 2-(3-phényloxyphényl)éthylcarbamate de 2-(méthylamino)-2-oxoéthyle

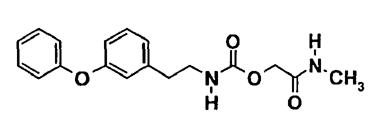

### 8.1. 2-(3-phényloxyphényl)éthanamine

On dissout 1,38 g (6,59 mmoles) de 3-phénoxyphénylacétonitrile et 1,57 g (6,59 mmoles) de chlorure de Cobalt (II) hexahydrate dans 25 ml de méthanol. On refroidit par un bain d'eau glacée et on ajoute par portion 1,74 g (46 mmoles) de borohydrure de sodium. On agite le mélange réactionnel pendant une nuit à température ambiante. On filtre sur papier et on rince par 2 fois 25 ml de méthanol. On concentre le filtrat sous pression réduite et on reprend le résidu par 50 ml d'acide chlorhydrique aqueux (1N) et 25 ml d'éther. On décante. On lave la phase aqueuse par 3 fois 25 ml d'éther. On alcalinise la phase aqueuse par 10 ml d'hydroxyde de sodium aqueux à 36 % et on extrait par 3 fois 50 ml de dichlorométhane. On lave les extraits par une solution aqueuse saturée en chlorure de sodium, on sèche sur sulfate de sodium et on concentre le filtrat sous pression réduite.

On obtient 0,67 g de produit sous forme d'huile brun-orange utilisé tel quel dans l'étape suivante.

### 8.2. (((2-(3-phényloxyphényl)éthylamino)carbonyl)oxy)-acétate d'éthyle

On chauffe à 60°C pendant une nuit un mélange de 0,66 g (3,09 mmoles) de 2-(3-phényloxyphényl)éthanamine obtenu à l'étape 8.1, et de 0,96 g (4,28 mmoles) de [(phényloxycarbonyl)oxy]acétate d'éthyle, décrit dans l'exemple 6 à l'étape 6.4, dans 15 ml de toluène. On concentre le filtrat sous pression réduite et on purifie le résidu par chromatographie sur gel de silice en éluant par un mélange 85/15 puis 70/30 de cyclohexane et d'acétate d'éthyle.

On obtient 0,80 g de produit sous forme d'huile utilisé tel quel dans l'étape suivante.

### 8.3. 2-(3-phényloxyphényl)éthylcarbamate de 2-(méthylamino)-2-oxoéthyle

On dissout 0,70 g (2,30 mmoles) de (((2-(3-phényloxyphényl)-éthylamino)carbonyl)oxy)acétate d'éthyle obtenu à l'étape 8.2, dans un mélange de 4,5 ml d'une solution (2M) de méthylamine dans le tétrahydrofurane et de 4,5 ml de méthanol. On laisse la nuit à température ambiante. On concentre le filtrat sous pression réduite et on purifie le résidu par chromatographie sur gel de silice en éluant par un mélange 98/2 puis 96/4 de dichlorométhane et de méthanol. On recristallise ensuite dans un mélange d'acétate d'éthyle et de diisopropyléther.

On obtient 0,51 g de fins cristaux blancs.
LC-MS : M+H = 329
PF(°C) : 82-84°C
RMN ¹H (DMSO-d₆) δ(ppm) : 7,4-7,25(m, 4H), 7,15(t, 1H), 7,05-6,9(m, 3H), 6,85(s, 1H), 6,1(m, 1H), 4,9(m, 1H), 4,6 (s, 2H), 3,5(q, 2H), 2,9-2,85(m, 5H).

### Exemple 9 (Composé N°81)

### 4-pyridin-2-ylbenzylcarbamate de 2-(méthylamino)-2-oxoéthyle

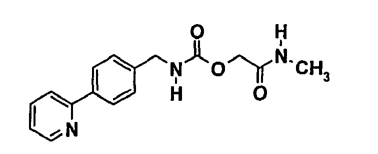

### 9.1. 3-(4-bromobenzyl)-1,3-oxazolidin-2,4-dione

A une solution de 1,50 g (6 mmoles) de bromure de 4-bromobenzyle et de 0,73 g (7,2 mmoles) de 1,3-oxazolidin-2,4-dione dans 6 ml de tétrahydrofurane, on ajoute goutte à goutte une solution de 1,39 g (12 mmoles) de 1,1,3,3-tetraméthylguanidine dans 6 ml de tétrahydrofurane. On agite le mélange à température ambiante pendant une nuit. On ajoute 50 ml d'acide chlorhydrique aqueux (1N) glacé et 100 ml d'acétate d'éthyle. On décante la phase organique et on la lave successivement par 25 ml d'eau et 25 ml d'une solution aqueuse saturée en chlorure de sodium. On la sèche sur sulfate de sodium et on concentre le filtrat sous pression réduite. On purifie le résidu par chromatographie sur gel de silice en éluant avec un mélange 80/20 de cyclohexane et d'acétate d'éthyle. On obtient 1,14 g de produit sous forme de cristaux blancs.
PF(°C) : 88-90°C

### 9.2. 3-(4-pyridin-2-ylbenzyl)-1,3-oxazolidin-2,4-dione

Sous atmosphère d'argon, on chauffe à reflux pendant une nuit un mélange de 0,59 g (2,18 mmoles) de 3-(4-bromobenzyl)-1,3-oxazolidin-2,4-dione obtenu à l'étape 9.1, 1,60 g (4,35 mmoles) de pyridin-2-*tri*-n-butylstannane, de 0,28 g (6,6 mmoles) de chlorure de lithium et de 0,125 g (0,10 mmole) de palladium tetrakis(triphénylphosphine) dans 15 ml de toluène. On refroidit à température ambiante, on filtre sur papier et on rince successivement par 10 ml de toluène, 10 ml d'acétate d'éthyle et 10 ml de toluène. On concentre les filtrats sous pression réduite. On reprend le résidu dans 50 ml d'acétonitrile et on le lave par 4 fois 25 ml de n-hexane. On concentre sous pression réduite la phase acétonitrile et on purifie le résidu par chromatographie sur gel de silice en éluant par un mélange 70/30 puis 60/40 de cyclohexane et d'acétate d'éthyle.

On obtient 0,428 g de produit sous forme de poudre blanche PF(°C) : 166°C

### 9.3. 4-pyridin-2-ylbenzylcarbamate de 2-(méthylamino)-2-oxoéthyle

On dissout 0,42 g (1,56 mmole) de 3-(4-pyridin-2-ylbenzyl)-1,3-oxazolidin-2,4-dione obtenu à l'étape 9.2, dans un mélange de 3,5 ml d'une solution (2M) de méthylamine dans le tétrahydrofurane et de 3,5 ml de méthanol. On laisse la nuit à température ambiante. On ajoute 1,5 g de silice, on concentre à sec sous pression réduite et on purifie par chromatographie sur gel de silice en éluant par un mélange 94/6 puis 93/7 de dichlorométhane et de méthanol. On recristallise le produit dans un mélange d'isopropanol et de diisopropyléther.

On obtient 0,30 g de produit sous forme de paillettes blanches.
LC-MS :M+H = 300
PF(°C) : 151-153°C
RMN ¹H (DMSO-d₆) δ(ppm) : 8,6(d,1H), 8,05(d, 2H), 7,95-7,7(m,4H dont 2 échangeables au D₂O), 7,35(d,2H), 7,3(m,1H), 4,35(s,2H), 4,25(d,2H,s à l'échange au D₂O), 2,6(d,3H,s à l'échange au D₂O).

### Exemple 10 (Composé N°98)

### 4-isoquinolin-4-ylbenzylcarbamate de 2-amino-2-oxoéthyle.

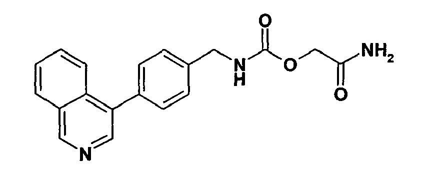

### 10.1. (4-isoquinolin-4-ylphényl)méthanol

Sous atmosphère d'argon, on chauffe au reflux pendant une nuit un mélange de 1,09 g (7,2 mmoles) d'acide (4-hydroxyméthyl)phénylboronique, de 1,24 g (6 mmoles) de 4-bromoisoquinoline et de 0,28 g (0,24 mmole) de palladium tetrakis-(triphénylphosphine) dans 50 ml de toluène et 10 ml d'une solution aqueuse (2M) de carbonate de sodium. On concentre le filtrat sous pression réduite et on reprend le résidu dans 150 ml d'acétate d'éthyle et 40 ml d'eau. On décante et on lave la phase organique successivement par 20 ml d'eau et 20 ml d'une solution aqueuse saturée de chlorure de sodium. On sèche sur sulfate de sodium et on concentre le filtrat sous pression réduite. On purifie le résidu par chromatographie sur gel de silice en éluant par un mélange 60/40 puis 40/60 de cyclohexane et d'acétate d'éthyle. On obtient 1,12 g de solide blanc.
PF(°C) : 130°C

### 10.2. 3-(4-isoquinolin-4-ylbenzyl)-1,3-oxazolidin-2,4-dione

On dissout 1,10 g (4,67 mmoles) de (4-isoquinolin-4-yl-phényl)méthanol obtenu à l'étape 10.1, dans 10 ml de chloroforme et on ajoute goutte à goutte 1,4 ml (19 mmoles) de chlorure de thionyle. On agite une nuit à température ambiante et on concentre le filtrat à sec sous pression réduite. On co-évapore le résidu avec 2 fois 10 ml de dichloroéthane. On reprend le résidu dans 15 ml de tétrahydrofurane. On ajoute 0,56 g (5,54 mmoles) de 1,3-oxazolidin-2,4-dione puis goutte à goutte une solution de 1,60 g (13,9 mmoles) de 1,1,3,3-tetraméthylguanidine dans 5 ml de tétrahydrofurane. On chauffe au reflux pendant une nuit. On refroidit à température ambiante. On ajoute 20 ml d'eau glacée et 100 ml d'acétate d'éthyle. On décante. On lave la phase organique par 3 fois 10 ml d'eau et 20 ml d'une solution aqueuse saturée de chlorure de sodium. On sèche sur sulfate de sodium et on concentre le filtrat sous pression réduite. On purifie le résidu par chromatographie sur gel de silice en éluant par un mélange 50/50 puis 40/60 de cyclohexane et d'acétate d'éthyle. On obtient 0,84 g de produit sous forme de mousse jaune solide.
PF(°C) : 65°C

### 10.3. 4-isoquinolin-4-ylbenzylcarbamate de 2-amino-2-oxoéthyle

On dissout 0,34 g (1,06 mmole) de 3-(4-isoquinolin-4-ylbenzyl)-1,3-oxazolidin-2,4-dione obtenu à l'étape 10.2, dans un mélange de 6 ml d'une solution (7N) d'ammoniac dans le méthanol et de 6 ml de tétrahydrofurane. On laisse la nuit à température ambiante. On ajoute 10 ml de dichlorométhane, 1 g de silice, on concentre à sec sous pression réduite et on purifie par chromatographie sur gel de silice en éluant par un mélange 95/5 puis 92/8 de dichlorométhane et de méthanol. On recristallise dans un mélange d'isopropanol et de diisopropyléther.

On obtient 0,26 g de produit sous forme de coton blanc.
LC-MS : M+H = 336
PF(°C) : 181-183°C
RMN ¹H (DMSO-d₆) δ(ppm) : 9,3(s,1H), 8,4(s,1H), 8,2(d,1H), 7,9-7,(m,4H, dont 1 échangeable au D₂O), 7,5(s,4H), 7,3(s,1H, échangeable au D₂O, 7,2(s,1H), 4,4(s,2H), 4,35(d,2H, s à l'échange au D₂O).

### Exemple 11 (composé n° 171)

### 2-(3'-cyano-1,1'-biphényl-4-yl)éthylcarbamate de 2-(méthylamino)-2-oxoéthyle

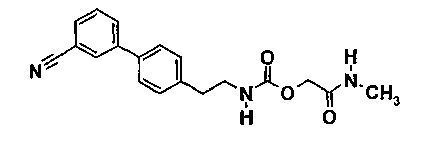

### 11.1. 4'-(2-hydroxyéthyl)-3-biphénylcarbonitrile

On chauffe à 100°C sous atmosphère d'argon pendant une nuit, un mélange de 3 g (14,92 mmoles) de 2-(4-bromophényl)éthanol, 2,85 g (19,40 mmoles) d'acide 3-cyanophénylboronique, 5,15 g (37,30 mmoles) de carbonate de potassium, 4,81 g (14,92 mmoles) de bromure de tetra-n-butylammonium et de 0,067 g (0,30 mmoles) de diacétate de palladium dans 15 ml d'eau. On refroidit à température ambiante, on dilue par de l'eau et on extrait avec de l'acétate d'éthyle. On sèche la phase organique sur sulfate de sodium et on évapore. On purifie ensuite par chromatographie sur gel de silice en éluant par un mélange 70/30 puis 50/50 de cyclohexane et d'acétate d'éthyle pour obtenir 2,90 g de produit sous forme d'huile utilisé tel quel dans l'étape suivante.

### 11.2. 4'-[2-(2,4-dioxo-1,3-oxazolidin-3-yl)éthyl]-3-biphénylcarbonitrile

A une solution de 2,90 g (12,99 mmoles) de 4'-(2-hydroxyéthyl)-3-biphénylcarbonitrile,préparé à l'étape 11.1., de 2,7 ml (14,29 mmoles) de triéthylamine et de 0,15 g (1,30 mmoles) de 4-diméthylaminopyridine dans 30 ml de dichlorométhane, refroidie par un bain de glace, on ajoute 1,1 ml (14,29 mmoles) de chlorure de méthanesulfonyle. On agite ensuite 2 heures à température ambiante. On ajoute 100 ml de dichlorométhane et 30 ml d'une solution aqueuse saturée en chlorure de sodium. On décante la phase organique, on la sèche sur sulfate de sodium et on évapore à sec pour obtenir 3,5 g de produit sous forme d'huile.

On redissout le produit dans 60 ml de tétrahydrofurane et on ajoute 1,40 g 13,94 mmoles) de 1,3-oxazolidine-2,4-dione et 2,87 ml (23,23 mmoles) de 1,1,3,3-tétraméthylguanidine. On chauffe à 70°C pendant une nuit. On évapore à sec. On reprend le résidu dans un mélange d'acétate d'éthyle et d'une solution aqueuse saturée en chlorure de sodium. On décante la phase organique, on la sèche sur sulfate de sodium et on évapore à sec. On purifie par chromatographie sur gel de silice en éluant par un mélange 60/40 puis 50/50 de cyclohexane et d'acétate d'éthyle pour obtenir 3,3 g de produit sous forme d'un solide blanc
Point de fusion (°C) : 121-123

### 11.3. 2-(3'-cyano-1,1'-biphényl-4-yl)éthylcarbamate de 2-(méthylamino)-2-oxoéthyle

On dissout 2,0 g (6,53 mmoles) de 4'-[2-(2,4-dioxo-1,3-oxazolidin-3-yl)éthyl]-3-biphénylcarbonitrile, préparé à l'étape 11.2., dans un mélange de 13 ml de méthanol et de 9,8 ml d'une solution 2N de méthylamine (19,6 mmoles) dans le tétrahydrofurane. On laisse réagir une nuit puis on évapore à sec et on purifie par chromatographie sur gel de silice en éluant par un mélange 96/4 puis 95/5 de dichlorométhane et de méthanol. On recristallise dans un mélange d'acétate d'éthyle et de diisopropyléther pour obtenir 1,07 g de produit sous forme de poudre blanche Point de fusion (°C) : 157-159
LC-MS : M+H = 338
RMN ¹H (CDCl₃) δ(ppm) : 7,85 (s,1H), 7,80 (dt, 1H), 7,65-7,50 (m,4H), 7,35 (d,2H), 6,05 (s large, 1H), 4,95 (s large, 1H), 4,60 (s,2H), 3,55 (m,2H), 2,95 (t,2H), 2,85 (d,3H)

### Exemple 12 (composé n°84)

### [4-(4-phényl-1H-imidazol-1-yl)phényl]méthylcarbamate de 2-amino-2-oxoéthyle

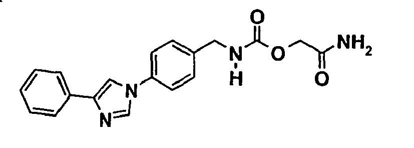

### 12.1. [4-(4-phényl-1H-imidazol-1-yl)phényl]méthanol

On dissout dans 20 ml de diméthylformamide 3,04 g (20 mmoles) d'acide 4-(hydroxyméthyl)phénylboronique, 1,44 g (10 mmoles) de 4-phénylimidazole, 2,8 ml (20 mmoles) de triéthylamine et 1,64 ml (20 mmoles) de pyridine. On ajoute 2,72 g (15 mmoles) de diacétate de cuivre et on agite 24 heures à température ambiante. On dilue par 200 ml de dichlorométhane et 200 ml d'une solution aqueuse d'ammoniaque à 28%. On décante et on extrait la phase aqueuse par 100 ml de dichlorométhane. On lave les phases organiques par 50 ml d'une solution aqueuse saturée en chlorure de sodium, on les sèche sur sulfate de sodium et on les évapore à sec. On purifie le produit par chromatographie sur gel de silice en éluant par un mélange 97/3 puis 95/5 de dichlorométhane et de méthanol. On recristallise dans un mélange de toluène et de diisopropyléther pour obtenir 1,82 g de produit sous forme de cristaux blancs.
Point de fusion ('C) : 137-139

### 12.2. [4-(4-phényl-1H-imidazol-1-yl)phényl]méthylcarbamate de 2-amino-2-oxoéthyle

A une solution de 1,0 g (4 moles) de [4-(4-phényl-1*H-*imidazol-1-yl)phényl]méthanol, préparé à l'étape 12.1., de 0,485 g (4,80 mmoles) de 1,3-oxazolidine-2,4-dione et de 1,15 g (4,38 mmoles) de triphénylphosphine dans 16 ml de tétrahydrofurane, refroidie par un bain d'acétone et de glace, on ajoute goutte à goutte 0,80 g (4 mmoles) de azodicarboxylate de diisopropyle dissous dans 2 ml de tétrahydrofurane. On réchauffe ensuite à température ambiante et on agite une nuit. On prélève 9 ml de la solution et on y ajoute 12 ml d'une solution 7N d'ammoniac (84 mmoles) dans le méthanol. On laisse réagir une nuit, on ajoute 4 g de silice et on évapore à sec. On purifie le produit par chromatographie sur gel de silice en éluant par un mélange 95/5 puis 93/7 et 90/10 de dichlorométhane et de méthanol. On recristallise dans un mélange de méthanol et de diisopropyléther pour obtenir 0,429 g de produit sous forme de cristaux blancs.
Point de fusion (°C) : 200-203
LC-MS : M+H = 351
RMN ¹H (DMSO) δ(ppm) : 8,30 (s, 1H), 8,20 (s, 1H), 7,80 (d+m,3H), 7,65 (d,2H), 7,45-7,20 (m,7H), 4,35 (s,2H), 4,25 (d,2H)

### Exemple 13 (composé n°224)

### 2-[4-1H-pyrrolo[2,3-b]pyridin-1-yl)phényl]éthylcarbamate de 2-amino-2-oxoéthyle

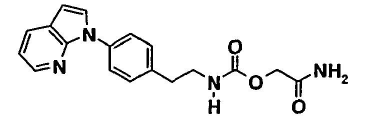

### 13.1. 2-[4-(1H-pyrrolo[2,3-b]pyridin-1-yl)phényl]éthanol

On chauffe à 80°C, sous bonne agitation et atmosphère d'argon, pendant une nuit, un mélange de 1,24 g (5 mmoles) de 2-(4-iodophényl)éthanol, de 0,62 g (5,25 mmoles) de 7-azaindole, de 2,33 g (11,0 mmoles) de phosphate de potasium, de 0,082 g (1,0 mmoles) de N,N'-diméthyléthylènediamine et de 0,095 g (0,50 mmoles) d'iodure cuivreux dans 4 ml de toluène. On refroidit à température ambiante et on dilue par 150 ml d'acétate d'éthyle et 50 ml d'eau. On décante et on lave la phase organique par 25 ml d'eau et 25 ml d'une solution aqueuse saturée en chlorure de sodium. On la sèche sur sulfate de sodium et on évapore à sec. On purifie par chromatographie sur gel de silice en éluant par un mélange 70/30 puis 60/40 et 50/50 de cyclohexane et d'acétate d'éthyle pour obtenir 1,05 g de produit sous forme d'huile.

### 13.2. 3-{2-[4-(1H-pyrrolo[2,3-b]pyridin-1-yl)phényl]éthyl}-1,3-oxazolidine-2,4-dione

A une solution de 1,0 g (4,20 mmoles) de 2-[4-(1*H-*pyrrolo[2,3-*b*]pyridin-1-yl)phényl]éthanol, préparé à l'étape 13.1., de 0,51 g (5,04 mmoles) de 1,3-oxazolidine-2,4-dione et de 1,21 g (4,62 mmoles) de triphénylphosphine dans 16 ml de tetrahydrofurane, refroidie par un bain d'acétone et de glace, on ajoute goutte à goutte 0,84 g (4,2 mmoles) de azodicarboxylate de diisopropyle dissous dans 2 ml de tétrahydrofurane. On réchauffe ensuite à température ambiante et on agite une nuit. On ajoute 4 g de silice et on évapore à sec. On purifie par chromatographie sur gel de silice en éluant par un mélange 70/30 puis 60/40, 50/50 et 40/60 de cyclohexane et d'acétate d'éthyle pour obtenir 1,52 g de produit sous forme de solide collant, utilisé tel quel dans l'étape suivante.

### 13.3. 2-[4-1H-pyrrolo[2,3-b]pyridin-1-yl)phényl]éthylcarbamate de 2-amino-2-oxoéthyle

On dissout 0,80 g (2,49 mmoles) de 3-12-[4-(1*H-*pyrrolo[2,3-*b*]pyridin-1-yl)phényl]éthyl}-1,3-oxazolidine-2,4-dione, préparé à l'étape 13.2., dans 6 ml de tetrahydrofurane et on ajoute 12 ml d'une solution d'ammoniac 7N (84 mmoles) dans le méthanol. On laisse réagir une nuit à température ambiante. On ajoute 2,5 g de silice et on évapore à sec. On purifie par chromatographie sur gel de silice en éluant par un mélange 98/2 puis 96/4 et 94/6 de dichlorométhane et de méthanol. On recristallise dans un mélange d'acétate d'éthyle et de diisopropyléther pour obtenir 0,478 g de produit sous forme de paillettes blanches.
Point de fusion (°C) : 110-112
LC-MS : M+H = 339
RMN ¹H (DMSO) δ(ppm) : 8,30 (dd,1H), 8,05 (d,1H), 7,90 (d,1H), 7,80 (d,2H), 7,30 (d+m,3H), 7,25-7,10 (m,3H), 6,70 (d,1H), 4,30 (s,2H), 3,25 (t large,2H), 2,80 (t,2H)

### Exemple 14 (composé n°196)

### 2-{4-[(4-chlorophényl)oxy]phényl}éthylcarbamate de 2-(méthylamino)-2-oxoéthyle

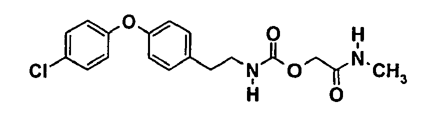

### 14.1. 2-(4-[(4-chlorophényl)oxy]phényl)éthanol

On chauffe à 90°C, sous bonne agitation et atmosphère d'argon, pendant 24 heures, un mélange de 1,14 g (4,60 mmoles) de 2-(4-iodophényl)éthanol, 0,88 g (6,89 mmoles) de 4-chlorophénol, 2,99 g (9,20 mmoles) de carbonate de césium, 0,14 g (1,38 mmoles) de N,N-diméthylglycine et 0,087 g (0,46 mmoles) d'iodure cuivreux dans 4 ml de dioxane. On refroidit à température ambiante et on reprend par 150 ml d'acétate d'éthyle et 50 ml d'eau. On décante, on lave la phase organique par 25 ml d'eau et 25 ml d'une solution aqueuse saturée en chlorure de sodium, on la sèche sur sulfate de magnésium et on évapore à sec. On purifie par chromatographie sur gel de silice en éluant par un mélange 85/15 puis 75/25 de cyclohexane et d'acétate d'éthyle pour obtenir 0,68 g de produit sous forme d'huile.

### 14.2. 3-(2-(4-[(4-chlorophényl)oxy]phényl)éthyl)-1,3-oxazolidine-2,4-dione

A une solution de 1,0 g (4,02 mmoles) de 2-(4-[(4-chlorophényl)oxy]phényl)éthanol, préparé suivant l'exemple 14.1. et de 1,1 ml (7 ,89 mmoles) de triéthylamine dans 12 ml de dichlorométhane, refroidie par un bain de glace, on ajoute une solution de 0,60 g (5,24 mmoles) de chlorure de méthanesulfonyle dans 2 ml de dichlorométhane. On agite ensuite 2 heures à température ambiante. On dilue par 25 ml d'eau et 75 ml de dichlorométhane. On décante, on lave la phase organique par 25 ml d'eau puis 25 ml d'une solution aqueuse saturée en chlorure de sodium, on la sèche sur sulfate de sodium et on l'évapore à sec pour obtenir 1,32 g de produit sous forme d'huile.

On redissout le produit dans 12 ml de tétrahydrofurane. On ajoute 0,50 g (5 mmoles) de 1,3-oxazolidine-2,4-dione et une solution de 0,92 g (8,0 mmoles) de 1,1,3,3-tétraméthylguanidine en solution dans 4 ml de tétrahydrofurane. On chauffe ensuite à reflux pendant une nuit. On refroidit par un bain de glace et on ajoute 25 ml d'une solution aqueuse 0,1N d'acide chlorhydrique et 100 ml d'acétate d'éthyle. On décante la phase organique, on la lave par 2 fois 25 ml d'eau puis par 25 ml d'une solution aqueuse saturée en chlorure de sodium, on la sèche sur sulfate de sodium et on l'évapore à sec. On purifie par chromatographie sur gel de silice en éluant par un mélange 85/15 puis 75/25 et 65/35 de cyclohexane et d'acétate d'éthyle pour obtenir 1,20 g de produit sous forme de solide blanc.
Point de fusion (°C) : 105-107

### 14.3. 2-{4-[(4-chlorophényl)oxy]phényl}éthylcarbamate de 2-(méthylamino)-2-oxoéthyle

On redissout 0,46 g (1,39 mmoles) de 3-(2-(4-[(4-chlorophényl)oxy]phényl)éthyl)-1,3-oxazolidine-2,4-dione, préparé à l'étape 14.2., dans un mélange de 3 ml de tétrahydrofurane et de 6 ml de méthanol. On ajoute 3 ml d'une solution 2M de méthylamine (6 mmoles) dans le tetrahydrofurane. On laisse réagir une nuit à température ambiante puis on ajoute 2 g de silice et on évapore à sec. On purifie par chromatographie sur gel de silice en éluant par un mélange 98/2 puis 96/4 et 94/6 de dichlorométhane et de méthanol. On recristallise dans un mélange d'acétate d'éthyle et de diisopropyléther pour obtenir 0,40 g de produit sous forme de poudre blanche.
Point de fusion (°C) : 133-135
LC-MS : M+H = 363
RMN ¹H (DMSO) δ(ppm) : 7,70 (m, 1H), 7,40 (d, 2H) , 7,25 (d+m, 3H), 6,95 (m,4H), 4,30 (s,2H), 3,25 (m,2H), 2,70 (t,2H), 2,55 (d,3H)

Le tableau 1 qui suit illustre les structures chimiques et les propriétés physiques de quelques composés selon l'invention.

Dans ce tableau :
- tous les composés sont sous forme de base libre,
- i-propyl, n-butyle et t-butyle représentent respectivement des groupes isopropyle, butyle linéaire et tertiobutyle.

**Tableau 1**

| | | | | | |
|---|---|---|---|---|---|
| 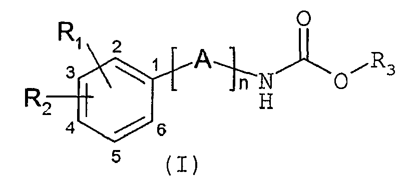 | | | | | |

| N° | [A]ₙ | R₁ | R₂ | R₃ | PF(°C) |
|---|---|---|---|---|---|
| 1. | CH₂ | H | 4-phényl | CH₂CONHCH₃ | 189-190 |
| 2. | CH₂ | H | 3-phényl | CH₂CONHCH₃ | 128-129 |
| 3. | CH₂ | H | 4-phényl | CH₂CONH₂ | 222-223 |
| 4. | CH₂ | H | 4-(2-F-phényl) | CH₂CONH₂ | 176-177 |
| 5. | CH₂ | H | 3-(2-F-phényl) | CH₂CONH₂ | 113-114 |
| 6. | CH₂ | H | 4-(3-F-phényl) | CH₂CONH₂ | 201-202 |
| 7. | CH₂ | H | 3-(3-F-phényl) | CH₂CONH₂ | 89-90 |
| 8. | CH₂ | H | 4-(4-F-phényl) | CH₂CONH₂ | 226-227 |
| 9. | CH₂ | 2-Cl | 4-(4-F-phényl) | CH₂CONHCH₃ | 170-172 |
| 10. | CH(CH₃) | H | 4-(4-F-phényl) | CH₂CONH₂ | 179-181 |
| 11. | CH₂ | H | 3-(4-F-phényl) | CH₂CONH₂ | 100-101 |
| 12. | CH₂ | H | 4-(2-Cl-phényl) | CH₂CONH₂ | 148-149 |
| 13. | CH₂ | H | 4-(3-Cl-phényl) | CH₂CONH₂ | 187-188 |
| 14. | CH₂ | H | 4-(4-Cl-phényl) | CH₂CONH₂ | 216-218 |
| 15. | CH₂ | H | 4-(2-CF₃-phényl) | CH₂CONH₂ | 178-179 |
| 16. | CH₂ | H | 4-(3-CF₃-phényl) | CH₂CONH₂ | 153-154 |
| 17. | CH₂ | H | 4-(4-CF₃-phényl) | CH₂CONH₂ | 213-215 |
| 18. | CH₂ | H | 4-(2-CF₃O-phényl) | CH₂CONH₂ | 177-179 |
| 19. | CH₂ | H | 4-(3-CF₃O-phényl) | CH₂CONH₂ | 167-168 |
| 20. | CH₂ | H | 4-(4-CF₃O-phényl) | CH₂CONH₂ | 218-220 |
| 21. | CH₂ | H | 4-(4-CN-phényl) | CH₂CONH₂ | 221-222 |
| 22. | CH₂ | H | 4-(3-CN-phényl) | CH₂CONH₂ | 126-127 |
| 23. | CH₂ | H | 4-(2-CH₃CO-phényl) | CH₂CONH₂ | 184-185 |
| 24. | CH₂ | H | 4-(3-CH₃CO -phényl) | CH₂CONH₂ | 142-145 |
| 25. | CH₂ | H | 4-(4-CH₃CO -phényl) | CH₂CONH₂ | 231-233 |
| 26. | CH₂ | H | 4-(4-CH₃SO₂-phényl) | CH₂CONH₂ | 233-235 |
| 27. | CH₂ | H | 4-(4-CH₃CONH - phényl) | CH₂CONH₂ | 342* |
| 28. | CH₂ | H | 4-(3-CH₃CONH - phényl) | CH₂CONH₂ | 189-190 |
| 29. | CH₂ | H | 3-(3-CH₃CONH - phényl) | CH₂CONH₂ | 144-145 |
| 30. | CH₂ | H | 4-(3-CH₃-phényl) | CH₂CONH₂ | 184-185 |
| 31. | CH₂ | H | 4-(4-CH₃-phényl) | CH₂CONH₂ | 229-231 |
| 32. | CH₂ | H | 4-(4-C₂H₅-phényl) | CH₂CONH₂ | 239-240 |
| 33. | CH₂ | H | 4-(3-i-propyl-phényl) | CH₂CONH₂ | 154-155 |
| 34. | CH₂ | H | 4-(4-i-propyl-phényl) | CH₂CONH₂ | 223-224 |
| 35. | CH₂ | H | 4-(4-t-butyl-phényl) | CH₂CONH₂ | 189-190 |
| 36. | CH₂ | H | 4-(4-n-butyl-phényl) | CH₂CONH₂ | 222-223 |
| 37. | CH₂ | H | 4-(3-phényl-phényl) | CH₂CONH₂ | 182-183 |
| 38. | CH₂ | H | 4-(2-CH₃O-phényl) | CH₂CONH₂ | 153-154 |
| 39. | CH₂ | H | 4-(2-CH₃S-phényl) | CH₂CONH₂ | 128-129 |
| 40. | CH₂ | H | 3-(2-CH₃O-phényl) | CH₂CONH₂ | 148-149 |
| 41. | CH₂ | H | 4-(3-CH₃O-phényl) | CH₂CONH₂ | 140-141 |
| 42. | CH₂ | H | 3-(3-CH₃O-phényl) | CH₂CONH₂ | 315* |
| 43. | CH₂ | H | 4-(4-CH₃O-phényl) | CH₂CONH₂ | 229-230 |
| 44. | CH₂ | H | 3-(4-CH₃O-phényl) | CH₂CONH₂ | 134-135 |
| 45. | CH₂ | H | 4-(3-C₂H₅O-phényi) | CH₂CONH₂ | 234-236 |
| 46. | CH₂ | H | 4-(4-C₂H₅O-phényl) | CH₂CONH₂ | 233-234 |
| 47. | CH₂ | H | 4-(3-benzyloxyphényl) | CH₂CONH₂ | 175-176 |
| 48. | CH₂ | H | 4-(4-benzyloxyphényl) | CH₂CONH₂ | 229-231 |
| 49. | CH₂ | H | 4-(2-F,5-F-phényl) | CH₂CONH₂ | 180-182 |
| 50. | CH₂ | H | 4-(3-F,4-F-phényl) | CH₂CONH₂ | 236-237 |
| 51. | CH₂ | H | 4-(3-F,5-F-phényl) | CH₂CONH₂ | 174-176 |
| 52. | CH₂ | H | 4-(2-Cl,3-Cl-phényl) | CH₂CONH₂ | 170-171 |
| 53. | CH₂ | H | 4-(2-Cl,4-Cl-phényl) | CH₂CONH₂ | 116-117 |
| 54. | CH₂ | H | 4-(2-Cl,5-Cl-phényl) | CH₂CONH₂ | 119-122 |
| 55. | CH₂ | H | 4-(3-Cl,4-Cl-phényl) | CH₂CONH₂ | 173-176 |
| 56. | CH₂ | H | 4-(3-Cl,5-Cl-phényl) | CH₂CONH₂ | 161-162 |
| 57. | CH₂ | H | 4-(2-F,3-CH₃O phényl) | CH₂CONH₂ | 114-115 |
| 58. | CH₂ | H | 4-(3-F,4-CH₃O-phényl) | CH₂CONH₂ | 225-226 |
| 59. | CH₂ | H | 4-(4-F,3-CH₃-phényl) | CH₂CONH₂ | 201-202 |
| 60. | CH₂ | H | 4-(4-F,3-Cl-phényl) | CH₂CONH₂ | 158-159 |
| 61. | CH₂ | H | 4-(2,4-(CH₃O)₂-phényl) | CH₂CONH₂ | 166-167 |
| 62. | CH₂ | H | 4-(2,5-(CH₃O)₂-phényl) | CH₂CONH₂ | 132-133 |
| 63. | CH₂ | H | 4-(3,4-OCH₂O-phényl) | CH₂CONH₂ | 329* |
| 64. | CH₂ | H | 4-(3,4-(CH₃)₂-phényl) | CH₂CONH₂ | 190-191 |
| 65. | CH₂ | H | 4-(3-CF₃,5-CF₃-phényl) | CH₂CONH₂ | 176-177 |
| 66. | CH₂ | H | 4-(5-Cl,2-CH₃O-phényl) | CH₂CONH₂ | 145-146 |
| 67. | CH₂ | H | 4-phényloxy | CH₂CONH₂ | 166-168 |
| 68. | CH₂ | H | 4-phényloxy | CH₂CONHCH₃ | 128-130 |
| 69. | CH₂ | H | 4-(4-Cl-phényloxy) | CH₂CONH₂ | 184-186 |
| 70. | CH₂ | H | 4-(4-Cl-phényloxy) | CH₂CONHCH₃ | 159-161 |
| 71. | CH₂ | H | 3-phényloxy | CH₂CONH₂ | 106-107 |
| 72. | CH₂ | H | 3-phényloxy | CH₂CONHCH₃ | 100-102 |
| 73. | CH₂ | H | 3-(4-Cl-phényloxy) | CH₂CONH₂ | 110-112 |
| 74. | CH₂ | H | 3-(4-Cl-phényloxy) | CH₂CONHCH₃ | 85-87 |
| 75. | CH₂ | H | 4-benzoyl | CH₂CONH₂ | 161-163 |
| 76. | CH₂ | H | 4-benzoyl | CH₂CONHCH₃ | 149-151 |
| 77. | CH₂ | H | 3-benzoyl | CH₂CONHCH₃ | 91-93 |
| 78. | CH₂ | H | 4-phénylsulfonyl | CH₂CONH₂ | 88-90 |
| 79. | CH₂ | H | 4-phénylsulfonyl | CH₂CONHCH₃ | 363* |
| 80. | CH₂ | H | 4-(pyridin-3-yl) | CH₂CONH₂ | 160-162 |
| 81. | CH₂ | H | 4-(pyridin-2-yl) | CH₂CONHCH₃ | 151-153 |
| 82. | CH₂ | H | 4-(pyrazin-2-yl) | CH₂CONHCH₃ | 186-189 |
| 83. | CH₂ | H | 4-(thién-3-yl) | CH₂CONH₂ | 311-312 |
| 84. | CH₂ | H | 4-phénylimidazol-1-yl | CH₂CONH₂ | 200-203 |
| 85. | CH₂ | H | 4-phénylimidazol-1-yl | CH₂CONHCH₃ | 191-193 |
| 86. | CH₂ | H | 3-phénylimidazol-1-yl | CH₂CONH₂ | 170-172 |
| 87. | CH₂ | H | 4-(4-CH₃-thién-2-yl) | CH₂CONH₂ | 203-204 |
| 88. | CH₂ | H | 4-(benzo[b]thién-3-yl) | CH₂CONH₂ | 144-145 |
| 89. | CH₂ | H | 4-(3,5-(CH₃)₂-isoxazol-4-yl) | CH₂CONH₂ | 164-165 |
| 90. | CH₂ | H | 4-(1,2,3-thiadiazol-4-yl) | CH₂CONHCH₃ | 185-187 |
| 91. | CH₂ | H | 4-(dibenzo[b,d]furan-2-yl) | CH₂CONH₂ | 194-195 |
| 92. | CH₂ | H | 4-(2-phényl-éthylèn-1-yl) | CH₂CONH₂ | 236-238 |
| 93. | CH₂ | H | 4-(naphtalèn-1-yl) | CH₂CONH₂ | 154-156 |
| 94. | CH₂ | H | 4-(4-CH₃-naphtalèn-1-yl) | CH₂CONH₂ | 114-115 |
| 95. | CH₂ | H | 4-(naphtalèn-2-yl) | CH₂CONH₂ | 240-241 |
| 96. | CH₂ | H | 4-(quinolin-8-yl) | CH₂CONH₂ | 140-142 |
| 97. | CH₂ | H | 4-(isoquinolin-1-yl) | CH₂CONH₂ | 180-183 |
| 98. | CH₂ | H | 4-(isoquinolin-4-yl) | CH₂CONH₂ | 181-183 |
| 99. | CH₂ | H | 4-(isoquinolin-4-yl) | CH₂CONHCH₃ | 187-189 |
| 100. | CH₂ | H | 4-(benzimidazol-1-yl) | CH₂CONH₂ | 208-211 |
| 101. | CH₂ | H | 4-(pyrrolo[2,3-b]pyridinyl) | CH₂CONH₂ | 113-116 |
| 102. | CH₂ | H | 3-( pyrrolo[2,3-b]pyridinyl) | CH₂CONH₂ | 130-132 |
| 103. | CH₂CH₂ | H | H | CH₂CONH₂ | 130-131 |
| 104. | (CH₂)₃ | H | H | CH₂CONH₂ | 113-114 |
| 105. | (CH₂)₃ | H | 4-phényl | CH₂CONH₂ | 187-189 |
| 106. | (CH₂)₃ | H | 3-phényl | CH₂CONH₂ | 151-153 |
| 107. | (CH₂)₄ | H | H | CH₂CONH₂ | 251* |
| 108. | (CH₂)₄ | H | 4-phényl | CH₂CONH₂ | 171-173 |
| 109. | (CH₂)₄ | H | 3-phényl | CH₂CONH₂ | 127-129 |
| 110. | (CH₂)₅ | H | H | CH₂CONHCH₃ | 86-88 |
| 111. | (CH₂)₅ | H | 4-phényl | CH₂CONH₂ | 225-227 |
| 112. | (CH₂)₅ | H | 3-phényl | CH₂CONH₂ | 135-137 |
| 113. | (CH₂)₆ | H | H | CH₂CONH₂ | 109-111 |
| 114. | (CH₂)₆ | H | H | CH₂CONHCH₃ | 70-72 |
| 115. | (CH₂)₇ | H | H | CH₂CONHCH₃ | 83-85 |
| 116. | 4-cyclohexyl -(CH₂)₂- | H | H | CH₂CONH₂ | 141-142 |
| 117. | CH₂CH₂ | H | 2-Cl | CH₂CONH₂ | 89-90 |
| 118. | CH₂CH₂ | H | 3-Cl | CH₂CONH₂ | 79-80 |
| 119. | CH₂CH₂ | H | 4-Cl | CH₂CONH₂ | 124-125 |
| 120. | CH₂CH₂ | 2-Cl | 4-Cl | CH₂CONH₂ | 104-105 |
| 121. | CH₂CH₂ | H | 4-F | CH₂CONH₂ | 132-133 |
| 122. | CH₂CH₂ | H | 4-CH₃ | CH₂CONH₂ | 159-160 |
| 123. | CH₂CH₂ | H | 4-Br | CH₂CONH₂ | 162-163 |
| 124. | CH₂CH₂ | H | 3-CF₃ | CH₂CONHCH₃ | 96-98 |
| 125. | CH₂CH₂ | H | 4-CF₃ | CH₂CONHCH₃ | 140-142 |
| 126. | CH₂CH₂ | H | 4-CF₃O | CH₂CONHCH₃ | 126-127 |
| 127. | CH₂CH₂ | H | 4-OH | CH₂CONHCH₃ | 98-99 |
| 128. | CH₂CH₂ | H | 4-CH₃O | CH₂CONH₂ | 133-134 |
| 129. | CH₂CH₂ | H | 4-CH₃O | CH₂CONHCH₃ | 101-102 |
| 130. | CH₂CH₂ | H | 4-CH₃O | CH₂CONHCH₂ CH₃ | 95-96 |
| 131. | CH₂CH₂ | H | 3-CH₃O | CH₂CONH₂ | 86-87 |
| 132. | CH₂CH₂ | 4-CH₃O | 3-CH₃O | CH₂CONH₂ | 110-111 |
| 133. | CH₂CH₂ | 5-CH₃O | 2-CH₃O | CH₂CONH₂ | 140-141 |
| 134. | CH₂CH₂ | H | 2-CH₃O | CH₂CONH₂ | 100-101 |
| 135. | CH₂CH₂ | H | 4-phényl | CH₂CONH₂ | 187-188 |
| 136. | CH₂CH₂ | H | 4-phényl | CH₂CONHCH₃ | 158-159 |
| 137. | CH₂CH₂ | H | 4-phényl | CH₂CONHCH₂ CH₃ | 152-153 |
| 138. | CH₂CH₂ | H | 4-(2-F-phényl) | CH₂CONHCH₃ | 106-107 |
| 139. | CH₂CH₂ | H | 4-(3-F-phényl) | CH₂CONHCH₃ | 157-158 |
| 140. | CH₂CH₂ | H | 4-(4-F-phényl) | CH₂CONHCH₃ | 182-184 |
| 141. | CH₂CH₂ | 2-F | 4-(4-F-phényl) | CH₂CONHCH₃ | 164-166 |
| 142. | CH₂CH₂ | 2-Cl | 4-(4-F-phényl) | CH₂CONHCH₃ | 141-143 |
| 143. | C(CH₃)₂CH₂ | H | 4-(4-F-phényl) | CH₂CONH₂ | 134-136 |
| 144. | C(CH₃)₂CH₂ | H | 4-(4-F-phényl) | CH₂CONHCH₃ | 112-114 |
| 145. | C[CH₂CH₂]-CH₂ | H | 4-(4-F-phényl) | CH₂CONH₂ | 139-141 |
| 146. | C[CH₂CH₂]-CH₂ | H | 4-(4-F-phényl) | CH₂CONHCH₃ | 152-154 |
| 147. | CH₂CH₂ | H | 4-(2-Cl-phényl) | CH₂CONHCH₃ | 148-149 |
| 148. | CH₂CH₂ | H | 4-(3-Cl-phényl) | CH₂CONHCH₃ | 181-182 |
| 149. | CH₂CH₂ | H | 4-(4-Cl-phényl) | CH₂CONH₂ | 198-200 |
| 150. | CH₂CH₂ | H | 4-(4-Cl-phényl) | CH₂CONHCH₃ | 186-188 |
| 151. | CH₂CH₂ | H | 4-(2-CH₃-phényl) | CH₂CONHCH₃ | 108-109 |
| 152. | CH₂CH₂ | H | 4-(3-CH₃-phényl) | CH₂CONHCH₃ | 126-127 |
| 153. | CH₂CH₂ | H | 4-(4-CH₃-phényl) | CH₂CONHCH₃ | 171-172 |
| 154. | CH₂CH₂ | H | 4-(4-CH₃CH₂-phényl) | CH₂CONHCH₃ | 166-167 |
| 155. | CH₂CH₂ | H | 4-(3-i-propyl-phényl) | CH₂CONHCH₃ | 355* |
| 156. | CH₂CH₂ | H | 4-(3-i-propyl-phényl) | CH₂CONHCH₂ -CH₃ | 103-104 |
| 157. | CH₂CH₂ | H | 4-(4-n-butyl-phényl) | CH₂CONHCH₃ | 168-169 |
| 158. | CH₂CH₂ | H | 4-(4-t-butyl-phényl) | CH₂CONHCH₃ | 174-175 |
| 159. | CH₂CH₂ | H | 4-(2-CH₃S-phényl) | CH₂CONHCH₃ | 359* |
| 160. | CH₂CH₂ | H | 4-(2-CH₃O-phényl) | CH₂CONHCH₃ | 111-112 |
| 161. | CH₂CH₂ | H | 4-(3-CH₃O-phényl) | CH₂CONHCH₃ | 343* |
| 162. | CH₂CH₂ | H | 4-(4-CH₃O-phényl) | CH₂CONHCH₃ | 182-183 |
| 163. | CH₂CH₂ | H | 4-(3-CH₃CH₂O-phényl) | CH₂CONHCH₃ | 105-106 |
| 164. | CH₂CH₂ | H | 4-(2-phényloxyphényl) | CH₂CONHCH₃ | 405* |
| 165. | CH₂CH₂ | H | 4-(2-benzyloxyphényl) | CH₂CONHCH₃ | 112-113 |
| 166. | CH₂CH₂ | H | 4-(4-CF₃O-phényl) | CH₂CONHCH₃ | 170-171 |
| 167. | CH₂CH₂ | H | 4-(3-CF₃-phényl) | CH₂CONHCH₃ | 131-132 |
| 168. | CH₂CH₂ | H | 4-(4-CF₃-phényl) | CH₂CONHCH₃ | 198-199 |
| 169. | CH₂CH₂ | H | 4-(4-CN-phényl) | CH₂CONH₂ | 196-198 |
| 170. | CH₂CH₂ | H | 4-(3-CN-phényl) | CH₂CONH₂ | 184-186 |
| 171. | CH₂CH₂ | H | 4-(3-CN-phényl) | CH₂CONHCH₃ | 157-159 |
| 172. | CH₂CH₂ | H | 4-(3-CH₃CO-phényl) | CH₂CONHCH₃ | 102-103 |
| 173. | CH₂CH₂ | H | 4-(4-CH₃O₂C-phényl) | CH₂CONHCH₃ | 184-185 |
| 174. | CH₂CH₂ | H | 4-(3-NO₂-phényl) | CH₂CONHCH₃ | 163-164 |
| 175. | CH₂CH₂ | H | 4-(4-(CH₃)₂N-phényl) | CH₂CONHCH₃ | 170-171 |
| 176. | CH₂CH₂ | H | 4-(2-Cl,3-Cl-phényl) | CH₂CONHCH₃ | 149-150 |
| 177. | CH₂CH₂ | H | 4-(2-Cl,4-Cl-phényl) | CH₂CONH₂ | 132-134 |
| 178. | CH₂CH₂ | H | 4-(2-Cl,4-Cl-phényl) | CH₂CONHCH₃ | 147-149 |
| 179. | CH₂CH₂ | H | 4-(2-Cl,4-Cl-phényl) | CH₂CONHCH₂ -CH₃ | 164-166 |
| 180. | CH₂CH₂ | H | 4-(3-Cl,4-Cl-phényl) | CH₂CONHCH₃ | 163-164 |
| 181. | CH₂CH₂ | H | 4-(2-CH₃O,4-CH₃O-phényl) | CH₂CONHCH₃ | 134-135 |
| 182. | CH₂CH₂ | H | 4-(2-CH₃O,5-CH₃O-phényl) | CH₂CONHCH₃ | 373* |
| 183. | CH₂CH₂ | H | 4-(2-CH₃O,6-CH₃O-phényl) | CH₂CONHCH₃ | 148-149 |
| 184. | CH₂CH₂ | H | 4-(3-CH₃O,4-CH₃O-phényl) | CH₂CONH₂ | 108-109 |
| 185. | CH₂CH₂ | H | 4-(3-CH₃O,4-CH₃O-phényl) | CH₂CONHCH₃ | 132-133 |
| 186. | CH₂CH₂ | H | 4-(3,4-(OCH₂O)-phényl) | CH₂CONHCH₃ | 165-166 |
| 187. | CH₂CH₂ | H | 4-(3-CH₃,4-CH₃-phényl) | CH₂CONHCH₃ | 144-145 |
| 188. | CH₂CH₂ | H | 4-(3-CF₃,5-CF₃-phényl) | CH₂CONHCH₃ | 136-137 |
| 189. | CH₂CH₂ | H | 4-(2-F,3-CH₃O-phényl) | CH₂CONHCH₃ | 130-131 |
| 190. | CH₂CH₂ | H | 4-(5-Cl,2-CH₃O-phényl) | CH₂CONHCH₃ | 106-107 |
| 191. | CH₂CH₂ | H | 4-(3-Cl,4-F-phényl) | CH₂CONH₂ | 151-153 |
| 192. | CH₂CH₂ | H | 4-(3-Cl,4-F-phényl) | CH₂CONHCH₃ | 178-180 |
| 193. | CH₂CH₂ | H | 4-(3-CH₃,4-F-phényl) | CH₂CONHCH₃ | 155-156 |
| 194. | CH₂CH₂ | H | 4-phényloxy | CH₂CONHCH₃ | 111-113 |
| 195. | CH₂CH₂ | H | 4-(4-Cl-phényloxy) | CH₂CONH₂ | 156-158 |
| 196. | CH₂CH₂ | H | 4-(4-Cl-phényloxy) | CH₂CONHCH₃ | 133-135 |
| 197. | CH₂CH₂ | H | 3-phényloxy | CH₂CONHCH₃ | 82-84 |
| 198. | CH₂CH₂ | H | 4-(2-F-benzyloxy) | CH₂CONHCH₃ | 112-113 |
| 199. | CH₂CH₂ | H | 4-(3-F-benzyloxy) | CH₂CONHCH₃ | 127-128 |
| 200. | CH₂CH₂ | H | 4-(4-F-benzyloxy) | CH₂CONHCH₃ | 129-130 |
| 201. | CH₂CH₂ | H | 4-(2-CH₃-benzyloxy) | CH₂CONHCH₃ | 103-104 |
| 202. | CH₂CH₂ | H | 4-(3-CH₃-benzyloxy) | CH₂CONHCH₃ | 112-113 |
| 203. | CH₂CH₂ | H | 4-(4-CH₃-benzyloxy) | CH₂CONHCH₃ | 135-136 |
| 204. | CH₂CH₂ | H | 4-(2-CF₃-benzyloxy) | CH₂CONHCH₃ | 110-111 |
| 205. | CH₂CH₂ | H | 4-(3-CF₃-benzyloxy) | CH₂CONHCH₃ | 103-104 |
| 206. | CH₂CH₂ | H | 4-(4-CF₃-benzyloxy) | CH₂CONHCH₃ | 127-128 |
| 207. | CH₂CH₂ | H | 4-(3-CH₃O-benzyloxy) | CH₂CONHCH₃ | 92-93 |
| 208. | CH₂CH₂ | H | 4-(4-CH₃O₂C-benzyloxy) | CH₂CONHCH₃ | 145-146 |
| 209. | CH₂CH₂ | H | 4-(3-phénylpropyl-1-oxy) | CH₂CONHCH₃ | 110-111 |
| 210. | CH₂CH₂ | H | 4-(pyridin-2-yl) | CH₂CONH₂ | 140-142 |
| 211. | CH₂CH₂ | H | 4-(pyridin-3-yl) | CH₂CONH₂ | 134-136 |
| 212. | CH₂CH₂ | H | 4-(pyridin-4-yl) | CH₂CONH₂ | 206-208 |
| 213. | CH₂CH₂ | H | 4-(pyrimidin-5-yl) | CH₂CONH₂ | 240-242 |
| 214. | CH₂CH₂ | H | 4-(furan-2-yl) | CH₂CONHCH₃ | 150-151 |
| 215. | CH₂CH₂ | H | 4-(thièn-2-yl) | CH₂CONHCH₃ | 157-158 |
| 216. | CH₂CH₂ | H | 4-(thièn-3-yl) | CH₂CONHCH₃ | 174-175 |
| 217. | CH₂CH₂ | H | 4-(benzo[b]thièn-3-yl) | CH₂CONHCH₃ | 124-125 |
| 218. | CH₂CH₂ | H | 4-(2-CH₃O,4-CH₃O-pyrimidin-5-yl) | CH₂CONHCH₃ | 142-143 |
| 219. | CH₂CH₂ | H | 4-(quinolin-2-yl) | CH₂CONH₂ | 214-216 |
| 220. | CH₂CH₂ | H | 4-(quinolin-4-yl) | CH₂CONH₂ | 181-183 |
| 221. | CH₂CH₂ | H | 4-(quinolin-8-yl) | CH₂CONHCH₃ | 130-131 |
| 222. | CH₂CH₂ | H | 4-(isoquinolin-1-yl) | CH₂CONH₂ | 138-140 |
| 223. | CH₂CH₂ | H | 4-(isoquinolin-4-yl) | CH₂CONH₂ | 193-195 |
| 224. | CH₂CH₂ | H | 4-(pyrrolo[2,3-b]pyridinyl) | CH₂CONH₂ | 110-112 |
| 225. | CH₂CH₂ | H | 4-(pyrrolo[2,3-b]pyridinyl) | CH₂CONHCH₃ | 124-126 |

| | | | | | |
|---|---|---|---|---|---|
| * M+H (LC-MS) | | | | | |

Les composés de l'invention ont fait l'objet d'essais pharmacologiques permettant de déterminer leur effet inhibiteur de l'enzyme FAAH (Fatty Acid amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH *(Life Sciences (1995 , 56, 1999-2005* et *Journal of Pharmacology and Experimental Therapeutics (1997), 283,* 729-734). Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10mM (pH 8,0) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est comptée par scintillation liquide.

Dans ces conditions, les composés les plus actifs de l'invention présentent des CI₅₀ (concentration inhibant de 50% l'activité enzymatique contrôle de la FAAH) comprises entre 0,001 et 1 µM.

Le tableau 2 ci-dessous présente les CI₅₀ de quelques composés selon l'invention.

**Tableau 2**

| Composé N° | CI₅₀ |
|---|---|
| 192 | 0,102 µM |
| 171 | 0,108 µM |
| 194 | 0,142 µM |
| 150 | 0,063 µM |
| 178 | 0,140 µM |

Il apparaît donc que les composés selon l'invention ont une activité inhibitrice sur l'enzyme FAAH.

L'activité *in vivo* des composés de l'invention a été évaluée dans un test d'analgésie.

Ainsi, l'administration intrapéritonéale (i.p.) de PBQ (phénylbenzoquinone, 2 mg/kg dans une solution de chlorure de sodium à 0,9% contenant 5% d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale en suspension dans du Tween 80 à 0,5%, 60 minutes ou 120 minutes avant l'administration de PBQ. Dans ces conditions, les composés les plus puissants de l'invention réduisent de 35 à 70 % le nombre d'étirements induits par le PBQ, dans une gamme de doses comprise entre 1 et 30 mg/kg.

Le tableau 3 ci-dessous présente les résultats du test d'analgésie pour quelques composés selon l'invention.

**Tableau 3**

| Composé N° | Réduction du nombre d'étirements (%) |
|---|---|
| 192 | - 43 % (a) |
| 171 | - 51 % (a) |
| 194 | - 55 % (b) |
| 150 | - 57 % (b) |
| 178 | - 53 % (b) |

| | |
|---|---|
| (a) 1 mg/kg p.o. à 2 heures ; (b)3 mg/kg p.o. à 1 heure | |

L'enzyme FAAH (Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyléthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.

Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoides endogènes et/ ou tous autres substrats métabolisés par l'enzyme FAAH, sont impliqués.

On peut par exemple citer les maladies et les affections suivantes :
La douleur notamment les douleurs aiguës ou chroniques de type neurogène : migraine, douleurs neuropathiques incluant les formes associées au virus de l'herpès et au diabète ;
les douleurs aiguës ou chroniques associées aux maladies inflammatoires : arthrite, arthrite rhumatoïde, ostéoarthrite, spondylite, goutte, vascularite, maladie de Crohn, syndrome du colon irritable ;
les douleurs aiguës ou chroniques périphériques ;
les vertiges, les vomissements, les nausées en particulier celles consécutives à une chimiothérapie ;
les troubles du comportement alimentaire en particulier les anorexies et cachexies de diverses natures ;
les pathologies neurologiques et psychiatrique : tremblements, dyskinésies, dystonies, spasticité, comportements compulsifs et obsessionnels, syndrome de Tourette, toutes les formes de dépression et d'anxiété de toute nature et origine, troubles de l'humeur, psychoses ; les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires ;
l'épilepsie ;
les troubles du sommeil incluant les apnées du sommeil ;
les maladies cardiovasculaires en particulier hypertension, arythmies cardiaques, artériosclérose, crise cardiaque, ischémies cardiaques ;
l'ischémie rénale ;
les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes) ;
les désordres du système immunitaire, notamment les maladies auto-immunes : psoriasis, lupus érythémateux, maladies du tissu conjonctif ou connectivites, syndrome de Sjögren's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire ;
les maladies allergiques : l'hypersensibilité immédiate ou retardée, rhinites ou conjonctivites allergiques, dermatites de contact ;
les maladies infectieuses parasitaires , virales ou bactériennes : SIDA, méningites ; les maladies inflammatoires, notamment les maladies articulaires :
arthrite, arthrite rhumatoïde, ostéoarthrite, spondylite, goutte, vascularite, maladie de Crohn, syndrome du colon irritable ; l'ostéoporose ; les affections oculaires :
hypertension oculaire, glaucome ;
les affections pulmonaires : maladies des voies respiratoires, bronchospasmes, toux, asthme, bronchite chronique, obstruction chronique des voies respiratoires, emphysème ;
les maladies gastro-intestinales: syndrome du colon irritable, désordres inflammatoires intestinaux, ulcères, diarrhées ;
l'incontinence urinaire et l'inflammation vésicale.

L'utilisation d'un composé de formule (I), à l'état de base, de sel, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel, ou un hydrate, ou un solvat pharmaceutiquement acceptable dudit composé, et éventuellement un ou plusieurs excipients pharmaceutiquement acceptables.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

## Revendications

1. Composé répondant à la formule (I) 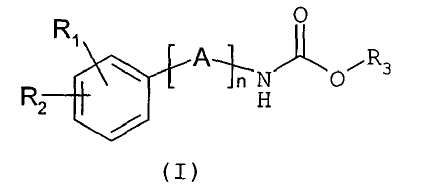 dans laquelle
n représente un nombre entier allant de 1 à 7 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente soit un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; soit un groupe C₂-alcynylène ;
Z représente un groupe C₃₋₇-cycloalkyle, de formule : 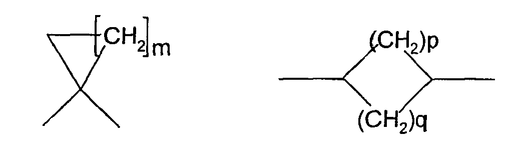 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxy, cyano, nitro, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle ;
R₂ représente
un atome d'hydrogène, d'halogène
ou un groupe cyano, nitro, hydroxy, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle,
ou un groupe choisi parmi notamment un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle , pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₆ et R₇ représentent indépendamment l'un de l'autre un groupe C₁₋₃-alkyle, un phényle ; et
R₃ représente un groupe de formule générale CHR₄CONHR₅ dans laquelle
R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène.
à l'état de base, de sel d'addition à un acide, d'hydrate ou de solvat ;
le benzylcarbamate de 2-amino-2-oxoéthyle étant exclu.

2. Composé de formule (I) selon la revendication 1, **caractérisé en ce que** :
n représente un nombre entier allant de 1 à 7 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente soit un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; soit un groupe C₂-alcynylène ;
Z représente un groupe C₃₋₇-cycloalkyle, de formule : 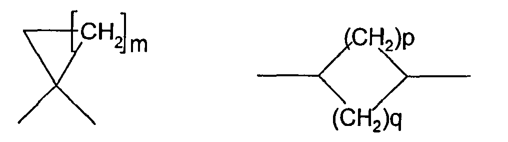
m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxy, cyano, nitro, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle ;
R₂ représente
un atome d'hydrogène, d'halogène
ou un groupe cyano, nitro, hydroxy, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle,
ou un groupe choisi parmi notamment un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle, pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₆ et R₇ représentent indépendamment l'un de l'autre un groupe C₁₋₃-alkyle, un phényle ; et
R₃ représente un groupe de formule générale CHR₄CONHR₅ dans laquelle
R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène ;
à la condition que si R₁ et R₂ représentent un atome d'hydrogène et A est un groupe X, X étant un méthylène, alors n est différent de 1 ;
à l'état de base, de sel d'addition à un acide, d'hydrate ou de solvat .

3. Composé de formule (I) selon la revendication 1 ou 2,
**caractérisé en ce que** :
- quand n est égal à 1 :
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente soit un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; soit un groupe C₂-alcynylène ;
Z représente un groupe C₃₋₇-cycloalkyle, de formule : 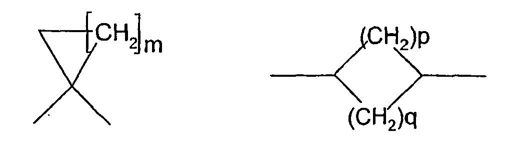
m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxy, cyano, nitro, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle ;
R₂ représente
un atome d'halogène
ou un groupe cyano, nitro, hydroxy, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle,
ou un groupe choisi parmi notamment un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle , pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphthalènylpropoxy, quinoléinoxy, isoquinoléinoxy, et éventuellement substitué par un ou plusieurs substituants choisis parmi un atome d'halogène, un groupe cyano, nitro, C₂₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène) -O-, 4-pipérazinyle éventuellement substitué par un C₁₋₃-alkyle ou par un benzyle ;
R₆ et R₇ représentent indépendamment l'un de l'autre un groupe C₁₋₃-alkyle, un phényle ; et
R₃ représente un groupe de formule générale CHR₄CONHR₅ dans laquelle
R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène ;
- quand n représente un nombre entier allant de 2 à 7 :
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente soit un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; soit un groupe C₂-alcynylène ;
Z représente un groupe C₃₋₇-cycloalkyle, de formule : 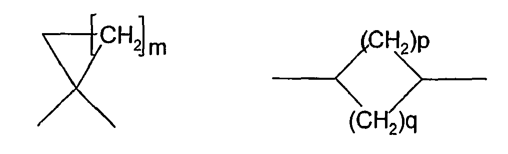
m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxy, cyano, nitro, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle ;
R₂ représente
un atome d'hydrogène, d'halogène
ou un groupe cyano, nitro, hydroxy, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle,
ou un groupe choisi parmi notamment un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle , pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₆ et R₇ représentent indépendamment l'un de l'autre un groupe C₁₋₃-alkyle, un phényle ; et
R₃ représente un groupe de formule générale CHR₄CONHR₅ dans laquelle
R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène ;
à l'état de base, de sel d'addition à un acide, d'hydrate ou de solvat .

4. Composé de formule (I) selon l'une quelconque des revendications 1 à 3, **caractérisé en ce que** :
n représente un nombre entier de 1 à 5 ;
A est choisi parmi un ou plusieurs groupes X et/ou Z ;
X représente un groupe C₁₋₂-alkylène, éventuellement substitué par un ou plusieurs groupes C₁₋₃-alkyle;
Z représente un groupe C₃₋₇-cycloalkyle, de formule : 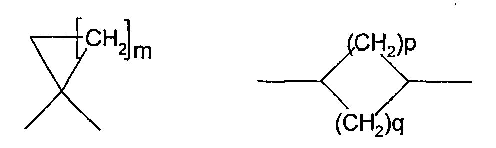
m représente un nombre entier allant de 1 à 5 ;
p et q représentent des nombres entiers et sont définis tels que p+q soit un nombre allant de 1 à 5 ;
R₁ représente un hydrogène ou un halogène, ou un groupe C₁₋₄-alcoxy ;
R₂ représente un atome d'hydrogène, d'halogène, plus particulièrement un chlore, un brome ou un fluor, ou un groupe hydroxy, C₁₋₄-alkyle, plus particulièrement méthyle, C₁₋₄-alcoxy, plus particulièrement méthoxy, C₁₋₄-fluoroalkyle, plus particulièrement trifluorométhyle, C₁₋₄-fluoroalcoxy, plus particulièrement trifluorométhoxy, ou un groupe choisi parmi un phényle, naphtalènyle, biphényle, phényléthylènyle, pyridinyle, pyrimidinyle, pyrazinyle, quinolinyle, isoquinolinyle, thiényle, furanyle, isoxazolyle, thiadiazolyle, phénylimidazolyle, benzothiényle, dibenzofuranyle, benzimidazolyle, pyrrolopyridinyle, phényloxy, phénylsulfonyle, benzoyle, benzyloxy ou phénylpropoxy, et éventuellement substitué par un ou plusieurs substituants choisis parmi un atome d'halogène, plus particulièrement un chlore ou un fluor, un groupe cyano, nitro, C₁₋₄-alkyle, plus particulièrement méthyle, éthyle, isopropyle, butyle, tertiobutyle, C₁₋₄-alcoxy, plus particulièrement méthoxy, éthoxy, C₁₋₄-thioalkyle, plus particulièrement thiométhyle, C₁₋₃-fluoroalkyle, plus particulièrement trifluorométhyle, C₁₋₃-fluoroalcoxy, plus particulièrement trifluorométhoxy, phényloxy, benzyloxy, NR₆R₇, NHCOR₆, COR₆, CO₂R₆, SO₂R₆, -O-(C₁₋₃-alkylène)-O-, plus particulièrement -O-(CH₂)-O- ;
R₆ et R₇ représentent indépendamment l'un de l'autre un groupe C₁₋₃-alkyle ;
R₃ représente un groupe de formule générale CHR₄CONHR₅ dans laquelle
R₄ représente un atome d'hydrogène ou un groupe C₁₋₃-alkyle et R₅ représente un atome d'hydrogène ou un groupe C₁₋₃-alkyle, C₃₋₅-cycloalkyle, C₃₋₇-cycloalkyle-C₁₋₆-alkylène ;
à l'état de base, de sel d'addition à un acide, d'hydrate ou de solvat .

5. Composé de formule (I) selon l'une quelconque des revendications 1 à 4, **caractérisé en ce que** :
n représente un nombre entier de 1 à 5 ;
A représente un groupe C₁₋₂-alkylène ;
R₁ représente un hydrogène ou un halogène ;
R₂ représente un groupe choisi parmi un phényle, naphtalènyle, phényloxy, benzyloxy, pyridinyle, quinolinyle, isoquinolinyle, phénylimidazole ou pyrrolopyridinyle, et éventuellement substitué par un ou plusieurs substituants choisis parmi un atome d'halogène, plus particulièrement un chlore ou un fluor, un groupe cyano, C₁₋₄-alkyle, plus particulièrement méthyle, C₁₋₄-alcoxy, plus particulièrement méthoxy, C₁₋₃-fluoroalkyle, plus particulièrement trifluorométhyle, C₁₋₃-fluoroalcoxy, plus particulièrement trifluorométhoxy ;
R₃ représente un groupe de formule générale CHR₄CONHR₅ dans laquelle
R₄ représente un hydrogène et R₅ représente un atome d'hydrogène ou un groupe C₁₋₃-alkyle, C₃₋₅-cycloalkyle, C₃₋₇-cycloalkyle-C₁₋₆-alkylène ;
à l'état de base, de sel d'addition à un acide, d'hydrate ou de solvat .

6. Composé de formule (I) selon l'une quelconque des revendications 1 à 5, **caractérisé en ce que** :
n présente un nombre entier de 5 à 7 ;
A représente un groupe C₁₋₂-alkylène ;
R₁ et R₂ représentent indépendamment l'un de l'autre un atome d'hydrogène ou d'halogène ou un groupe cyano, hydroxy, C₁₋₄-alkyle, C₁₋₄-alcoxy, C₁₋₄-fluoroalkyle, C₁₋₄-fluoroalcoxy ;
R₃ représente un groupe de formule générale CHR₄CONHR₅ dans laquelle
R₄ représente un hydrogène et R₅ représente un atome d'hydrogène ou un groupe C₁₋₃-alkyle, C₃₋₅-cycloalkyle, C₃₋₇-cycloalkyle-C₁₋₆-alkylène ;
à l'état de base, de sel d'addition à un acide, d'hydrate ou de solvat .

7. Procédé de préparation d'un composé de formule (I) selon l'une quelconque des revendications 1 à 6, comprenant l'étape consistant à
transformer le carbamate-ester de formule générale (Ia) 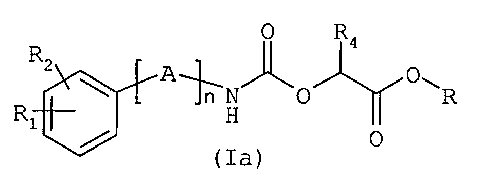 dans laquelle A, n, R₁, R₂ et R₄ sont tels que définis dans la formule (I) selon la revendication 1 et R représente un groupe méthyle ou éthyle,
par aminolyse au moyen d'une amine de formule générale R₅NH₂
dans laquelle R₅ est tel que défini dans la formule (I) selon la revendication 1.

8. Procédé de préparation d'un composé de formule (I) selon l'une quelconque des revendications 1 à 6, comprenant l'étape consistant à
transformer le dérivé oxazolidine-dione de formule générale (V) 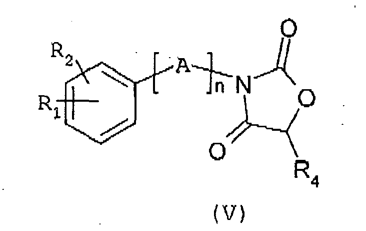 dans laquelle A, n, R₁, R₂ et R₄ sont tels que définis dans la formule (I) selon la revendication 1,
par aminolyse au moyen d'une amine de formule générale R₅NH₂ dans laquelle R₅ est tel que défini dans la formule (I) selon la revendication 1.

9. Composé répondant à la formule générale (Ia), 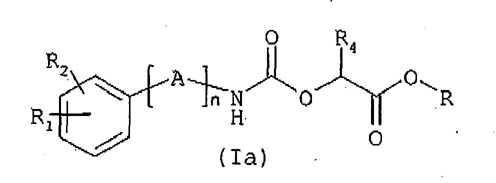 dans laquelle
n représente un nombre entier allant de 1 à 7 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente soit un groupe C₂-alcènylène éventuellement substitué par un ou plusieurs groupes C₁₋₁₂ alkyle, C₃₋₇-cycloalkyle, ou C₃₋₇-cycloalkyl-C₁₋₆-alkylène; soit un groupe C₂-alcynylène ;
Z représente un groupe C₃₋₇-cycloalkyle, de formule : 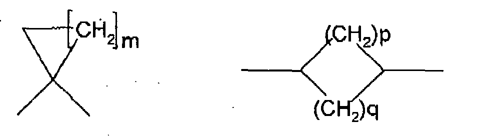 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxy, cyano, nitro, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle ;
R2 représente
un atome d'hydrogène, d'halogène
ou un groupe cyano, nitro, hydroxy, C₁₋₄-alkyle, C₁₋₄-alcoxy, C₁₋₄-thioalkyle, C₁₋₄-fluoroalkyle, C₁₋₄-flucroalcoxy, C₁₋₄-fluorothioalkyle,
ou un groupe choisi parmi un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle , pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, -O-(C₁₋₃-alkylène)-O-, 4-pipérazinyle éventuellement substitué par un C₁₋₃-alkyle ou par un benzyle ;
R₆ et R₇ représentent indépendamment l'un de l'autre un groupe C₁₋₃-alkyle, un phényle ;
R₄ représente un atome d'hydrogène ; et R représente un groupe méthyle ;

10. Composé répondant à la formule générale (V), 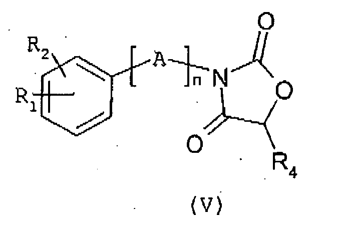 dans laquelle
n représente un nombre entier allant de 1 à 7 ;
A est choisi parmi un ou plusieurs groupes X, Y et/ou Z ;
X représente un groupe C₁₋₂-alkylène éventuellement substitué par un ou plusieurs groupes C₁₋₁₂-alkyle, C₃₋₇-cycloalkyle ou C₃₋₇-cycloalkyl-C₁₋₆-alkylène;
Y représente soit un groupe C₂-alcènylène éventuellement substitué par un ou plusieurs groupes C₁₋₁₂ alkyle, C₃₋₇-cycloalkyle ou C₃₋₇-cycloalkyl-C₁₋₆-alkylène; soit un groupe C₂-alcynylène ;
Z représente un groupe C₃₋₇-cycloalkyle, de formule : 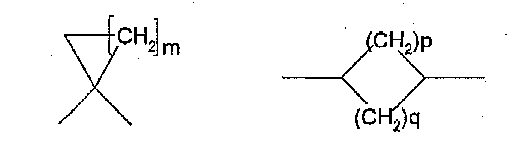 m représente un nombre entier allant de 1 à 5 ;
p et q représentent des nombres entiers et sont définis tels que p+q soit un nombre allant de 1 à 5 ;
R₁ représente un atome d'hydrogène, d'halogène ou un groupe hydroxy, cyano, nitro, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle ;
R₂ représente un atome d'hydrogène, de brome, d'iode ou de fluor, ou un groupe cyano, nitro, hydroxy, C₁₋₄-alkyle, C₁₋₄-alcoxy, C₁₋₄-thioalkyle, C₁₋₄-fluoroalkyle, C₁₋₄-fluoroalcoxy, C₁₋₄-fluorothioalkyle,
ou un groupe choisi parmi un phényle, naphtalènyle, biphényle, phényléthylènyle, naphtyléthylènyle, pyridinyle, pyrimidinyle, pyrazinyle, pyridazinyle, triazinyle, indanyle, indènyle, quinolinyle, isoquinolinyle, quinazolinyle, quinoxalinyle, phthalazinyle, cinnolinyle, thiényle, furanyle, pyrrolyle, imidazolyle, pyrazolyle, oxazolyle, thiazolyle, isoxazolyle, isothiazolyle, thiadiazolyle, oxadiazolyle, triazolyle, phénylimidazolyle, benzothiényle, benzofuranyle, dibenzofuranyle, benzimidazolyle, benzotriazolyle, indolyle, isoindolyle, indazolyle, benzoxazolyle, benzisoxazolyle, benzothiazolyle, benzisothiazolyle, dihydroindolyle , pyrrolopyridinyle, furopyridinyle, thiénopyridinyle, imidazopyridinyle, oxazolopyridinyle, thiazolopyridinyle, pyrazolopyridinyle, isoxazolopyridinyle, isothiazolopyridinyle, tétrahydroquinolinyle, tétrahydroisoquinolinyle, phényloxy, phénylthio, phénylsulfonyle, benzoyle, benzyloxy, phényléthoxy, phénylpropoxy, naphtalènyloxy, naphtalènylméthoxy, naphtalènyléthoxy, naphtalènylpropoxy, quinoléinoxy, isoquinoléinoxy, et éventuellement substitué par un ou plusieurs substituants choisis parmi un atome d'halogène, un groupe cyano, nitro, C₁₋₄-alkyle, hydroxy, C₁₋₄-alcoxy, C₁₋₄-thioalkyle, C₁₋₃-fluoroalkyle, C₁₋₃-fluoroalcoxy, C₁₋₃-fluorothioalkyle, phényloxy, benzyloxy, pipéridinyle, pyrrolidinyle, morpholinyle, NR₆R₇, NHCOR₆, COR₆, CO₂R₆, SO₂R₆, - O-(C₁₋₃-alkylène)-O-, 4-pipérazinyle éventuellement substitué par un C₁₋₃-alkyle ou par un benzyle ;
R₆ et R₇ représentent indépendamment l'un de l'autre un groupe C₁₋₃-alkyle, un phényle ; et
R₄ représente un atome d'hydrogène ; à la condition que les composés pour lesquels le groupe 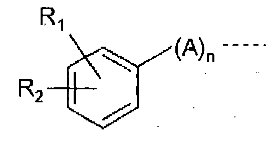 représente un groupe benzyle, paramethoxybenzyle, paranitrobenzyle et 1-méthyl-2-phényl-éthyle sont exclus.

11. Composition pharmaceutique contenant au moins un composé de formule (I) selon l'une quelconque des revendications 1 à 6, à l'état de base, de sel, d'hydrate ou de solvat pharmaceutiquement acceptable et éventuellement un ou plusieurs excipients pharmaceutiquement acceptables.

12. Composé de formule (I) selon l'une quelconque des revendications 1 à 6, à l'état de base, de sel, d'hydrate ou de solvat pharmaceutiquement acceptable, pour son utilisation comme médicament.

13. Utilisation d'un composé de formule (I) selon l'une quelconque des revendications 1 à 6, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales ou l'incontinence urinaire.

## Claims

1. Compound of the formula (I) 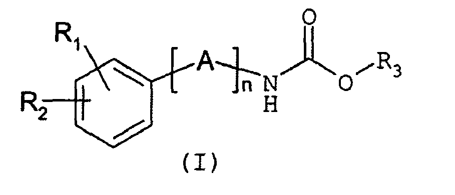 in which
n represents an integer ranging from 1 to 7;
A is selected from one or more groups X, Y and/or Z;
X represents a C₁₋₂-alkylene group optionally substituted by one or more C₁₋₁₂-alkyl, C₃₋₇-cycloalkyl or C₃₋₇-cycloalkyl-C₁₋₆-alkylene groups;
Y represents either a C₂-alkenylene group optionally substituted by one or more C₁₋₁₂-alkyl, C₃₋₇-cycloalkyl or C₃₋₇-cycloalkyl-C₁₋₆-alkylene groups; or a C₂-alkynylene group;
Z represents a C₃₋₇-cycloalkyl group of formula: 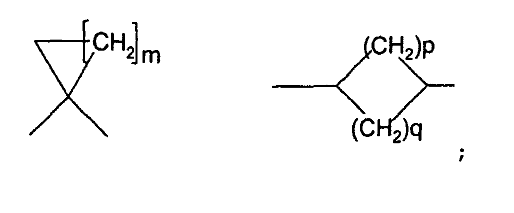 m represents an integer ranging from 1 to 5;
p and q represent integers and are defined such that p+q is a number ranging from 1 to 5;
R₁ represents a hydrogen or halogen atom or a hydroxy, cyano, nitro, C₁₋₄-alkyl, C₁₋₄-alkoxy, C₁₋₄-thioalkyl, C₁₋₄-fluoroalkyl, C₁₋₄-fluoroalkoxy or C₁₋₄-fluorothioalkyl group;
R₂ represents
a hydrogen or halogen atom or
a cyano, nitro, hydroxy, C₁₋₄-alkyl, C₁₋₄-alkoxy, C₁₋₄-thioalkyl, C₁₋₄-fluoroalkyl, C₁₋₄-fluoroalkoxy, C₁₋₄-fluorothioalkyl group, or
a group selected from in particular a phenyl, naphthyl, biphenyl, phenylethylenyl, naphthylethylenyl, pyridinyl, pyrimidinyl, pyrazinyl, pyridazinyl, triazinyl, indanyl, indenyl, quinolinyl, isoquinolinyl, quinazolinyl, quinoxalinyl, phthalazinyl, cinnolinyl, thienyl, furanyl, pyrrolyl, imidazolyl, pyrazolyl, oxazolyl, thiazolyl, isoxazolyl, isothiazolyl, thiadiazolyl, oxadiazolyl, triazolyl, phenylimidazolyl, benzothienyl, benzofuranyl, dibenzofuranyl, benzimidazolyl, benzotriazolyl, indolyl, isoindolyl, indazolyl, benzoxazolyl, benzisoxazolyl, benzothiazolyl, benzisothiazolyl, dihydroindolyl, pyrrolopyridinyl, furopyridinyl, thienopyridinyl, imidazopyridinyl, oxazolopyridinyl, thiazolopyridinyl, pyrazolopyridinyl, isoxazolopyridinyl, isothiazolopyridinyl, tetrahydroquinolinyl, tetrahydroisoquinolinyl, phenyloxy, phenylthio, phenylsulphonyl, benzoyl, benzyloxy, phenylethoxy, phenylpropoxy, naphthyloxy, naphthylmethoxy, naphthylethoxy, naphthylpropoxy, quinolinoxy and isoquinolinoxy and optionally substituted by one or more substituents selected from a halogen atom or a cyano, nitro, C₁₋₄-alkyl, hydroxy, C₁₋₄-alkoxy, C₁₋₄-thioalkyl, C₁₋₃-fluoroalkyl, C₁₋₃-fluoroalkoxy, C₁₋₃-fluorothioalkyl, phenyloxy, benzyloxy, piperidinyl, pyrrolidinyl, morpholinyl, NR₆R₇, NHCOR₆, COR₆, CO₂R₆, SO₂R₆, -O-(C₁₋₃-alkylene)-O- or 4-piperazinyl group optionally substituted by a C₁₋₃-alkyl or by a benzyl; R₆ and R₇ represent independently of one another a C₁₋₃-alkyl group or a phenyl; and
R₃ represents a group of general formula CHR₄CONHR₅ in which
R₄ represents a hydrogen atom or a C₁₋₃-alkyl group and
R₅ represents a hydrogen atom or a C₁₋₃-alkyl, C₃₋₅-cycloalkyl or C₃₋₇-cycloalkyl-C₁₋₆-alkylene group, in the form of the base, acid addition salt, hydrate or solvate;
with the exception of 2-amino-2-oxoethyl benzylcarbamate.

2. Compound of formula (I) according to Claim 1, **characterized in that**:
n represents an integer ranging from 1 to 7;
A is selected from one or more groups X, Y and/or Z;
X represents a C₁₋₂-alkylene group optionally substituted by one or more C₁₋₁₂-alkyl, C₃₋₇-cycloalkyl or C₃₋₇-cycloalkyl-C₁₋₆-alkylene groups;
Y represents either a C₂-alkenylene group optionally substituted by one or more C₁₋₁₂-alkyl, C₃₋₇-cycloalkyl or C₃₋₇-cycloalkyl-C₁₋₆-alkylene groups; or a C₂-alkynylene group;
Z represents a C₃₋₇-cycloalkyl group of formula: 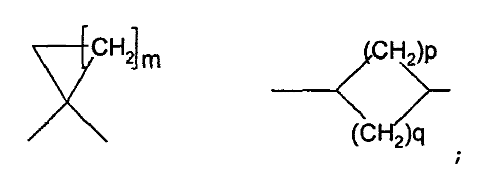
m represents an integer ranging from 1 to 5;
p and q represent integers and are defined such that p+q is a number ranging from 1 to 5;
R₁ represents a hydrogen or halogen atom or a hydroxy, cyano, nitro, C₁₋₄-alkyl, C₁₋₄-alkoxy, C₁₋₄-thioalkyl, C₁₋₄-fluoroalkyl, C₁₋₄-fluoroalkoxy or C₁₋₄-fluorothioalkyl group;
R₂ represents
a hydrogen or halogen atom or
a cyano, nitro, hydroxy, C₁₋₄-alkyl, C₁₋₄-alkoxy, C₁₋₄-thioalkyl, C₁₋₄-fluoroalkyl, C₁₋₄-fluoroalkoxy, C₁₋₄-fluorothioalkyl group, or
a group selected from in particular a phenyl, naphthyl, biphenyl, phenylethylenyl, naphthylethylenyl, pyridinyl, pyrimidinyl, pyrazinyl, pyridazinyl, triazinyl, indanyl, indenyl, quinolinyl, isoquinolinyl, quinazolinyl, quinoxalinyl, phthalazinyl, cinnolinyl, thienyl, furanyl, pyrrolyl, imidazolyl, pyrazolyl, oxazolyl, thiazolyl, isoxazolyl, isothiazolyl, thiadiazolyl, oxadiazolyl, triazolyl, phenylimidazolyl, benzothienyl, benzofuranyl, dibenzofuranyl, benzimidazolyl, benzotriazolyl, indolyl, isoindolyl, indazolyl, benzoxazolyl, benzisoxazolyl, benzothiazolyl, benzisothiazolyl, dihydroindolyl, pyrrolopyridinyl, furopyridinyl, thienopyridinyl, imidazopyridinyl, oxazolopyridinyl, thiazolopyridinyl, pyrazolopyridinyl, isoxazolopyridinyl, isothiazolopyridinyl, tetrahydroquinolinyl, tetrahydroisoquinolinyl, phenyloxy, phenylthio, phenylsulphonyl, benzoyl, benzyloxy, phenylethoxy, phenylpropoxy, naphthyloxy, naphthylmethoxy, naphthylethoxy, naphthylpropoxy, quinolinoxy and isoquinolinoxy and optionally substituted by one or more substituents selected from a halogen atom and a cyano, nitro, C₁₋₄-alkyl, hydroxy, C₁₋₄-alkoxy, C₁₋₄-thioalkyl, C₁₋₃-fluoroalkyl, C₁₋₃-fluoroalkoxy, C₁₋₃-fluorothioalkyl, phenyloxy, benzyloxy, piperidinyl, pyrrolidinyl, morpholinyl, NR₆R₇, NHCOR₆ , COR₆, CO₂R₆, SO₂R₆, -O-(C₁₋₃-alkylene)-O- or 4-piperazinyl group optionally substituted by a C₁₋₃-alkyl or by a benzyl; R₆ and R₇ represent independently of one another a C₁₋₃-alkyl group or a phenyl; and
R₃ represents a group of general formula CHR₄CONHR₅ in which
R₄ represents a hydrogen atom or a C₁₋₃-alkyl group and R₅ represents a hydrogen atom or a C₁₋₃-alkyl, C₃₋₅-cycloalkyl or C₃₋₇-cycloalkyl-C₁₋₆-alkylene group; with the proviso that if R₁ and R₂ represent a hydrogen atom and A is a group X, X being a methylene, then n is other than 1;
in the form of the base, acid addition salt, hydrate or solvate.

3. Compound of formula (I) according to Claim 1 or 2, **characterized in that**:
- when n is 1:
A is selected from one or more groups X, Y and/or Z;
X represents a C₁₋₂-alkylene group optionally substituted by one or more C₁₋₁₂-alkyl, C₃₋₇-cycloalkyl or C₃₋₇-cycloalkyl-C₁₋₆-alkylene groups;
Y represents either a C₂-alkenylene group optionally substituted by one or more C₁₋₁₂-alkyl, C₃₋₇-cycloalkyl or C₃₋₇-cycloalkyl-C₁₋₆-alkylene groups; or a C₂-alkynylene group;
Z represents a C₃₋₇-cycloalkyl group of formula: 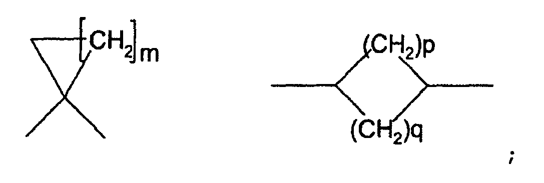
m represents an integer ranging from 1 to 5;
p and q represent integers and are defined such that p+q is a number ranging from 1 to 5;
R₁ represents a hydrogen or halogen atom or a hydroxy, cyano, nitro, C₁₋₄-alkyl, C₁₋₄-alkoxy, C₁₋₄-thioalkyl, C₁₋₄-fluoroalkyl, C₁₋₄-fluoroalkoxy or C₁₋₄-fluorothioalkyl group;
R₂ represents
a halogen atom or
a cyano, nitro, hydroxy, C₁₋₄-alkyl, C₁₋₄-alkoxy, C₁₋₄-thioalkyl, C₁₋₄-fluoroalkyl, C₁₋₄-fluoroalkoxy, C₁₋₄-fluorothioalkyl group, or
a group selected from in particular a phenyl, naphthyl, biphenyl, phenylethylenyl, naphthylethylenyl, pyridinyl, pyrimidinyl, pyrazinyl, pyridazinyl, triazinyl, indanyl, indenyl, quinolinyl, isoquinolinyl, quinazolinyl, quinoxalinyl, phthalazinyl, cinnolinyl, thienyl, furanyl, pyrrolyl, imidazolyl, pyrazolyl, oxazolyl, thiazolyl, isoxazolyl, isothiazolyl, thiadiazolyl, oxadiazolyl, triazolyl, phenylimidazolyl, benzothienyl, benzofuranyl, dibenzofuranyl, benzimidazolyl, benzotriazolyl, indolyl, isoindolyl, indazolyl, benzoxazolyl, benzisoxazolyl, benzothiazolyl, benzisothiazolyl, dihydroindolyl, pyrrolopyridinyl, furopyridinyl, thienopyridinyl, imidazopyridinyl, oxazolopyridinyl, thiazolopyridinyl, pyrazolopyridinyl, isoxazolopyridinyl, isothiazolopyridinyl, tetrahydroquinolinyl, tetrahydroisoquinolinyl, phenyloxy, phenylthio, phenylsulphonyl, benzoyl, benzyloxy, phenylethoxy, phenylpropoxy, naphthyloxy, naphthylmethoxy, naphthylethoxy, naphthylpropoxy, quinolinoxy and isoquinolinoxy and optionally substituted by one or more substituents selected from a halogen atom and a cyano, nitro, C₁₋₄-alkyl, hydroxy, C₁₋₄-alkoxy, C₁₋₄-thioalkyl, C₁₋₃-fluoroalkyl, C₁₋₃-fluoroalkoxy, C₁₋₃-fluorothioalkyl, phenyloxy, benzyloxy, piperidinyl, pyrrolidinyl, morpholinyl, NR₆R₇, NHCOR₆, COR₆, CO₂R₆, SO₂R₆, -O-(C₁₋₃-alkylene)-O- or 4-piperazinyl group optionally substituted by a C₁₋₃-alkyl or by a benzyl;
R₆ and R₇ represent independently of one another a C₁₋₃-alkyl group or a phenyl; and
R₃ represents a group of general formula CHR₄CONHR₅ in which
R₄ represents a hydrogen atom or a C₁₋₃-alkyl group and R₅ represents a hydrogen atom or a C₁₋₃-alkyl, C₃₋₅-cycloalkyl or C₃₋₇-cycloalkyl-C₁₋₆-alkylene group;
- when n represents an integer ranging from 2 to 7:
A is selected from one or more groups X, Y and/or Z;
X represents a C₁₋₂-alkylene group optionally substituted by one or more C₁₋₁₂-alkyl, C₃₋₇-cycloalkyl or C₃₋₇-cycloalkyl-C₁₋₆-alkylene groups;
Y represents either a C₂-alkenylene group optionally substituted by one or more C₁₋₁₂-alkyl, C₃₋₇-cycloalkyl or C₃₋₇-cycloalkyl-C₁₋₆-alkylene groups; or a C₂-alkynylene group;
Z represents a C₃₋₇-cycloalkyl group of formula: 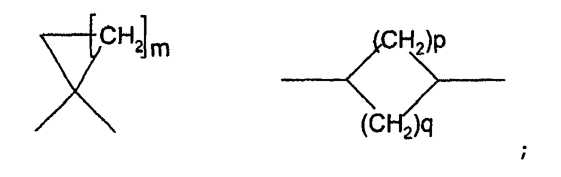
m represents an integer ranging from 1 to 5;
p and q represent integers and are defined such that p+q is a number ranging from 1 to 5;
R₁ represents a hydrogen or halogen atom or a hydroxy, cyano, nitro, C₁₋₄-alkyl, C₁₋₄-alkoxy, C₁₋₄-thioalkyl, C₁₋₄-fluoroalkyl, C₁₋₄-fluoroalkoxy or C₁₋₄-fluorothioalkyl group;
R₂ represents
a hydrogen or halogen atom or
a cyano, nitro, hydroxy, C₁₋₄-alkyl, C₁₋₄-alkoxy, C₁₋₄-thioalkyl, C₁₋₄-fluoroalkyl, C₁₋₄-fluoroalkoxy, C₁₋₄-fluorothioalkyl group, or
a group selected from in particular a phenyl, naphthyl, biphenyl, phenylethylenyl, naphthylethylenyl, pyridinyl, pyrimidinyl, pyrazinyl, pyridazinyl, triazinyl, indanyl, indenyl, quinolinyl, isoquinolinyl, quinazolinyl, quinoxalinyl, phthalazinyl, cinnolinyl, thienyl, furanyl, pyrrolyl, imidazolyl, pyrazolyl, oxazolyl, thiazolyl, isoxazolyl, isothiazolyl, thiadiazolyl, oxadiazolyl, triazolyl, phenylimidazolyl, benzothienyl, benzofuranyl, dibenzofuranyl, benzimidazolyl, benzotriazolyl, indolyl, isoindolyl, indazolyl, benzoxazolyl, benzisoxazolyl, benzothiazolyl, benzisothiazolyl, dihydroindolyl, pyrrolopyridinyl, furopyridinyl, thienopyridinyl, imidazopyridinyl, oxazolopyridinyl, thiazolopyridinyl, pyrazolopyridinyl, isoxazolopyridinyl, isothiazolopyridinyl, tetrahydroquinolinyl, tetrahydroisoquinolinyl, phenyloxy, phenylthio, phenylsulphonyl, benzoyl, benzyloxy, phenylethoxy, phenylpropoxy, naphthyloxy, naphthylmethoxy, naphthylethoxy, naphthylpropoxy, quinolinoxy and isoquinolinoxy and optionally substituted by one or more substituents selected from a halogen atom and a cyano, nitro, C₁₋₄-alkyl, hydroxy, C₁₋₄-alkoxy, C₁₋₄-thioalkyl, C₁₋₃-fluoroalkyl, C₁₋₃-fluoroalkoxy, C₁₋₃-fluorothioalkyl, phenyloxy, benzyloxy, piperidinyl, pyrrolidinyl, morpholinyl, NR₆R₇, NHCOR₆, COR₆, CO₂R₆, SO₂R₆, -O-(C₁₋₃-alkylene)-O- or 4-piperazinyl group optionally substituted by a C₁₋₃-alkyl or by a benzyl; R₆ and R₇ represent independently of one another a C₁₋₃-alkyl group or a phenyl; and
R₃ represents a group of general formula CHR₄CONHR₅ in which
R₄ represents a hydrogen atom or a C₁₋₃-alkyl group and
R₅ represents a hydrogen atom or a C₁₋₃-alkyl, C₃₋₅-cycloalkyl or C₃₋₇-cycloalkyl-C₁₋₆-alkylene group; in the form of the base, acid addition salt, hydrate or solvate.

4. Compound of formula (I) according to any one of Claims 1 to 3, **characterized in that**:
n represents an integer between 1 and 5;
A is selected from one or more groups X and/or Z;
X represents a C₁₋₂-alkylene group optionally substituted by one or more C₁₋₃-alkyl groups;
Z represents a C₃₋₇-cycloalkyl group of formula: 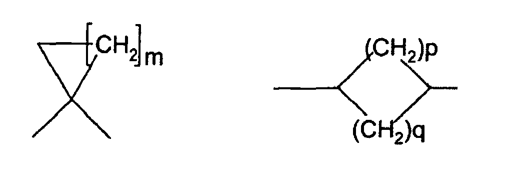
m represents an integer ranging from 1 to 5;
p and q represent integers and are defined such that p+q is a number ranging from 1 to 5;
R₁ represents a hydrogen or a halogen or a C₁₋₄-alkoxy group;
R₂ represents a hydrogen or halogen atom, more particularly chlorine, bromine or fluorine, or a hydroxy group, C₁₋₄-alkyl group, more particularly methyl, C₁₋₄-alkoxy group, more particularly methoxy, C₁₋₄-fluoroalkyl group, more particularly trifluoromethyl, or C₁₋₄-fluoroalkoxy group, more particularly trifluoromethoxy, or a group selected from phenyl, naphthyl, biphenyl, phenylethylenyl, pyridinyl, pyrimidinyl, pyrazinyl, quinolinyl, isoquinolinyl, thienyl, furanyl, isoxazolyl, thiadiazolyl, phenylimidazolyl, benzothienyl, dibenzofuranyl, benzimidazolyl, pyrrolopyridinyl, phenyloxy, phenylsulphonyl, benzoyl, benzyloxy or phenylpropoxy, optionally substituted by one or more substituents selected from a halogen atom, more particularly chlorine or fluorine, or a cyano, nitro or C₁₋₄-alkyl group, more particularly methyl, ethyl, isopropyl, butyl or tert-butyl, C₁₋₄-alkoxy group, more particularly methoxy or ethoxy, C₁₋₄-thioalkyl group, more particularly thiomethyl, C₁₋₃-fluoroalkyl group, more particularly trifluoromethyl, C₁₋₃-fluoroalkoxy group, more particularly trifluoromethoxy, phenyloxy, or benzyloxy, NR₆R₇, NHCOR₆, COR₆, CO₂R₆, SO₂R₆ or -O- (C₁₋₃-alkylene) -O-, more particularly -O- (CH₂) -O-; R₆ and R₇ represent independently of one another a C₁₋₃-alkyl group;
R₃ represents a group of general formula CHR₄CONHR₅ in which
R₄ represents a hydrogen atom or a C₁₋₃-alkyl group and R₅ represents a hydrogen atom or a C₁₋₃-alkyl group, C₃₋₅-cycloalkyl group or C₃₋₇-cycloalkyl-C₁₋₆-alkylene group;
in the form of a base, acid addition salt, hydrate or solvate.

5. Compound of formula (I) according to any one of Claims 1 to 4, **characterized in that**:
n represents an integer from 1 to 5;
A represents a C₁₋₂-alkylene group;
R₁ represents a hydrogen or a halogen;
R₂ represents a group selected from phenyl, naphthyl, phenyloxy, benzyloxy, pyridinyl, quinolinyl, isoquinolinyl, phenylimidazole or pyrrolopyridinyl, optionally substituted by one or more substituents selected from a halogen atom, more particularly chlorine or fluorine, a cyano group, a C₁₋₄-alkyl group, more particularly methyl, C₁₋₄-alkoxy group, more particularly methoxy, C₁₋₃-fluoroalkyl group, more particularly trifluoromethyl, C₁₋₃-fluoroalkoxy group, more particularly trifluoromethoxy;
R₃ represents a group of general formula CHR₄CONHR₅ in which
R₄ represents a hydrogen and R₅ represents a hydrogen atom or a C₁₋₃-alkyl group, C₃₋₅-cycloalkyl group or C₃₋₇-cycloalkyl-C₁₋₆-alkylene group;
in the form of a base, acid addition salt, hydrate or solvate.

6. Compound of formula (I) according to any one of Claims 1 to 5, **characterized in that**:
n represents an integer from 5 to 7;
A represents a C₁₋₂-alkylene group;
R₁ and R₂ represent independently of one another a hydrogen or halogen atom or a cyano, hydroxy, C₁₋₄-alkyl, C₁₋₄-alkoxy, C₁₋₄-fluoroalkyl or C₁₋₄-fluoroalkoxy group;
R₃ represents a group of general formula CHR₄CONHR₅ in which
R₄ represents a hydrogen and R₅ represents a hydrogen atom or C₁₋₃-alkyl group, C₃₋₅-cycloalkyl group, or C₃₋₇-cycloalkyl-C₁₋₆-alkylene group;
in the form of a base, acid addition salt, hydrate or solvate.

7. Process for preparing a compound of formula (I) according to any one of Claims 1 to 6, comprising the step consisting in converting the carbamate ester of general formula (Ia) 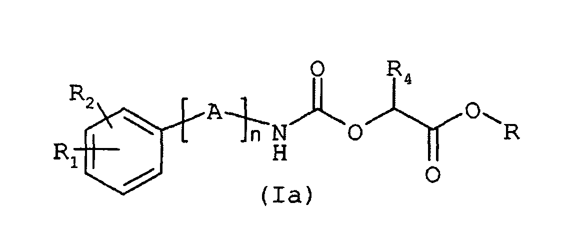 in which A, n, R₁, R₂ and R₄ are as defined for the formula (I) according to Claim 1 and R represents a methyl or ethyl group
by aminolysis using an amine of general formula R₅NH₂ in which R₅ is as defined for formula (I) according to Claim 1.

8. Process for preparing a compound of formula (I) according to any one of Claims 1 to 6, comprising the step consisting in converting the oxazolidinedione derivative of general formula (V) 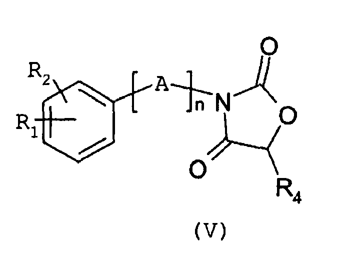 in which A, n, R₁, R₂ and R₄ are as defined for the formula (I) according to Claim 1,
by aminolysis using an amine of general formula R₅NH₂ in which R₅ is as defined for the formula (I) according to Claim 1.

9. Compound of the general formula (Ia) 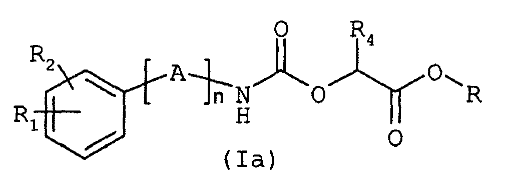 in which
n represents an integer ranging from 1 to 7;
A is selected from one or more groups X, Y and/or Z;
X represents a C₁₋₂-alkylene group optionally substituted by one or more C₁₋₁₂-alkyl, C₃₋₇-cycloalkyl or C₃₋₇-cycloalkyl-C₁₋₆-alkylene groups;
Y represents either a C₂-alkenylene group optionally substituted by one or more C₁₋₁₂-alkyl, C₃₋₇-cycloalkyl or C₃₋₇-cycloalkyl-C₁₋₆-alkylene groups; or a C₂-alkynylene group;
Z represents a C₃₋₇-cycloalkyl group of formula: 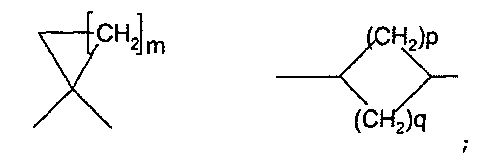 m represents an integer ranging from 1 to 5;
p and q represent integers and are defined such that p+q is a number ranging from 1 to 5;
R₁ represents a hydrogen or halogen atom or a hydroxy, cyano, nitro, C₁₋₄-alkyl, C₁₋₄-alkoxy, C₁₋₄-thioalkyl, C₁₋₄-fluoroalkyl, C₁₋₄-fluoroalkoxy or C₁₋₄-fluorothioalkyl group;
R₂ represents
a hydrogen or halogen atom
or a cyano, nitro, hydroxy, C₁₋₄-alkyl, C₁₋₄-alkoxy, C₁₋₄-thioalkyl, C₁₋₄-fluoroalkyl, C₁₋₄-fluoroalkoxy, C₁₋₄-fluorothioalkyl group,
or a group selected from a phenyl, naphthyl, biphenyl, phenylethylenyl, naphthylethylenyl, pyridinyl, pyrimidinyl, pyrazinyl, pyridazinyl, triazinyl, indanyl, indenyl, quinolinyl, isoquinolinyl, quinazolinyl, quinoxalinyl, phthalazinyl, cinnolinyl, thienyl, furanyl, pyrrolyl, imidazolyl, pyrazolyl, oxazolyl, thiazolyl, isoxazolyl, isothiazolyl, thiadiazolyl, oxadiazolyl, triazolyl, phenylimidazolyl, benzothienyl, benzofuranyl, dibenzofuranyl, benzimidazolyl, benzotriazolyl, indolyl, isoindolyl, indazolyl, benzoxazolyl, benzisoxazolyl, benzothiazolyl, benzisothiazolyl, dihydroindolyl, pyrrolopyridinyl, furopyridinyl, thienopyridinyl, imidazopyridinyl, oxazolopyridinyl, thiazolopyridinyl, pyrazolopyridinyl, isoxazolopyridinyl, isothiazolopyridinyl, tetrahydroquinolinyl, tetrahydroisoquinolinyl, phenyloxy, phenylthio, phenylsulphonyl, benzoyl, benzyloxy, phenylethoxy, phenylpropoxy, naphthyloxy, naphthylmethoxy, naphthylethoxy, naphthylpropoxy, quinolinoxy and isoquinolinoxy and optionally substituted by one or more substituents selected from a halogen atom or a cyano, nitro, C₁₋₄-alkyl, hydroxy, C₁₋₄-alkoxy, C₁₋₄-thioalkyl, C₁₋₃-fluoroalkyl, C₁₋₃-fluoroalkoxy, C₁₋₃-fluorothioalkyl, phenyloxy, benzyloxy, piperidinyl, pyrrolidinyl, morpholinyl, NR₆R₇, NHCOR₆, COR₆, CO₂R₆, SO₂R₆, -O-(C₁₋₃-alkylene)-O- or 4-piperazinyl group optionally substituted by a C₁₋₃-alkyl or by a benzyl;
R₆ and R₇ represent independently of one another a C₁₋₃-alkyl group or a phenyl; and
R₄ represents a hydrogen atom; and
R represents a methyl group.

10. Compound of the general formula (V) 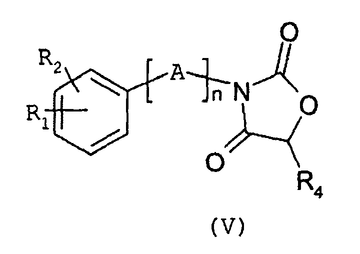 in which
n represents an integer ranging from 1 to 7;
A is selected from one or more groups X, Y and/or Z;
X represents a C₁₋₂-alkylene group optionally substituted by one or more C₁₋₁₂-alkyl, C₃₋₇-cycloalkyl or C₃₋₇-cycloalkyl-C₁₋₆-alkylene groups;
Y represents either a C₂-alkenylene group optionally substituted by one or more C₁₋₁₂-alkyl, C₃₋₇-cycloalkyl or C₃₋₇-cycloalkyl-C₁₋₆-alkylene groups; or a C₂₋alkynylene group;
Z represents a C₃₋₇-cycloalkyl group of formula: 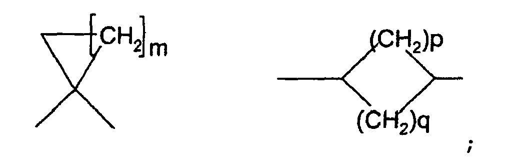 m represents an integer ranging from 1 to 5;
p and q represent integers and are defined such that p+q is a number ranging from 1 to 5;
R₁ represents a hydrogen or halogen atom or a hydroxy, cyano, nitro, C₁₋₄-alkyl, C₁₋₄-alkoxy, C₁₋₄-thioalkyl, C₁₋₄-fluoroalkyl, C₁₋₄-fluoroalkoxy or C₁₋₄-fluorothioalkyl group;
R₂ represents a hydrogen, bromine, iodine or fluorine atom or a cyano, nitro, hydroxy, C₁₋₄-alkyl, C₁₋₄-alkoxy, C₁₋₄-thioalkyl, C₁₋₄-fluoroalkyl, C₁₋₄-fluoroalkoxy, C₁₋₄-fluorothioalkyl group,
or a group selected from a phenyl, naphthyl, biphenyl, phenylethylenyl, naphthylethylenyl, pyridinyl, pyrimidinyl, pyrazinyl, pyridazinyl, triazinyl, indanyl, indenyl, quinolinyl, isoquinolinyl, quinazolinyl, quinoxalinyl, phthalazinyl, cinnolinyl, thienyl, furanyl, pyrrolyl, imidazolyl, pyrazolyl, oxazolyl, thiazolyl, isoxazolyl, isothiazolyl, thiadiazolyl, oxadiazolyl, triazolyl, phenylimidazolyl, benzothienyl, benzofuranyl, dibenzofuranyl, benzimidazolyl, benzotriazolyl, indolyl, isoindolyl, indazolyl, benzoxazolyl, benzisoxazolyl, benzothiazolyl, benzisothiazolyl, dihydroindolyl, pyrrolopyridinyl, furopyridinyl, thienopyridinyl, imidazopyridinyl, oxazolopyridinyl, thiazolopyridinyl, pyrazolopyridinyl, isoxazolopyridinyl, isothiazolopyridinyl, tetrahydroquinolinyl, tetrahydroisoquinolinyl, phenyloxy, phenylthio, phenylsulphonyl, benzoyl, benzyloxy, phenylethoxy, phenylpropoxy, naphthyloxy, naphthylmethoxy, naphthylethoxy, naphthylpropoxy, quinolinoxy and isoquinolinoxy and optionally substituted by one or more substituents selected from a halogen atom or a cyano, nitro, C₁₋₄ -alkyl, hydroxy, C₁₋₄-alkoxy, C₁₋₄-thioalkyl, C₁₋₃-fluoroalkyl, C₁₋₃-fluoroalkoxy, C₁₋₃-fluorothioalkyl, phenyloxy, benzyloxy, piperidinyl, pyrrolidinyl, morpholinyl, NR₆R₇, NHCOR₆, COR₆, CO₂R₆, SO₂R₆, -O-(C₁₋₃-alkylene)-O- or 4-piperazinyl group optionally substituted by a C₁₋₃-alkyl or by a benzyl;
R₆ and R₇ represent independently of one another a C₁₋₃-alkyl group or a phenyl; and
R₄ represents a hydrogen atom;
with the proviso that the compound for which the group 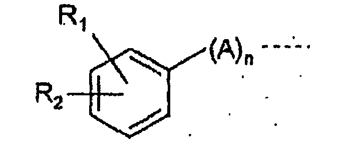 represents a benzyl, para-methoxy benzyl, paranitrobenzyl and 1-methyl-2-phenylethyl are excluded.

11. Pharmaceutical composition comprising at least one compound of formula (I) according to any one of Claims 1 to 6, in base, salt, hydrate or pharmaceutically acceptable solvate form, and optionally one or more pharmaceutically acceptable excipients.

12. Compound of formula (I) according to any one of Claims 1 to 6, in base, salt, hydrate or pharmaceutically acceptable solvate form, for its use as a medicinal product.

13. Use of a compound of formula (I) according to any one of Claims 1 to 6, in base, salt, hydrate or pharmaceutically acceptable solvate form, for preparing a medicinal product intended for preventing or treating acute or chronic pain, dizziness, vomiting, nausea, eating disorders, neurological and psychiatric pathologies, acute or chronic neurodegenerative diseases, epilepsy, sleep disorders, cardiovascular diseases, renal ischaemia, cancers, disorders of the immune system, allergic diseases, parasitic, viral or bacterial infectious diseases, inflammatory diseases, osteoporosis, ocular conditions, pulmonary conditions, gastrointestinal diseases or urinary incontinence.

## Patentansprüche

1. Verbindung der Formel (I) 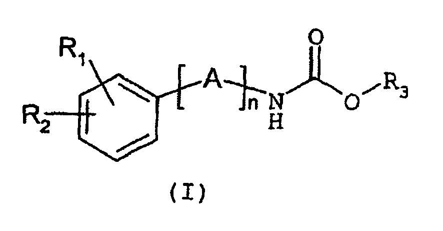 worin
n für eine ganze Zahl von 1 bis 7 steht;
A unter einer oder mehreren Gruppen X, Y und/oder
Z ausgewählt ist;
X für eine gegebenenfalls durch eine oder mehrere C₁₋₁₂-Alkyl-, C₃₋₇-Cycloalkyl- oder C₃₋₇-Cycloalkyl-C₁₋₆-alkylengruppen substituierte C₁₋₂-Alkylengruppe steht;
Y entweder für eine gegebenenfalls durch eine oder mehrere C₁₋₁₂-Alkyl-, C₃₋₇-Cycloalkyl- oder C₃₋₇-Cycloalkyl-C₁₋₆-alkylengruppen substituierte C₂-Alkenylengruppe oder eine C₂-Alkinylengruppe steht; Z für eine C₃-₇-Cycloalkylgruppe der Formel: 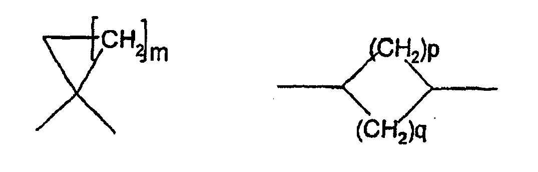 steht;
m für eine ganze Zahl von 1 bis 5 steht;
p und q für ganze Zahlen stehen und so definiert sind, daß p+q für eine ganze Zahl von 1 bis 5 steht;
R₁ für ein Wasserstoff- oder Halogenatom oder eine Hydroxy-, Cyano-, Nitro-, C₁₋₄-Alky1-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe steht;
R₂ für
ein Wasserstoff- oder Halogenatom oder
eine Cyano-, Nitro-, Hydroxy-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe oder eine Gruppe, die insbesondere unter Phenyl, Naphthalinyl, Biphenyl, Phenylethylenyl, Naphthylethylenyl, Pyridinyl, Pyrimidinyl, Pyrazinyl, Pyridazinyl, Triazinyl, Indanyl, Indenyl, Chinolinyl, Isochinolinyl, Chinazolinyl, Chinoxalinyl, Phthalazinyl, Cinnolinyl, Thienyl, Furanyl, Pyrrolyl, Imidazolyl, Pyrazolyl, Oxazolyl, Thiazolyl, Isoxazolyl, Isothiazolyl, Thiadiazolyl, Oxadiazolyl, Triazolyl, Phenylimidazolyl, Benzothienyl, Benzofuranyl, Dibenzofuranyl, Benzimidazolyl, Benzotriazolyl, Indolyl, Isoindolyl, Indazolyl, Benzoxazolyl, Benzisoxazolyl, Benzothiazolyl, Benzisothiazolyl, Dihydroindolyl, Pyrrolopyridinyl, Furopyridinyl, Thienopyridinyl, Imidazopyridinyl, Oxazolopyridinyl, Thiazolopyridinyl, Pyrazolopyridinyl, Isoxazolopyridinyl, Isothiazolopyridinyl, Tetrahydrochinolinyl, Tetrahydroisochinolinyl, Phenyloxy, Phenylthio, Phenylsulfonyl, Benzoyl, Benzyloxy, Phenylethoxy, Phenylpropoxy, Naphthalinyloxy, Naphthalinylmethoxy, Naphthalinylethoxy, Naphthalinylpropoxy, Chinolinoxy und Isochinolinoxy ausgewählt ist und gegebenenfalls durch einen oder mehrere unter einem Halogenatom und einer Cyano-, Nitro-, C₁₋₄-Alkyl-, Hydroxy-, C₁₋₄-Alkoxy-, C₁₋₄-Thioalkyl-, C₁₋₃-Fluoralkyl-, C₁₋₃-Fluoralkoxy-, C₁₋₃-Fluorthioalkyl-, Phenyloxy-, Benzyloxy-, Piperidinyl-, Pyrrolidinyl-, Morpholinyl-, NR₆R₇-, NHCOR₆-, COR₆-, CO₂R₆-, SO₂R₆-, -O-(C₁₋₃-Alkylen)-O- und einer gegebenenfalls durch ein C₁₋₃-Alkyl oder ein Benzyl substituierten 4-Piperazinylgruppe ausgewählte Substituenten substituiert ist, steht;
R₆ und R₇ unabhängig voneinander für eine C₁₋₃-Alkyl- oder Phenylgruppe stehen und
R₃ für eine Gruppe der allgemeinen Formel CHR₄CONHR₅ steht, worin
R₄ für ein Wasserstoffatom oder eine C₁₋₃-Alkylgruppe steht und R₅ für ein Wasserstoffatom oder eine C₁₋₃-Alkyl-, C₃₋₅-Cycloalkyl- oder C₃₋₇-Cycloalkyl-C₁₋₆-alkylengruppe steht;
in Basen-, Säureadditionssalz-, Hydrat- oder Solvatform;
wobei Benzylcarbamidsäure-2-amino-2-oxoethylester ausgeschlossen ist.

2. Verbindung der Formel (I) nach Anspruch 1, **dadurch gekennzeichnet, daß:**
n für eine ganze Zahl von 1 bis 7 steht;
A unter einer oder mehreren Gruppen X, Y und/oder
Z ausgewählt ist;
X für eine gegebenenfalls durch eine oder mehrere C₁₋₁₂-Alkyl-, C₃₋₇-Cycloalkyl- oder C₃₋₇-Cycloalkyl-C₁₋₆-alkylengruppen substituierte C₁₋₂-Alkylengruppe steht;
Y entweder für eine gegebenenfalls durch eine oder mehrere C₁₋₁₂-Alkyl-, C₃₋₇-Cycloalkyl- oder C₃-₇-Cycloalkyl-C₁₋₆-alkylengruppen substituierte C₂-Alkenylengruppe oder eine C₂-Alkinylengruppe steht;
Z für eine C₃-₇-Cycloalkylgruppe der Formel: 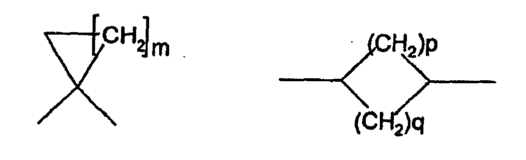 steht;
m für eine ganze Zahl von 1 bis 5 steht;
p und q für ganze Zahlen stehen und so definiert sind, daß p+q für eine ganze Zahl von 1 bis 5 steht;
R₁ für ein Wasserstoff- oder Halogenatom oder eine Hydroxy-, Cyano-, Nitro-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe steht;
R₂ für
ein Wasserstoff- oder Halogenatom oder
eine Cyano-, Nitro-, Hydroxy-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe oder eine Gruppe, die insbesondere unter Phenyl, Naphthalinyl, Biphenyl, Phenylethylenyl, Naphthylethylenyl, Pyridinyl, Pyrimidinyl, Pyrazinyl, Pyridazinyl, Triazinyl, Indanyl, Indenyl, Chinolinyl, Isochinolinyl, Chinazolinyl, Chinoxalinyl, Phthalazinyl, Cinnolinyl, Thienyl, Furanyl, Pyrrolyl, Imidazolyl, Pyrazolyl, Oxazolyl, Thiazolyl, Isoxazolyl, Isothiazolyl, Thiadiazolyl, Oxadiazolyl, Triazolyl, Phenylimidazolyl, Benzothienyl, Benzofuranyl, Dibenzofuranyl, Benzimidazolyl, Benzotriazolyl, Indolyl, Isoindolyl, Indazolyl, Benzoxazolyl, Benzisoxazolyl, Benzothiazolyl, Benzisothiazolyl, Dihydroindolyl, Pyrrolopyridinyl, Furopyridinyl, Thienopyridinyl, Imidazopyridinyl, Oxazolopyridinyl, Thiazolopyridinyl, Pyrazolopyridinyl, Isoxazolopyridinyl, Isothiazolopyridinyl, Tetrahydrochinolinyl, Tetrahydroisochinolinyl, Phenyloxy, Phenylthio, Phenylsulfonyl, Benzoyl, Benzyloxy, Phenylethoxy, Phenylpropoxy, Naphthalinyloxy, Naphthalinylmethoxy, Naphthalinylethoxy, Naphthalinylpropoxy, Chinolinoxy und Isochinolinoxy ausgewählt ist und gegebenenfalls durch einen oder mehrere unter einem Halogenatom und einer Cyano-, Nitro-, C₁₋₄-Alkyl-, Hydroxy-, C₁₋₄-Alkoxy-, C₁₋₄-Thioalkyl-, C₁₋₃-Fluoralkyl-, C₁₋₃-Fluoralkoxy-, C₁₋₃-Fluorthioalkyl-, Phenyloxy-, Benzyloxy-, Piperidinyl-, Pyrrolidinyl-, Morpholinyl-, NR₆R₇-, NHCOR₆-, COR₆-, CO₂R₆-, SO₂R₆-, -O-(C₁₋₃-Alkylen)-O- und einer gegebenenfalls durch ein C₁₋₃-Alkyl oder ein Benzyl substituierten 4-Piperazinylgruppe ausgewählte Substituenten substituiert ist,
steht;
R₆ und R₇ unabhängig voneinander für eine C₁₋₃-Alkyl- oder Phenylgruppe stehen und
R₃ für eine Gruppe der allgemeinen Formel CHR₄CONHR₅ steht, worin
R₄ für ein Wasserstoffatom oder eine C₁₋₃-Alkylgruppe steht und R₅ für ein Wasserstoffatom oder eine C₁₋₃-Alkyl-, C₃₋₅-Cycloalkyl- oder C₃₋₇-Cycloalkyl-C₁₋₆-alkylengruppe steht;
mit der Maßgabe, daß dann, wenn R₁ und R₂ für ein Wasserstoffatom stehen und A für eine Gruppe X steht, wobei X für ein Methylen steht, n von 1 verschieden ist;
in Basen-, Säureadditionssalz-, Hydrat- oder Solvatform.

3. Verbindung der Formel (I) nach Anspruch 1 oder 2,
**dadurch gekennzeichnet, daß**:
- wenn n gleich 1 ist:
A unter einer oder mehreren Gruppen X, Y und/oder
Z ausgewählt ist;
X für eine gegebenenfalls durch eine oder mehrere C₁₋₁₂-Alkyl-, C₃₋₇-Cycloalkyl- oder C₃₋₇-Cycloalkyl-C₁₋₆-alkylengruppen substituierte C₁₋₂-Alkylengruppe steht;
Y entweder für eine gegebenenfalls durch eine oder mehrere C₁₋₁₂-Alkyl-, C₃₋₇-Cycloalkyl- oder C₃₋₇-Cycloalkyl-C₁-₆-alkylengruppen substituierte C₂-Alkenylengruppe oder eine C₂-Alkinylengruppe steht; Z für eine C₃-₇-Cycloalkylgruppe der Formel: 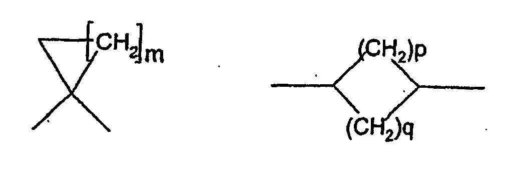 steht;
m für eine ganze Zahl von 1 bis 5 steht;
p und q für ganze Zahlen stehen und so definiert sind, daß p+q für eine ganze Zahl von 1 bis 5 steht;
R₁ für ein Wasserstoff- oder Halogenatom oder eine Hydroxy-, Cyano-, Nitro-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe steht;
R₂ für
ein Wasserstoff- oder Halogenatom oder
eine Cyano-, Nitro-, Hydroxy-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe oder eine Gruppe, die insbesondere unter Phenyl, Naphthalinyl, Biphenyl, Phenylethylenyl, Naphthylethylenyl, Pyridinyl, Pyrimidinyl, Pyrazinyl, Pyridazinyl, Triazinyl, Indanyl, Indenyl, Chinolinyl, Isochinolinyl, Chinazolinyl, Chinoxalinyl, Phthalazinyl, Cinnolinyl, Thienyl, Furanyl, Pyrrolyl, Imidazolyl, Pyrazolyl, Oxazolyl, Thiazolyl, Isoxazolyl, Isothiazolyl, Thiadiazolyl, Oxadiazolyl, Triazolyl, Phenylimidazolyl, Benzothienyl, Benzofuranyl, Dibenzofuranyl, Benzimidazolyl, Benzotriazolyl, Indolyl, Isoindolyl, Indazolyl, Benzoxazolyl, Benzisoxazolyl, Benzothiazolyl, Benzisothiazolyl, Dihydroindolyl, Pyrrolopyridinyl, Furopyridinyl, Thienopyridinyl, Imidazopyridinyl, Oxazolopyridinyl, Thiazolopyridinyl, Pyrazolopyridinyl, Isoxazolopyridinyl, Isothiazolopyridinyl, Tetrahydrochinolinyl, Tetrahydroisochinolinyl, Phenyloxy, Phenylthio, Phenylsulfonyl, Benzoyl, Benzyloxy, Phenylethoxy, Phenylpropoxy, Naphthalinyloxy, Naphthalinylmethoxy, Naphthalinylethoxy, Naphthalinylpropoxy, Chinolinoxy und Isochinolinoxy ausgewählt ist und gegebenenfalls durch einen oder mehrere unter einem Halogenatom und einer Cyano-, Nitro-, C₁₋₄-Alkyl-, Hydroxy-, C₁_₄-Alkoxy-, C₁₋₄-Thioalkyl-, C₁₋₃-Fluoralkyl-, C₁₋₃-Fluoralkoxy-, C₁₋₃-Fluorthioalkyl-, Phenyloxy-, Benzyloxy-, Piperidinyl-, Pyrrolidinyl-, Morpholinyl-, NR₆R₇-, NHCOR₆-, COR₆-, CO₂R₆-, SO₂R₆-, -O-(C₁₋₃-Alkylen)-O- und einer gegebenenfalls durch ein C₁₋₃-Alkyl oder ein Benzyl substituierten 4-Piperazinylgruppe ausgewählte Substituenten substituiert ist, steht;
R₆ und R₇ unabhängig voneinander für eine C₁₋₃-Alkyl- oder Phenylgruppe stehen und
R₃ für eine Gruppe der allgemeinen Formel CHR₄CONHR₅ steht, worin
R₄ für ein Wasserstoffatom oder eine C₁₋₃-Alkylgruppe steht und R₅ für ein Wasserstoffatom oder eine C₁₋₃-Alkyl-, C₃₋₅-Cycloalkyl- oder C₃₋₇-Cycloalkyl-C₁₋₆-alkylengruppe steht;
- wenn n für eine ganze Zahl von 2 bis 7 steht:
A unter einer oder mehreren Gruppen X, Y und/oder Z ausgewählt ist;
X für eine gegebenenfalls durch eine oder mehrere C₁₋₁₂-Alkyl-, C₃₋₇-Cycloalkyl- oder C₃₋₇-Cycloalkyl-C₁₋₆-alkylengruppen substituierte C₁₋₂-Alkylengruppe steht;
Y entweder für eine gegebenenfalls durch eine oder mehrere C₁₋₁₂-Alkyl-, C₃₋₇-Cycloalkyl- oder C₃₋₇-Cycloalkyl-C₁₋₆-alkylengruppen substituierte C₂-Alkenylengruppe oder eine C₂-Alkinylengruppe steht;
Z für eine C₃₋₇-Cycloalkylgruppe der Formel: 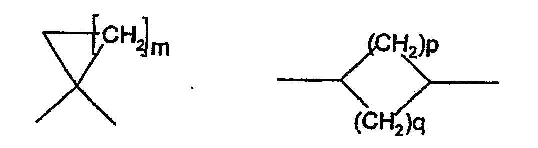 steht;
m für eine ganze Zahl von 1 bis 5 steht;
p und q für ganze Zahlen stehen und so definiert sind, daß p+q für eine ganze Zahl von 1 bis 5 steht;
R₁ für ein Wasserstoff- oder Halogenatom oder eine Hydroxy-, Cyano-, Nitro-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe steht;
R₂ für
ein Wasserstoff- oder Halogenatom oder
eine Cyano-, Nitro-, Hydroxy-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe oder eine Gruppe, die insbesondere unter Phenyl, Naphthalinyl, Biphenyl, Phenylethylenyl, Naphthylethylenyl, Pyridinyl, Pyrimidinyl, Pyrazinyl, Pyridazinyl, Triazinyl, Indanyl, Indenyl, Chinolinyl, Isochinolinyl, Chinazolinyl, Chinoxalinyl, Phthalazinyl, Cinnolinyl, Thienyl, Furanyl, Pyrrolyl, Imidazolyl, Pyrazolyl, Oxazolyl, Thiazolyl, Isoxazolyl, Isothiazolyl, Thiadiazolyl, Oxadiazolyl, Triazolyl, Phenylimidazolyl, Benzothienyl, Benzofuranyl, Dibenzofuranyl, Benzimidazolyl, Benzotriazolyl, Indolyl, Isoindolyl, Indazolyl, Benzoxazolyl, Benzisoxazolyl, Benzothiazolyl, Benzisothiazolyl, Dihydroindolyl, Pyrrolopyridinyl, Furopyridinyl, Thienopyridinyl, Imidazopyridinyl, Oxazolopyridinyl, Thiazolopyridinyl, Pyrazolopyridinyl, Isoxazolopyridinyl, Isothiazolopyridinyl, Tetrahydrochinolinyl, Tetrahydroisochinolinyl, Phenyloxy, Phenylthio, Phenylsulfonyl, Benzoyl, Benzyloxy, Phenylethoxy, Phenylpropoxy, Naphthalinyloxy, Naphthalinylmethoxy, Naphthalinylethoxy, Naphthalinylpropoxy, Chinolinoxy und Isochinolinoxy ausgewählt ist und gegebenenfalls durch einen oder mehrere unter einem Halogenatom und einer Cyano-, Nitro-, C₁₋₄-Alkyl-, Hydroxy-, C₁₋₄-Alkoxy-, C₁₋₄-Thioalkyl-, C₁₋₃-Fluoralkyl-, C₁₋₃-Fluoralkoxy-, C₁₋₃-Fluorthioalkyl-, Phenyloxy-, Benzyloxy-, Piperidinyl-, Pyrrolidinyl-, Morpholinyl-, NR₆R₇-, NHCOR₆-, COR₆-, CO₂R₆-, SO₂R₆-, -O-(C₁₋₃-Alkylen)-O- und einer gegebenenfalls durch ein C₁₋₃-Alkyl oder ein Benzyl substituierten 4-Piperazinylgruppe ausgewählte Substituenten substituiert ist, steht;
R₆ und R₇ unabhängig voneinander für eine C₁₋₃-Alkyl- oder Phenylgruppe stehen und
R₃ für eine Gruppe der allgemeinen Formel CHR₄CONHR₅ steht, worin
R₄ für ein Wasserstoffatom oder eine C₁₋₃-Alkylgruppe steht und R₅ für ein Wasserstoffatom oder eine C₁₋₃-Alkyl-, C₃₋₅-Cycloalkyl- oder C₃₋₇-Cycloalkyl-C₁₋₆-alkylengruppe steht;
in Basen-, Säureadditionssalz-, Hydrat- oder Solvatform.

4. Verbindung der Formel (I) nach einem der Ansprüche 1 bis 3, **dadurch gekennzeichnet, daß**
n für eine ganze Zahl von 1 bis 5 steht;
A unter einer oder mehreren Gruppen X und/oder Z ausgewählt ist;
X für eine gegebenenfalls durch eine oder mehrere C₁₋₃-Alkylgruppen substituierte C₁₋₂-Alkylengruppe steht;
Z für eine C₃₋₇-Cycloalkylgruppe der Formel: 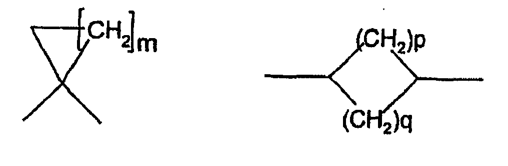 steht;
m für eine ganze Zahl von 1 bis 5 steht;
p und q für ganze Zahlen stehen und so definiert sind, daß p+q für eine ganze Zahl von 1 bis 5 steht;
R₁ für ein Wasserstoff- oder Halogenatom oder eine C₁₋₄-Alkoxygruppe steht;
R₂ für
ein Wasserstoff- oder Halogenatom, insbesondere Chlor, Brom oder Fluor, oder eine C₁₋₄-Alkylgruppe, insbesondere Methyl, eine C₁₋₄-Alkoxygruppe, insbesondere Methoxy, eine C₁₋₄-Fluoralkylgruppe, insbesondere Trifluormethyl, eine C₁₋₄-Fluoralkoxygruppe, insbesondere Trifluormethoxy, oder eine Gruppe, die unter Phenyl, Naphthalinyl, Biphenyl, Phenylethylenyl, Pyridinyl, Pyrimidinyl, Pyrazinyl, Chinolinyl, Isochinolinyl, Thienyl, Furanyl, Isoxazolyl, Thiadiazolyl, Phenylimidazolyl, Benzothienyl, Dibenzofuranyl, Benzimidazolyl, Pyrrolopyridinyl, Phenyloxy, Phenylsulfonyl, Benzoyl, Benzyloxy oder Phenylpropoxy ausgewählt ist und gegebenenfalls durch einen oder mehrere unter einem Halogenatom, insbesondere Chlor oder Fluor, und einer Cyanogruppe, Nitrogruppe, C₁₋₄-Alkylgruppe, insbesondere Methyl, Ethyl, Isopropyl, Butyl oder tert.-Butyl, C₁₋₄-Alkoxygruppe, insbesondere Methoxy oder Ethoxy, C₁₋₄-Thioalkylgruppe, insbesondere Thiomethyl, C₁₋₃-Fluoralkylgruppe, insbesondere Trifluormethyl, C₁₋₃-Fluoralkoxygruppe, insbesondere Trifluormethoxy, Phenyloxygruppe, Benzyloxygruppe, NR₆R₇-Gruppe, NHCOR₆-Gruppe, COR₆-Gruppe, CO₂R₆-Gruppe, SO₂R₆-Grup und einer -O-(C₁₋₃-Alkylen)-O-Gruppe, insbesondere -O-(CH₂)-O-, ausgewählte Substituenten substituiert ist, steht;
R₆ und R₇ unabhängig voneinander für eine C₁₋₃-Alkylgruppe stehen und
R₃ für eine Gruppe der allgemeinen Formel CHR₄CONHR₅ steht, worin
R₄ für ein Wasserstoffatom oder eine C₁₋₃-Alkylgruppe steht und R₅ für ein Wasserstoffatom oder eine C₁₋₃-Alkyl-, C₃₋₅-Cycloalkyl- oder C₃-₇-Cycloalkyl-C₁₋₆-alkylengruppe steht;
in Basen-, Säureadditionssalz-, Hydrat- oder Solvatform.

5. Verbindung der Formel (I) nach einem der Ansprüche 1 bis 4, **dadurch gekennzeichnet, daß**
n für eine ganze Zahl von 1 bis 5 steht;
A für eine C₁₋₂-Alkylengruppe steht;
R₁ für ein Wasserstoff- oder Halogenatom steht;
R₂ für eine Gruppe, die unter Phenyl, Naphthalinyl, Phenyloxy, Benzyloxy, Pyridinyl, Chinolinyl, Isochinolinyl, Phenylimidazol oder Pyrrolopyridinyl ausgewählt ist und gegebenenfalls durch einen oder mehrere unter einem Halogenatom, insbesondere Chlor oder Fluor, und einer Cyanogruppe, C₁₋₄-Alkylgruppe, insbesondere Methyl, C₁₋₄-Alkoxygruppe, insbesondere Methoxy, C₁₋₃-Fluoralkylgruppe, insbesondere Trifluormethyl, und einer C₁₋₃-Fluoralkoxygruppe, insbesondere Trifluormethoxy, ausgewählte Substituenten substituiert ist, steht;
R₃ für eine Gruppe der allgemeinen Formel CHR₄CONHR₅ steht, worin
R₄ für ein Wasserstoffatom steht und R₅ für ein Wasserstoffatom oder eine C₁₋₃-Alkyl-, C₃₋₅-Cycloalkyl- oder C₃₋₇-Cycloalkyl-C₁₋₆-alkylengruppe steht;
in Basen-, Säureadditionssalz-, Hydrat- oder Solvatform.

6. Verbindung der Formel (I) nach einem der Ansprüche 1 bis 5, **dadurch gekennzeichnet, daß**
n für eine ganze Zahl von 5 bis 7 steht;
A für eine C₁₋₂-Alkylengruppe steht;
R₁ und R₂ jeweils unabhängig voneinander für ein Wasserstoff- oder Halogenatom oder eine Cyano-, Hydroxy-, C₁₋₄-Alkyl-, C₁₋₄-Alkoxy-, C₁₋₄-Fluoralkyl-, C₁₋₄-Fluoralkoxygruppe stehen;
R₃ für eine Gruppe der allgemeinen Formel CHR₄CONHR₅ steht, worin
R₄ für ein Wasserstoffatom steht und R₅ für ein Wasserstoffatom oder eine C₁₋₃-Alkyl-, C₃₋₅-Cycloalkyl- oder C₃₋₇-Cycloalkyl-C₁₋₆-alkylengruppe steht;
in Basen-, Säureadditionssalz-, Hydrat- oder Solvatform.

7. Verfahren zur Herstellung einer Verbindung der Formel (I) nach einem der Ansprüche 1 bis 6, bei dem man
den Carbamatester der allgemeinen Formel (Ia) 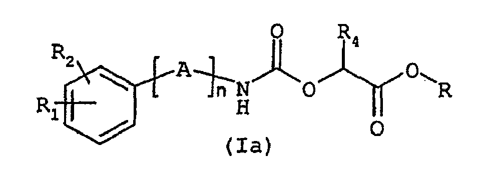 worin A, n, R₁, R₂ und R₄ die in der Formel (I) nach Anspruch 1 angegebene Bedeutung besitzen und R für eine Methyl- oder Ethylgruppe steht,
durch Aminolyse mit einem Amin der allgemeinen Formel R₅NH₂, worin R₅ die in der Formel (I) nach Anspruch 1 angegebene Bedeutung besitzt, umwandelt.

8. Verfahren zur Herstellung einer Verbindung der Formel (I) nach einem der Ansprüche 1 bis 6, bei dem man
das Oxazolidindionderivat der allgemeinen Formel (V) 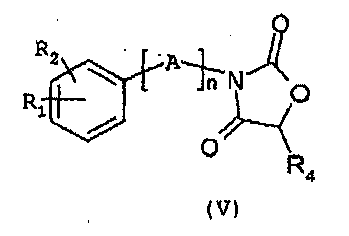 worin A, n, R₁, R₂ und R₄ die in der Formel (I) nach Anspruch 1 angegebene Bedeutung besitzen,
durch Aminolyse mit einem Amin der allgemeinen Formel R₅NH₂, worin R₅ die in der Formel (I) nach Anspruch 1 angegebene Bedeutung besitzt, umwandelt.

9. Verbindung der allgemeinen Formel (Ia) 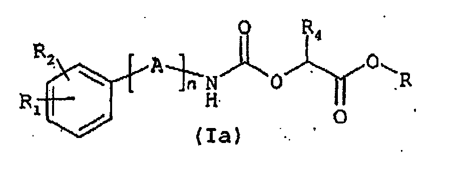 worin
n für eine ganze Zahl von 1 bis 7 steht;
A unter einer oder mehreren Gruppen X, Y und/oder Z ausgewählt ist;
X für eine gegebenenfalls durch eine oder mehrere C₁₋₁₂-Alkyl-, C₃₋₇-Cycloalkyl- oder C₃₋₇-Cycloalkyl-C₁₋₆-alkylengruppen substituierte C₁₋₂-Alkylengruppe steht;
Y entweder für eine gegebenenfalls durch eine oder mehrere C₁₋₁₂-Alkyl-, C₃₋₇-Cycloalkyl- oder C₃₋₇-Cycloalkyl-C₁₋₆-alkylengruppen substituierte C₂-Alkenylengruppe oder eine C₂-Alkinylengruppe steht;
Z für eine C₃₋₇-Cycloalkylgruppe der Formel: 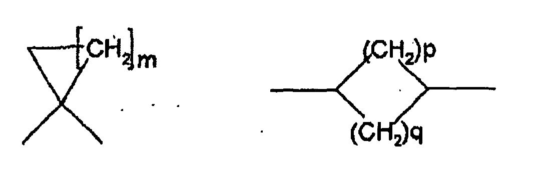 steht;
m für eine ganze Zahl von 1 bis 5 steht;
p und q für ganze Zahlen stehen und so definiert sind, daß p+q für eine ganze Zahl von 1 bis 5 steht;
R₁ für ein Wasserstoff- oder Halogenatom oder eine Hydroxy-, Cyano-, Nitro-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe steht;
R₂ für
ein Wasserstoff- oder Halogenatom oder
eine Cyano-, Nitro-, Hydroxy-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe oder eine Gruppe, die unter Phenyl, Naphthalinyl, Biphenyl, Phenylethylenyl, Naphthylethylenyl, Pyridinyl, Pyrimidinyl, Pyrazinyl, Pyridazinyl, Triazinyl, Indanyl, Indenyl, Chinolinyl, Isochinolinyl, Chinazolinyl, Chinoxalinyl, Phthalazinyl, Cinnolinyl, Thienyl, Furanyl, Pyrrolyl, Imidazolyl, Pyrazolyl, Oxazolyl, Thiazolyl, Isoxazolyl, Isothiazolyl, Thiadiazolyl, Oxadiazolyl, Triazolyl, Phenylimidazolyl, Benzothienyl, Benzofuranyl, Dibenzofuranyl, Benzimidazolyl, Benzotriazolyl, Indolyl, Isoindolyl, Indazolyl, Benzoxazolyl, Benzisoxazolyl, Benzothiazolyl, Benzisothiazolyl, Dihydroindolyl, Pyrrolopyridinyl, Furopyridinyl, Thienopyridinyl, Imidazopyridinyl, Oxazolopyridinyl, Thiazolopyridinyl, Pyrazolopyridinyl, Isoxazolopyridinyl, Isothiazolopyridinyl, Tetrahydrochinolinyl, Tetrahydroisochinolinyl, Phenyloxy, Phenylthio, Phenylsulfonyl, Benzoyl, Benzyloxy, Phenylethoxy, Phenylpropoxy, Naphthalinyloxy, Naphthalinylmethoxy, Naphthalinylethoxy, Naphthalinylpropoxy, Chinolinoxy und Isochinolinoxy ausgewählt ist und gegebenenfalls durch einen oder mehrere unter einem Halogenatom und einer Cyano-, Nitro-, C₁₋₄-Alkyl-, Hydroxy-, C₁₋₄-Alkoxy-, C₁₋₄-Thioalkyl-, C₁₋₃-Fluoralkyl-, C₁₋₃-Fluoralkoxy-, C₁₋₃-Fluorthioalkyl-, Phenyloxy-, Benzyloxy-, Piperidinyl-, Pyrrolidinyl-, Morpholinyl-, NR₆R₇-, NHCOR₆-, COR₆-, CO₂R₆-, SO₂R₆-, -O- (C₁₋₃-Alkylen) -O- und einer gegebenenfalls durch ein C₁₋₃-Alkyl oder ein Benzyl substituierten 4-Piperazinylgruppe ausgewählte Substituenten substituiert ist,
steht;
R₆ und R₇ unabhängig voneinander für eine C₁₋₃-Alkyl- oder Phenylgruppe stehen;
R₄ für ein Wasserstoffatom steht und
R für eine Methylgruppe steht.

10. Verbindung der allgemeinen Formel (V) 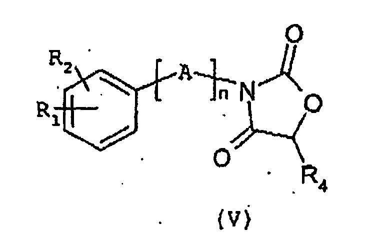 worin
n für eine ganze Zahl von 1 bis 7 steht;
A unter einer oder mehreren Gruppen X, Y und/oder Z ausgewählt ist;
X für eine gegebenenfalls durch eine oder mehrere C₁₋₁₂-Alkyl-, C₃-₇-Cycloalkyl- oder C₃₋₇-Cycloalkyl-C₁₋₆-alkylengruppen substituierte C₁₋₂-Alkylengruppe steht;
Y entweder für eine gegebenenfalls durch eine oder mehrere C₁₋₁₂-Alkyl-, C₃₋₇-Cycloalkyl- oder C₃₋₇-Cycloalkyl-C₁₋₆-alkylengruppen substituierte C₂-Alkenylengruppe oder eine C₂-Alkinylengruppe steht; Z für eine C₃₋₇-Cycloalkylgruppe der Formel: 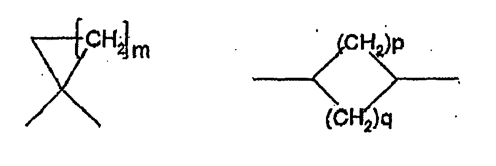 steht;
m für eine ganze Zahl von 1 bis 5 steht;
p und q für ganze Zahlen stehen und so definiert sind, daß p+q für eine ganze Zahl von 1 bis 5 steht;
R₁ für ein Wasserstoff- oder Halogenatom oder eine Hydroxy-, Cyano-, Nitro-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe steht;
R₂ für ein Wasserstoff-, Brom-, Iod- oder Fluoratom oder eine Cyano-, Nitro-, Hydroxy-, C₁₋₄-Alkyl-, C₁₋₄-Alkoxy-, C₁₋₄-Thioalkyl-, C₁₋₄-Fluoralkyl-, C₁₋₄-Fluoralkoxy- oder C₁₋₄-Fluorthioalkylgruppe
oder eine Gruppe, die unter Phenyl, Naphthalinyl, Biphenyl, Phenylethylenyl, Naphthylethylenyl, Pyridinyl, Pyrimidinyl, Pyrazinyl, Pyridazinyl, Triazinyl, Indanyl, Indenyl, Chinolinyl, Isochinolinyl, Chinazolinyl, Chinoxalinyl, Phthalazinyl, Cinnolinyl, Thienyl, Furanyl, Pyrrolyl, Imidazolyl, Pyrazolyl, Oxazolyl, Thiazolyl, Isoxazolyl, Isothiazolyl, Thiadiazolyl, Oxadiazolyl, Triazolyl, Phenylimidazolyl, Benzothienyl, Benzofuranyl, Dibenzofuranyl, Benzimidazolyl, Benzotriazolyl, Indolyl, Isoindolyl, Indazolyl, Benzoxazolyl, Benzisoxazolyl, Benzothiazolyl, Benzisothiazolyl, Dihydroindolyl, Pyrrolopyridinyl, Furopyridinyl, Thienopyridinyl, Imidazopyridinyl, Oxazolopyridinyl, Thiazolopyridinyl, Pyrazolopyridinyl, Isoxazolopyridinyl, Isothiazolopyridinyl, Tetrahydrochinolinyl, Tetrahydroisochinolinyl, Phenyloxy, Phenylthio, Phenylsulfonyl, Benzoyl, Benzyloxy, Phenylethoxy, Phenylpropoxy, Naphthalinyloxy, Naphthalinylmethoxy, Naphthalinylethoxy, Naphthalinylpropoxy, Chinolinoxy und Isochinolinoxy ausgewählt ist und gegebenenfalls durch einen oder mehrere unter einem Halogenatom und einer Cyano-, Nitro-, C₁₋₄-Alkyl-, Hydroxy-, C₁₋₄-Alkoxy-, C₁₋₄-Thioalkyl-, C₁₋₃-Fluoralkyl-, C₁₋₃-Fluoralkoxy-, C₁₋₃-Fluorthioalkyl-, Phenyloxy-, Benzyloxy-, Piperidinyl-, Pyrrolidinyl-, Morpholinyl-, NR₆R₇-, NHCOR₆-, COR₆-, CO₂R₆-, SO₂R₆-, -O-(C₁₋₃-Alkylen)-O- und einer gegebenenfalls durch ein C₁₋₃-Alkyl oder ein Benzyl substituierten 4-Piperazinylgruppe ausgewählte Substituenten substituiert ist,
steht;
R₆ und R₇ unabhängig voneinander für eine C₁₋₃-Alkyl- oder Phenylgruppe stehen und
R₄ für ein Wasserstoffatom steht;
mit der Maßgabe, daß die Verbindungen, für die die Gruppe 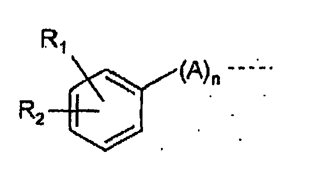 für eine Benzyl-, para-Methoxybenzyl-, para-Nitrobenzyl- und 1-Methyl-2-phenylethylgruppe steht, ausgeschlossen sind.

11. Pharmazeutische Zusammensetzung, enthaltend mindestens eine Verbindung der Formel (I) nach einem der Ansprüche 1 bis 6 in pharmazeutisch unbedenklicher Basen-, Salz-, Hydrat- oder Solvatform und gegebenenfalls einen oder mehrere pharmazeutisch unbedenkliche Hilfsstoffe.

12. Verbindung der Formel (I) nach einem der Ansprüche 1 bis 6 in pharmazeutisch unbedenklicher Basen-, Salz-, Hydrat- oder Solvatform zur Verwendung als Arzneimittel.

13. Verwendung einer Verbindung der Formel (I) nach einem der Ansprüche 1 bis 6 in pharmazeutisch unbedenklicher Basen-, Salz-, Hydrat- oder Solvatform zur Herstellung eines Arzneimittels zur Prävention oder Behandlung von akuten oder chronischen Schmerzen, Schwindel, Erbrechen, Übelkeit, Eßstörungen, neurologischen und psychiatrischen Pathologien, akuten oder chronischen neurodegenerativen Erkrankungen, Epilepsie, Schlafstörungen, Herz-Kreislauf-Erkrankungen, Nierenischämie, Krebs, Störungen des Immunsystems, allergischen Erkrankungen, parasitären, viralen oder bakteriellen Infektionserkrankungen, entzündlichen Erkrankungen, Osteoporose, Augenleiden, Lungenleiden, Magen-Darm-Erkrankungen oder Harninkontinenz.
